(19) Europäisches Patentamt
European Patent Office
Office européen des brevets

(11) Publication number: **0 048 954**
**B1**

# (12) EUROPEAN PATENT SPECIFICATION

(45) Date of publication of patent specification: **30.12.86**

(21) Application number: **81107575.3**

(22) Date of filing: **23.09.81**

(51) Int. Cl.$^4$: **C 07 D 501/20,** C 07 D 499/50, C 07 D 307/54, C 07 C 125/065, C 07 D 277/38, C 07 D 333/28, C 07 D 333/24, C 07 D 207/32, C 07 C 59/56, C 07 C 59/60, C 07 C 59/64

## (54) Beta-lactam compounds, process for the preparation thereof and intermediate products for the preparation thereof.

(30) Priority: **26.09.80 JP 132994/80**
**30.03.81 JP 45510/81**

(43) Date of publication of application:
**07.04.82 Bulletin 82/14**

(45) Publication of the grant of the patent:
**30.12.86 Bulletin 86/52**

(84) Designated Contracting States:
**CH DE FR GB LI LU NL SE**

(56) References cited:
**FR-A-2 253 516**
**GB-A-1 299 295**

**DERWENT JAPANESE PATENT REPORTS vol. 6, no. 24, 1967**

**TETRAHEDRON, vol. 22, 1966 Pergamonn Press Y. ITO et al.: "The Mechanism of the Reaction of P-ylides with Dichlorocarbene", pages 2615-2619**

(73) Proprietor: **SAGAMI CHEMICAL RESEARCH CENTER**
**4-5, Marunouchi 1-chome**
**Chiyoda-ku, Tokyo, 100 (JP)**

(72) Inventor: **Tunemoto, Daiei**
**2020-59, Midorigaoka**
**Zama-shi Kanagawa-ken (JP)**
Inventor: **Kobori, Takeo**
**3-27-14, Moridai**
**Atsugi-shi Kanagawa-ken (JP)**
Inventor: **Kono, Hiromichi**
**1-9-2, Minamidai**
**Sagamihara-shi Kanagawa-ken (JP)**
Inventor: **Kondo, Kiyosi**
**5-16-4, Chuorinkan**
**Yamato-shi Kanagawa-ken (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 048 954 B1

(56) References cited:

**BULLETIN DE LA SOCIETE CHIMIQUE DE FRANCE, mars-avril 1974 C. RAULET: "Synthèse de composés conjugués renfermant un groupement CCL2-C(C6H5): vinylogue et éthynylogue de l'alpha-phényl Beta, Beta-dichloroacroléine et alpha-phényl Beta, Beta-dichlorovinylcétones; préparation de dichlorométhylène-4 4 H-naphtalénones-1.", pages 531-537**

**Description**

The present invention relates to novel β-lactam compounds, a process for the preparation of these compounds, and intermediate products useful for synthesizing these compounds.

Penicillin and cephalosporin compounds show broad antibacterial activities against gram-positive and gram-negative bacteria. Thus, several types of semi-synthetic penicillin and cephalosporin compounds are sold commercially and used as medicines for various infectious diseases in clinical applications. However, there are only a few medicine that can show antibacterial activities against *Pseudomonas aerugionosa, proteus vulgaris* or the like which are causative of clinically serious infectious diseases. In addition, some of the above-mentioned commercially available agents are relatively unstable against β-lactamase generated by resistant strains of bacteria. These agents are also inadvantageous in that they show only a low antibacterial activity against agent-resistant bacteria, arousing problems in clinical applications (W. E. Wick, Cephalosporins and Penicillins, Chemistry and Biology, E. H. Flynn, Academic Press, New York, N. Y., 1972, Chapter 11). Accordingly, there has been continuous search for β-lactam compounds that have effective antibacterial properties against such pathogens, and there is a need for novel and effective β-lactam compounds.

An object of the present invention is to provide β-lactam compounds useful as antibacterial agents.

Another object of the present invention is to provide a novel process for preparing such useful β-lactam compounds.

A further object of the present invention is to provide intermediate products which are useful for the synthesis of the aforesaid β-lactam compounds or other useful compounds.

The novel β-lactam compounds provided according to the present invention are defined by the following general formula I:

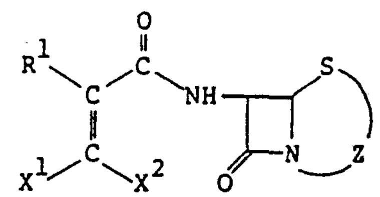

I

in which $R^1$ denotes an aromatic group selected from the group consisting of a phenyl radical, a thiazolyl radical, a thienyl radical, a furyl radical and a pyrrolyl radical, which may be substituted by a halogen atom, a nitro radical, an amino radical, a cyano radical, a hydroxy radical, a lower alkyl radical having 1 to 4 carbon atoms, a lower alkoxy radical having 1 to 4 carbon atoms, a lower alkylthio radical having 1 to 4 carbon atoms; Z represents a group

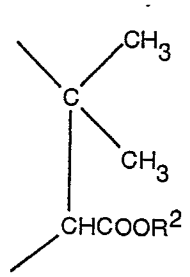

or

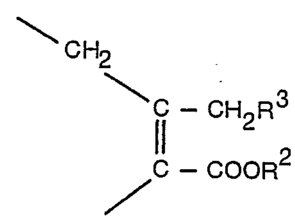

(wherein $R^2$ denotes hydrogen, a salt forming cation or a protective group for carboxylic acid, and $R^3$ denotes hydrogen or a residue selected from the group consisting of a hydroxyl radical, a mercapto radical, a lower aliphatic carboxylic acid acyloxy radical having 2 to 4 carbon atoms, an aromatic carboxylic acid acyloxy radical selected from the group consisting of mandelyloxy, 2-carboxy-benzoyloxy, 2-(carboethoxy-carbamoyl)benzoyloxy and 2-(carboethoxysulfamoyl)benzoyloxy, a carbamoyloxy radical, a phenylglycyloxy radical, a quaternary ammonium salt and a heterocyclic ring which is bound via S selected from the group comprising pyridyl, N-oxidepyridyl, pyridazinyl, N-oxidepyridazinyl, pyrazolyl, diazolyl, thiazolyl, thiadiazolyl, oxadiazolyl, triazolyl, and tetrazolyl, which may be substituted by a lower alkyl having from 1 to 3 carbon atoms, a lower alkoxy radical having from 1 to 3 carbon atoms, a halogen atom, a trihalogeno-substituted lower alkyl radical having from 1 to 3 carbon atoms, a hydroxy radical, a mercapto radical, an amino radical, a carboxyl radical, a carbamoyl radical, a di-lower alkylamino lower alkyl having from 1 to 3 carbon atoms in each alkyl radical or a carboxymethyl radical; and $X^1$ and $X^2$ each represent hydrogen or a halogen atom, provided that both $X^1$ and $X^2$ cannot represent hydrogen at the same time.

The inventors have succeeded in synthesizing the β-lactam compounds defined by general formula I, and found out, as shown in The Test Example mentioned below, that these compounds have a broad antibacterial spectrum and show strong antibacterial activities against not only the gram-positive bacteria but also the gram-negative bacteria, particularly the above-mentioned *Pseudomonas aeruginosa, Proteus vulgaris* and *Escherichia coli,* which arouse problems in clinical applications. It has also been found out that

these β-lactam compounds according to the present invention show extremely powerful antibacterial activities against the resistive gram-negative bacteria.

In the following the compounds of the present invention represented by above general formula I are explained in more detail:

The meaning of $R^1$ being an unsubstituted or substituted aromatic group is described above. Also the substituents for $R^1$ are given above. When the substituent is a halogen atom, this can be fluorine, chlorine, bromine and iodine.

$X^1$ and $X^2$ stand for hydrogen or a halogen atom with the proviso that $X^1$ and $X^2$ cannot represent hydrogen at the same time. For example, both $X^1$ and $X^2$ may denote the same halogen atom. Alternatively, one of $X^1$ and $X^2$ may represent hydrogen, and the other a halogen atom. Further, $X^1$ and $X^2$ may represent different halogen atoms.

$R^2$ represents a hydrogen atom, a salt-forming cation or a protective radical for a carboxylic acid. Examples of the cations which $R^2$ represents include those forming alkaline metal salts, alkaline earth metal salts, ammonium salts, etc. The protective radical which $R^2$ represents may be any radical that is normally used for this purpose in the conventional penicillin and caphalosporin compounds, such as alkyl, lower alkoxymethyl, lower alkylthiomethyl, lower alkanoyloxymethyl, aralkyl, aryl and silyl radicals, etc.

The meaning of $R^3$ is described above. When the heterocyclic radical is substituted by substituents as described above, the number of substituents is normally 1 or 2. The quaternary ammonium radical represented by $R^3$ may be, for example, pyridinium, methyl-pyridinium, 3-chloropyridinium, 3-bromo-pyridinium, 3-iodo-pyridinium, 4-carbamoylpyridinium, 4-(N-hydroxymethylcarbamyl)pyridinium, 4-(N-carbomethoxycarbamoyl)pyridinium, 4-(N-cyanocarbamoyl)pyridinium, 4-(carboxymethyl)pyridinium, 4-(hydroxymethyl)pyridinium, 4-(trifluoromethyl)pyridinium, quinolinium, purinium, lutidium, etc.

Next, specific compounds according to the present invention will be exemplified as follows.

1. 7-[2-dichloromethylene-2-(2-furyl)acetamido]-3-(1-methyl-1H-tetrazol-5-yl)thiomethyl-3-cephem-4-carboxylic acid

2. 7-[2-dichloromethylene-2-(4-hydroxyphenyl)acetamido]-3-acetoxymethyl-3-cephem-4-carboxylic acid

3. 7-[2-dichloromethylene-2-(3-hydroxyphenyl)acetamido]-3-acetoxymethyl-3-cephem-4-carboxylic acid

4-(1). 7-[2-dichloromethylene-2-(4-tert-butoxycarbonylaminophenyl)acetamido]-3-acetoxymethyl-3-cephem-4-carboxylic acid

4-(2). 7-[2-dichloromethylene-2-(4-aminophenyl)acetamido]-3-acetoxymethyl-3-cephem-4-carboxylic acid

5-(1). 7-[2-dichloromethylene-2-(2-tert-butoxycarbonylaminothiazol-4-yl)acetamido]-3-acetoxymethyl-3-cephem-4-carboxylic acid

5-(2). 7-[2-dichloromethylene-2-(2-aminothiazol-4-yl)acetamido]-3-acetoxymethyl-3-cephem-4-carboxylic acid

6-(1). 7-[2-dichloromethylene-2-(2-tert-butoxycarbonylaminothiazol-4-yl)acetamido]-3-(1-methyl-1H-tetrazol-5-yl)thiomethyl-3-cephem-4-carboxylic acid

6-(2). 7-[2-dichloromethylene-2-(2-aminothiazol-4-yl)acetamido]-3-(1-methyl-1H-tetrazol-5-yl)thiomethyl-3-cephem-4-carboxylic acid

6-(3). 7-[2-dichloromethylene-(2-aminothiazol-4-yl)acetamido]-3-(1-methyl-1H-tetrazol-5-yl)thiomethyl-3-cephem-4-carboxylic acid sodium salt

7-(1). 7-[2-dichloromethylene-2-(2-tert-butoxycarbonylaminothiazol-4-yl)acetamido]-3-(1-carboxymethyltetrazol-5-yl)thiomethyl-3-cephem-4-carboxylic acid

7-(2). 7-[2-dichloromethylene-2-(2-aminothiazol-4-yl)acetamido]-3-(1-carboxymethyltetrazol-5-yl)thiomethyl-3-cephem-4-carboxylic acid

7-(3). 7-[2-dichloromethylene-2-(2-aminothiazol-4-yl)acetamido]-3-(1-carboxymethyltetrazol-5-yl)thiomethyl-3-cephem-4-carboxylic acid

8-(1). 7-[2-dichloromethylene-2-(2-tert-butoxycarbonylaminothiazol-4-yl)acetamido]desacetoxy-cephalosporanic acid

8-(2). 7-[2-dichloromethylene-2-(2-aminothiazol-4-yl)acetamido]desacetoxycephalosporanic acid

8-(3). 7-[2-dichloromethylene-2-(2-aminothiazol-4-yl)acetamido]-desacetoxycephalosporanic acid sodium salt

9-(1). 7-[2-dichloromethylene-2-(aminothiazol-4-yl)acetamido]-3-(2-methylthio-1,3,4-thiadiazol-5-yl)thiomethyl-3-cephem-4-carboxylic acid

9-(2). 7-[2-dichloromethylene-2-(aminothiazol-4-yl)acetamido]-3-(2-methylthio-1,3,4-thiadiazol-5-yl)thiomethyl-3-cephem-4-carboxylic acid sodium salt

10-(1). 7-[2-dichloromethylene-2-(2-aminothiazol-4-yl)acetamido]-3-[1-(2-dimethylaminoethyl)-1H-tetrazol-5-yl]thiomethyl-3-cephem-4-carboxylic acid

10-(2). 7-[2-dichloromethylene-2-(2-aminothiazol-4-yl)acetamido]-3-[1-(2-dimethylaminoethyl)-1H-tetrazol-5-yl]thiomethyl-3-cephem-4-carboxylic acid sodium salt

11-(1). 7-[2-chloromethylene-2-(2-tert-butoxycarbonylaminothiazol-4-yl)acetamido]-3-(1-methyl-1H-tetrazol-5-yl)thiomethyl-3-cephem-4-carboxylic acid

11-(2). 7-[2-chloromethylene-2-(2-aminothiazol-4-yl)acetamido]-3-(1-methyl-1H-tetrazol-5-yl)thiomethyl-3-cephem-4-carboxylic acid

11-(3). 7-[2-chloromethylene-2-(2-aminothiazol-4-yl)acetamido]-3-(1-methyl-1H-tetrazol-5-yl)thiomethyl-3-cephem-4-carboxylic acid sodium salt

12. 7-[2-chloromethylene-2-(2-tert-butoxycarbonylaminothiazol-4-yl)acetamido]-3-acetoxymethyl-3-cephem-4-carboxylic acid

13. 7-[2-chloromethylene-2-(2-tert-butoxycarbonylaminothiazol-4-yl)acetamido]-3-(2-methylthio-1,3,4-thiadiazol-5-yl) thiomethyl-3-cephem-4-carboxylic acid

14. 7-[2-chloromethylene-2-(2-tert-butoxycarbonylaminothiazol-4-yl)acetamido]-3-[(2,5-dihydro-6-hydroxy-2-methyl-5-oxo-as-triazin-3-yl)thiomethyl]-3-cephem-4-carboxylic acid

15. 7-[2-chloromethylene-2-(2-tert-butoxycarbonylaminothiazol-4-yl)acetamido]desacetoxycephalosporanic acid

16. 7-[2-chloromethylene-2-(2-aminothiazol-4-yl)acetamido]-3-acetoxymethyl-3-cephem-4-carboxylic acid

17. 7-[2-chloromethylene-2-(2-aminothiazol-4-yl)acetamido]-3-(2-methylthio-1,3,4-thiadiazol-5-yl)thiomethyl-3-cephem-4-carboxylic acid

18. 7-[2-chloromethylene-2-(2-aminothiazol-4-yl)acetamido]-3-[(2,5-dihydro-6-hydroxy-2-methyl-5-oxo-as-triazin-3-yl)thiomethyl-3-cephem-4-carboxylic acid

19. 7-[2-chloromethylene-2-(2-aminothiazol-4-yl)acetamido]desacetoxycephalosporanic acid

20. 7-[2-chloromethylene-2-(2-aminothiazol-4-yl)acetamido]-3-acetoxymethyl-3-cephem-4-carboxylic acid sodium salt

21. 7-[2-chloromethylene-2-(2-aminothiazol-4-yl)acetamido]-3-(2-methylthio-1,3,4-thiadiazol-5-yl)thiomethyl-3-cephem-4-carboxylic acid sodium salt

22. 7-[2-chloromethylene-2-(2-aminothiazol-4-yl)acetamido]-3-[(2,5-dihydro-6-hydroxy-2-methyl-5-oxo-as-triazin-3-yl)thiomethyl-3-cephem-4-carboxylic acid disodium salt

23. 7-[2-chloromethylene-2-(2-aminothiazol-4-yl)acetamido]-desacetoxycephelosporanic acid sodium salt

24. 7-[2-chloromethylene-2-(2-aminothiazol-4-yl)acetamido]-3-pyridiniummethyl-3-cephem-4-carboxylic acid betaine

25-(2). 7-[2-chloromethylene-2-(2-formylaminothiazol-4-yl)acetamido]-3-(1-methyl-1H-tetrazol-5-yl)thiomethyl-3-cephem-4-carboxylic acid

25-(3). 7-[2-chloromethylene-2-(2-aminothiazol-4-yl)acetamido]-3-(1-methyl-1H-tetrazol-5-yl)thiomethyl-3-cephem-4-carboxylic acid

26. 7-[2-chloromethylene-2-(2-aminothiazol-4-yl)acetamido]-3-(1-methyl-1H-tetrazol-4-yl)thiomethyl-3-cephem-4-carboxylic acid sodium salt

27. 7-[2-dichloromethylene-2-(4-methoxyphenyl)acetamido]-3-cephem-4-carboxylic acid

28. 7-[2-dichloromethylene-2-(4-methylphenyl)acetamido]-3-acetoxymethyl-3-cephem-4-carboxylic acid

29. 7-[2-dichloromethylene-2-(4-chlorophenyl)acetamido]-3-acetoxymethyl-3-cephem-4-carboxylic acid

30. 7-[2-dichloromethylene-2-(3,4-dichlorophenyl)acetamido]-3-acetoxymethyl-3-cephem-4-carboxylic acid

31. 7-[2-dichloromethylene-2-(5-chloro-2-thienyl)acetamido]-3-acetoxymethyl-3-cephem-4-carboxylic acid

32. 7-[2-dichloromethylene-2-(5-bromo-2-thienyl)acetamido]-3-acetoxymethyl-3-cephem-4-carboxylic acid

33. 7-[2-dichloromethylene-2-(2-furyl)acetamido]-3-acetoxymethyl-3-cephem-4-carboxylic acid

34. 7-[2-chloromethylene-2-(2-thienyl)acetamido]-3-(1-methyl-1H-tetrazol-5-yl)thiomethyl-3-cephem-4-carboxylic acid

35. 7-[2-dichloromethylene-2-(3-thienyl)acetamido]-3-(1-methyl-1H-tetrazol-5-yl)thiomethyl-3-cephem-4-carboxylic acid

36. 7-[2-dichloromethylene-2-(1-methyl-2-pyrrolyl)acetamido]-3-acetoxymethyl-3-cephem-4-carboxylic acid

37. 6-[2-dichloromethylene-2-(2-thienyl)acetamido]penicillanic acid

38. 7-[2-dichloromethylene-2-phenylacetamido)-3-acetoxymethyl-3-cephem-4-carboxylic acid

39. 7-[2-dichloromethylene-2-(2-thienyl)acetamido]-3-(1-methyl-1H-tetrazol-5-yl)thiomethyl-3-cephem-4-carboxylic acid

40-(1). 7-[2-dichloromethylene-2-(3-thienyl)acetamido]-3-acetoxymethyl-3-cephem-4-carboxylic acid

40-(2). 7-[2-dichloromethylene-2-(2-thienyl)acetamido]-3-acetoxymethyl-3-cephem-4-carboxylic acid

41. 6-[2-chloromethylene-2-(2-thienyl)acetamido]penicillanic acid

42. 7-[2-dichloromethylene-2-(2-aminothiazol-4-yl)acetamido]-3-carbamoyloxymethyl-3-cephem-4-carboxylic acid

43. 7-[2-dichloromethylene-2-(aminothiazol-4-yl)acetamido]-3-carbamoyloxymethyl-3-cephem-4-carboxylic acid

The compopounds of the present invention can be prepared by reacting a compound having the general formula II:

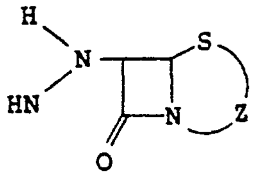

II

(in which Z has the same meaning as described above), a reactive derivative of the compound with respect to the amino radical or a salt thereof, with an acid having the general formula III:

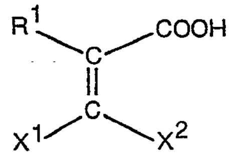

III

(in which $R^1$, $X^1$ and $X^2$ have the same meanings as defined above), a reactive derivative of the acid with respect to the carboxyl radical or a salt thereof.

The compounds represented by general formula III, which are used as raw materials for the preparation of the compounds of the present invention, can be prepared, for example, by reacting a glyoxylic acid derivative substituted by an aromatic group, with a Wittig reagent or by reacting a 2-formyl acetic acid derivative substituted by an aromatic group, with a halogenating agent, as described below.

The acids represented by general formula II, which are used as raw materials for the preparation of the compounds according to the present invention, are easily available commercially.

Embodiments of the process for preparing the compounds according to the present invention will now be described below. When the compounds represented by general formula III are to be used as a reactant in the form of free acids (or in the form of salts thereof), it is normally preferable that the reaction be carried out in the presence of a dehydrating or condensing agent such as N,N'-dicyclohexylcarbodiimide, N-cyclohexyl-N'-morpholinoethylcarbodiimide, N,N'-diethylcarbodiimide, N,N'-carbonyl(2-methylimidazole), trialkyl phosphate, polyphosphoric acid ethyl ester, phosphorus oxychloride, phosphorus trichloride, 2-chloro-1,3,2-dioxaphospholane, oxalyl chloride, etc. Examples of the salts of the compounds represented by general formula III include alkaline metal salts, alkaline earth metal salts, ammonium salts, and salts with organic bases such as triethylamine, dicyclohexylamine, etc.

As the reactive derivatives of the compounds represented by general formula III, those reactive derivatives which are usually used in the synthesis of acid amides can be used. Examples of such reactive derivatives include acid halides, acid anhydrides, mixed acid anhydrides formed between organic or inorganic acids, active acid amides, acid cyanides, active esters, acid azides, etc. of the compounds represented by general formula III. Acid chlorides, mixed acid anhydrides and active amides are particularly preferred.

Examples of the mixed acid anhydrides include those of aliphatic carboxylic acids, aromatic carboxylic acids, alkylcarbonic acids, aralkylcarbonic acids, dialkylphosphoric acids, diphenylphosphoric acids, methanesulfonic acid, p-toluenesulfonic acid and the like. The active esters may be, for example, cyanomethyl esters, substituted phenyl esters, substituted benzyl esters, substituted thienyl esters, etc. The active acid amides may be, for example, imidazole, 4-substituted imidazole, dimethylpyrazole, triazole, tetrazole, saccharin and the like.

The compounds represented by general formula II are subjected to the reaction in the form of free form or in the form of salts or esters thereof. The salts of the compounds represented by general formula II may be, for example, salts with organic acids such as acetates, benzenesulfonates, tartartes, trifluoroacetates, and formates; salts with inorganic acids such as hydrochlorides, hydrobromides, sulfates, and phosphates; alkaline metal salts such as sodium salts and potassium salts; and salts with organic bases such as trimethylamine salts, triethylamine salts and dibutylamine salts. Examples of the esters of the compounds represented by general formula II include benzhydryl esters, trichloroethyl esters, p-nitrobenzyl esters, alkylsilyl esters, etc.

Usually, the above-described reaction for preparing the compounds according to the present invention is carried out in the presence of a solvent. The solvents may be any general organic solvents that do not participate in the reaction, such as dimethyl sulfoxide, methyl alcohol, ethyl alcohol, methoxyethanol, diethyl ether, isopropyl ether, benzene, toluene, methylene chloride, chloroform, ethyl acetate, methyl isobutyl ketone, etc. These solvents may be used in admixture with water. In addition, the reaction may be carried out in the presence of a base. Examples of such bases include inorganic bases such as caustic alkalis, alkali carbonates, alkali bicarbonates, and alkali acetates; tertiary amines such as trimethylamine, triethylamine, tributylamine, pyridine, N-methylpiperidine, N-methylmorpholine, lutidine and uridine; secondary amines such as diethylamine, and dicyclohexylamine, etc.

The reaction may be carried out at any temperature. Normally, however, it is conducted with cooling or at room temperature.

The β-lactam compounds of the present invention prepared in the manner described above may then be purified by a method known to the art, for example, by column chromatography, extraction, precipitation, recrystallization, etc. When necessary, the obtained β-lactam compounds may be converted into a desired salt or an ester by a procedure known to the art.

The present invention further provides novel intermediate products (2-halomethylene aromatic acetic acid derivatives) having the following general formula IV:

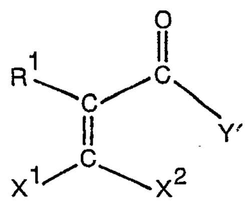

IV

in which $R^1$, $X^1$ and $X^2$ have the same meanings defined above provided $R^1$ is not phenyl or substituted phenyl, and Y denotes a hydroxyl group, a halogen atom or a radical —OR wherein R stands for a protective radical for the carboxylic acid. General formula IV embraces the compounds represented by the aforedescribed general formula III.

R of the radical —OR represented by Y in general formula IV may be any of the protective radicals that are usually used for carboxylic acids, such as alkyl, aryl, aralkyl, trialkylsilyl, alkoxycarbonyl, aryloxycarbonyl, aralkyloxycarbonyl, etc. Furthermore, at least one of $X^1$ and $X^2$ is a halogen atom. Of course, this means that both $X^1$ and $X^2$ may denote the same halogen atom. Alternatively, one of $X^1$ and $X^2$ may represent hydrogen, and the other a halogen atom. Further, $X^1$ and $X^2$ may represent different halogen atoms.

The intermediate compounds having general formula IV in which Y denotes radical —OR wherein R stands for a protective radical for carboxylic acid can be prepared, for example, by (A) reacting a glyoxylic acid derivative substituted by an aromatic group, with a Wittig reagent, or (B) reacting a 2-formylacetic acid derivative substituted by an aromatic group, with a halogenating agent. The 2-halomethylene aromatic acetic acids having general formula IV in which Y denotes a hydroxyl group can be obtained easily by subjecting the compounds prepared by the method (A) or (B) described above to usual hydrolytic conditions. The 2-halomethylene aromatic acetic acid halides having general formula IV in which Y denotes a halogen atom can be prepared by reacting the aforesaid 2-halomethylene aromatic acetic acids or salts thereof with a halogenating agent, for example, phosphorus pentachloride, thionyl chloride or oxalyl chloride.

The preparative methods (A) and (B) are described below in more detail.

(A)

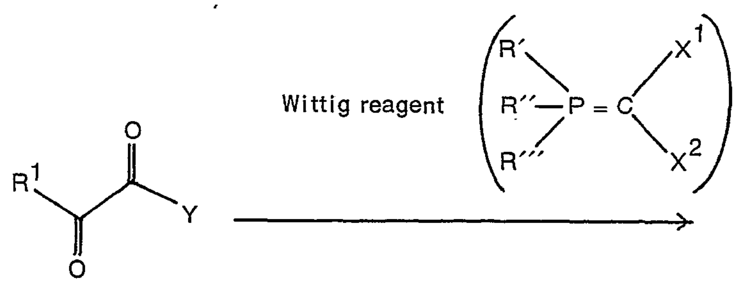


IV

wherein $R^1$, Y, $X^1$ and $X^2$ have the same meanings as defined above; R′, R″ and R‴ may be the same or different and each represent an aryl radical or an alkyl radical, preferably a phenyl radical, most preferably R′, R″ and R‴ being a phenyl radical at the same time.

(B)

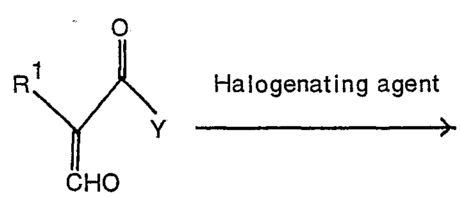


IV

wherein $R^1$ and Y have the same meanings as defined above.

The reaction scheme (A) shows the reaction between a glyoxylic acid derivative substituted by an aromatic group and a Wittig reagent (for example, A. J. Speziale, Org. Syn. Coll. Vol. V, 361). The glyoxylic acid derivatives substituted by an aromatic group used as raw materials can easily be obtained, for

example, by the oxidation of acetic acid derivatives substituted by an aromatic group. The Wittig reagent used can also be easily prepared from dihalomethanes or trihalomethanes and triphenylphosphine. When carrying out the reaction according to scheme (A), it is preferable to use a solvent such as hydrocarbons, e.g. hexane, heptane, benzene and toluene; ethers, e.g. diethyl ether, tetrahydrofuran, and dimethoxyethane; and polar solvents, e.g. dimethylformamide, dimethyl sulfoxide and hexamethylphosphoric triamide. The reaction may be conducted at a temperature ranging from −78°C to the reflux temperature of the solvent used. To obtain the desired compound at a high yield, it is preferred to use a temperature ranging from 0°C to room temperature.

The above reaction scheme (B) represents the reaction between a 2-formylacetic acid derivative substituted by an aromatic group and a halogenating agent [for example, W. Wislicenus, et al., Ber., *51,* 1366 (1918)]. The 2-formylacetic acid derivatives substituted by an aromatic group used as raw materials can easily be prepared by the condensation reaction of the acetic acid derivatives substituted by an aromatic group with formic acid esters in the presence of a base [W. Wislicenus, Ann., 413, 205 (1917)]. In this way, 2-formylacetic acid derivatives substituted by an aromatic group such as 2-formyl-substituted-phenylacetic acids, 2-formyl-(substituted or unsubstituted)-thienylacetic acids, 2-formyl-(substituted or unsubstituted)-furylacetic acids, 2-formyl-(substituted or unsubstituted-thiazolacetic acids, etc. can be obtained. The halogenating agent used may be, for example, phosphorus pentachloride, phosphorus oxychloride, thionyl chloride, phosphorus trichloride, phosgene, and the Vilsmeier reagent prepared from these halogenating agents and dimethylformamide. When carrying out the reaction according to scheme (B), it is preferable to use a solvent. The solvent may be, for example, halogen-containing solvents such as chloroform and methylene chloride, in addition to the solvents described above with reference to reaction scheme (A). The reaction may be conducted at a temperature ranging from −78°C to the reflux temperature of the solvent used. To obtain the desired compound at a high yield, it is preferable to carry out the reaction at a temperature ranging from room temperature to about 100°C.

The above-described method (A) can give the intermediate compounds having general formula IV in which both $X^1$ and $X^2$ denote halogen atom(s) or one of $X^1$ and $X^2$ denotes a halogen atom. The method (B) can yield the intermediate compounds having general formula IV in which one of $X^1$ and $X^2$ dentoes a halogen atom. The intermediate compounds having general formula IV in which $X^1$ and $X^2$ denote different atoms involve structural isomers. These structural isomers can be separated by a procedure normally used to separate structural isomers, such as distillation, chromatography, fractional recrystallization, etc.

By the methods described above, the following compounds embraced in general formula IV can be prepared: 2 - diahalomethylene - 2 - phenylacetic acid, 2 - dihalomethylene - 2 - (substituted phenyl)acetic acids, such as 2 - dichloromethylene - 2 - (4 - chlorophenyl)acetic acid, 2 - dichloromethylene - 2 - (4 - hydroxyphenyl)acetic acid, 2 - dichloromethylene - 2 - (3 - hydroxyphenyl)acetic acid, 2 - dichloromethylene - 2 - (4 - aminophenyl)acetic acid, 2 - dichloromethylene - 2 - (4 - tert - butoxycarbonylaminophenyl)acetic acid, 2 - dichloromethylene - 2 - (4 - tert - butoxycarbonylaminophenyl)acetic acid methyl ester, 2 - dichloromethylene - 2 - (4 - methoxyphenyl)acetic acid, 2 - dichloromethylene - 2 - (4 - methylphenyl)acetic acid and 2 - dichloromethylene - 2 - (4 - chlorophenyl)acetic acid, 2 - dichloromethylene - 2 - (3,4 - dichlorophenyl)acetic acid; 2 - mono and dihalomethylene - 2 - (substituted or unsubstituted)naphthylacetic acids such as 2 - chloromethylene - 2 - (1 - naphthyl)acetic acid and 2 - dichloromethylene - 2 - (1 - naphthyl)acetic acid; 2 - mono and dihalomethylene - 2 - (substituted or unsubstituted)thienylacetic acid such as 2 - chloromethylene - 2 - (2 - thienyl)acetic acid, 2 - chloromethylene - 2 - (2 - thienyl)acetic acid tert butyl ester, 2 - chloromethylene - 2 - (3 - thienyl)acetic acid, 2 - dichloromethylene - 2 - (2 - thienyl)acetic acid tert-butyl ester, 2 - dichloromethylene - 2 - (2 - thienyl)acetic acid, 2 - dichloromethylene - 2 - (3 - thienyl)acetic acid, 2 - chloromethylene - 2 - (5 - chloro - 2 - thienyl)acetic acid, 2 - dichloromethylene - 2 - (5 - chloro - 2 - thienyl)acetic acid and 2 - dichloromethylene - 2 - (5 - bromo - 2 - thienyl)acetic acid; 2 - mono and dihalomethylene - 2 - (substituted or unsubstituted)furylacetic acids such as 2 - chloromethylene - 2 - (2 - furyl)acetic acid, 2 - dichloromethylene - 2 - (2 - furyl)acetic acid, 2 - dichloromethylene - 2 - (2 - furyl)acetic acid tert-butyl ester, 2 - dichloromethylene - 2 - (3 - furyl)acetic acid and 2 - chloromethylene - 2 - (3 - furyl)acetic acid; 2 - mono and dihalomethylene - 2 - (substituted or unsubstituted)pyridylacetic acids such as 2 - dichloromethylene - 2 - (2 - pyridyl)acetic acid and 2 - chloromethylene - 2 - (2 - pyridyl)acetic acid; 2 - mono and dihalomethylene - 2 - (substituted and unsubstituted)benzothienylacetic acids such as 2 - dichloromethylene - 2 - (2 - benzothienyl)acetic acid and 2 - chloromethylene - 2 - (2 - benzothienyl)acetic acid, 2 - mono and dihalomethylene - 2 - (substituted or unsubstituted)benzofuranylacetic acids such as 2 - dichloromethylene - 2 - (2 - benzofuranyl)acetic acid and 2 - chloromethylene - 2 - (3 - benzofuranyl)-acetic acid; 2 - mono and dihalomethylene - 2 - (substituted or unsubstituted)thiazolylacetic acids such as 2 - dichloromethylene - 2 - (2 - tert - butoxycarbonylaminothiazol - 4 - yl)acetic acid ethyl ester, 2 - dichloromethylene - 2 - (2 - tert - butoxycarbonylaminothiazol - 4 - yl)acetic acid, 2 - chloromethylene - 2 - (2 - tert - butoxycarbonylaminothiazol - 4 - yl)acetic acid ethyl ester, 2 - chloromethylene - 2 - (2 - tert - butoxycarbonylaminothiazol - 4 - yl)acetic acid, 2 - chloromethylene - 2 - (2 - aminothiazol - 4 - yl)acetic acid ethyl ester, 2 - chloromethylene - 2 - (2 - aminothiazol - 4 - yl)acetic acid, 2 - chloromethylene - 2 - (2 - formylaminothiazol - 4 - yl)acetic acid ethyl ester, 2 - chloromethylene - 2 - (2 - formylaminothiazol - 4 - yl)acetic acid and 2-dichloromethylene - 2 - (2 - aminothiazol - 4 - yl)acetic acid, 2 - dichloromethylene - 2 - (2 - aminothiazol - 5 - yl)acetic acid, 2 - chloromethylene - 2 - (2 -

aminothiazol - 5 - yl)acetic acid; 2 - mono and dihalomethylene - (substituted or unsubstituted) pyrimidylacetic acids, 2 - mono and dihalomethylene - (substituted or unsubstituted) thiadiazolylacetic acids, 2 - mono and dihalomethylene - (substituted or unsubstituted) diazolylacetic acids, 2 - mono and dihalomethylene - (substituted or unsubstituted) triazolylacetic acids, 2 - mono and dihalomethylene - (substituted or unsubstituted) tetrazolylacetic acids, 2 - mono and dihalomethylene - (substituted or unsubstituted) thiatriazolylacetic acids, 2 - mono and dihalomethylene - (substituted or unsubstituted) oxazolylacetic acids, 2 - mono and dihalomethylene - (substituted or unsubstituted) oxadiazolylacetic acids, 2 - mono and dihalomethylene - (substituted or unsubstituted) benzimidazolylacetic acids, 2 - mono and dihalomethylene - (substituted or unsubstituted) benzoxazolylacetic acids (and halides and esters of these 2 - mono and dihalomethylene aromatic acetic acids).

The present invention will be explained in more detail by way of the following Examples, Synthesis Examples and Test Example, which however should not be construed to limit the present invention.

Synthesis Example 1

(1) 2-Furylglyoxylic acid tert-butyl ester (8.2 g, 41.8 mmol) and triphenylphosphine (21.9 g, 84 mmol) were dissolved in carbon tetrachloride (66 ml), and the resulting solution was heated under reflux for 7 hours. After the reaction was completed, the reaction mixture was concentrated under reduced pressure to afford a brownish-black oily residue. This residue was extracted with hexane. The hexane was distilled off from the obtained extract to yield 2-dichloromethylene-2-(2-furyl)acetic acid tert-butyl ester (6.8 g, 85%).

IR (oil): 1730 $cm^{-1}$.

NMR δ ppm ($CDCl_3$): 1.56 (s, 9H), 6.50 (m, 1H), 6.83 (m, 1H), 7.46 (m, 1H).

(2) 2-Dichloromethyl-2-(2-furyl)acetic acid tert-butyl ester (2.00 g, 7.6 mmol) was dissolved in acetic acid (40 ml), 6N hydrochloric acid (5 ml) was added thereto, and the resulting mixture was stirred for 2.5 hours. The reaction mixture was poured into water and extracted with ether for several times. The obtained ethereal extract was washed with water containing sodium chloride, dried over anhydrous, sodium sulfate, and treated with activated carbon. Then, the solvent was distilled off to yield 2-dichloromethylene-2-(2-furyl)acetic acid (1.2 g, 76%).

IR (KBr disk): 1727 $cm^{-1}$.

NMR δ ppm ($CDCl_3$): 6.50 (q, 1H), 6.88 (d, 1H), 7.55 (d, 1H), 9.90 (s, 1H).

Example 1

Anhydrous, dimethylformamide (0.45 g, 6.28 mmol) and phosphorus oxychloride (0.96 g, 6.28 mmol) were added to anhydrous ethyl acetate (2 ml), and the Vilsmeier's reagent was prepared according to a conventional method. The obtained reaction mixture was cooled to 0° to 5°C, and a solution of 2-dichloromethylene-2-(2-furyl)acetic acid (1.00 g, 5.71 mmol) in dry ethyl acetate (10 ml) was added dropwise to the reaction mixture with stirring. The mixture was stirred at that temperature for further one hour.

On the other hand, 7-amino-3-(1-methyl-1H-tetrazol-5-yl)thiomethyl-3-cephem-4-carboxylic acid (1.87 g, 5.71 mmol) was suspended in anhydrous ethyl acetate (20 ml) and bis(trimethylsilyl)acetamide (1.39 g, 6.85 mmol) was added. This mixture was stirred for 1 hour at room temperature, and then cooled to −10°C. To this mixture was added dropwise the previously prepared reaction mixture described above, and stirring was continued for 1.5 hours at that temperature. Water (30 ml) was added to the reaction mixture. The pH-value of this mixture was adjusted to 7.5 to 8.0 by the addition of aqueous sodium hydrogencarbonate, and the ethyl acetate phase was separated. The aqueous phase was adjusted to pH 2 with a 10% hydrochloric acid and extracted thrice with ethyl acetate (30 ml). The combined ethyl acetate phase and extracts were washed with aqueous sodium chloride and dried over anhydrous sodium sulfate. The solvent was distilled off to give 7 - [2 - dichloromethylene - 2 - (2 - furyl)acetamido] - 3 - (1 - methyl - 1H - tetrazol - 5 - yl)thiomethyl - 3 - cephem - 4 - carboxylic acid (0.82 g, 30%).

Melting point: 111—114°C.

NMR δ ppm ($d_6$-acetone): 3.73 ($q_{AB}$, J=18Hz, 2H), 3.92 (s, 3H), 4.35 ($q_{AB}$, J=18Hz, 2H), 5.17 (d, J=4.5Hz, 1H), 5.87 (dd, J=4.5Hz, 9Hz, 1H), 6.53 (m, 1H), 6.83 (m, 1H), 7.61 (d, J=1.5Hz, 1H).

Elemental Analysis (for $C_{17}H_{14}Cl_2N_6O_5S_2$).

| | | | |
|---|---|---|---|
| Calculated: | C: 39.47; | H: 2.73; | N: 15.46. |
| Found: | C: 39.36; | H: 2.94; | N: 15.72. |

Synthesis Example 2

By following the procedure of Synthesis Example 1 but substituting 4-hydroxyphenylacetic acid tert-butyl ester for 2-furylglyoxylic acid tert-butyl ester, 2-dichloromethylene-2-(4-hydroxyphenyl)acetic acid was prepared.

Melting point: 147—150°C.

IR (KBr): $1695^{-1}$.

NMR δ ppm ($CDCl_3$): 6.89 (d, 2H), 7.36 (d, 2H), 9.50 (bs, 2H).

Example 2

Phosphorus oxychloride (0.97 g, 6.32 mmol) was added to ice-cooled anhydrous dimethylformamide (0.46 g, 6.32 mmol), and the mixture was stirred for 30 minutes, followed by stirring at room temperature

for further 1 hour. To the resulting reaction mixture was added anhydrous ethyl acetate (3 ml), and the mixture was cooled to 0° to 5°C. A solution of 2-dichloromethylene-2-(4-hydroxyphenyl)acetic acid (1.34 g, 5.75 mmol) in anhydrous ethyl acetate (12 ml) was added dropwise to the above reaction mixture, and the resulting mixture was stirred at that temperature for 1 hour.

On the other hand, 7-aminocephalosporanic acid (1.72 g, 6.32 mmol) ws suspended in chloroform (20 ml), hexamethyldisilazane (5.08 g, 31.6 mmol) was added, and the mixture was heated under reflux for 1.5 hours. After completion of the reaction, the chloroform and excess hexamethyldisilazane were distilled off under reduced pressure. To the obtained residue was added anhydrous ethyl acetate (20 ml), and the mixture was cooled to 0° to 5°C. Then, the previously prepared reaction mixture described above was added dropwise thereto, and the resulting mixture was stirred at that temperature for 2 hours. To this reaction mixture was added ice-water (40 ml), the pH-value was adjusted to 7.5 to 8.0 by the addition of aqueous sodium hydrogencarbonate, and the ethyl acetate phase was separated. The aqueous phase was adjusted to pH 2 with a 10% hydrochloric acid and the mixture was extracted with ethyl acetate (30 ml × 3). The combined ethyl acetate phase and extracts were washed with aqueous sodium chloride, and dried over anhydrous sodium sulfate. The solvent was distilled off to yield 7-[2-dichloromethylene-2-(4-hydroxyphenyl)acetamido]-3-acetoxymethyl-3-cephem-4-carboxylic acid (2.11 g, 76%).

Melting point: 142—148°C (dec.).

NMR δ ppm ($d_6$-acetone): 2.00 (s, 3H), 3.56 ($q_{AB}$, J=18Hz, 2H), 4.92 ($q_{AB}$, J=12Hz, 2H), 5.20 ($q_{AB}$, J=4.5Hz, 1H), 5.83 (dd, J=4.5Hz, 9Hz, 1H), 6.83 (d, J=9Hz, 2H) 7.36 (d, J=9Hz, 2H).

Elemental Analysis (for $C_{19}H_{16}Cl_2N_2O_7S$).

| | | | |
|---|---|---|---|
| Calculated: | C: 46.83; | H: 3.31; | N: 5.75. |
| Found: | C: 46.47; | H: 3.38; | N: 5.59. |

Synthesis Example 3

By following the procedure of Synthesis Example 2 but substituting 3-hydroxyphenylacetic acid tert-butyl ester for 4-hydroxyphenylacetic acid tert-butyl ester, 2-dichloromethylene-2-(3-hydroxyphenyl)acetic acid was prepared.

IR (oil): 1700 $cm^{-1}$.

NMR δ ppm ($CDCl_3$): 6.79 (m, 3H), 7.10 (m, 1H), 8.33 (bs, 2H).

Example 3

By following the procedure of Example 2 but substituting 2-dichloromethylene-2-(3-hydroxyphenyl)-acetic acid for 2-dichloromethylene-2-(4-hydroxyphenyl)acetic acid, 7-[2-dichloromethylene-2-(3-hydroxyphenyl)acetamido]-3-acetoxymethyl-3-cephem-4-carboxylic acid was prepared.

Melting point: 126—133°C (dec.).

NMR δ ppm ($d_6$-acetone): 2.00 (s, 3H), 3.53 ($q_{AB}$, J=18Hz, 2H), 4.90 ($q_{AB}$, J=15Hz, 2H), 5.17 ($q_{AB}$, J=4.5Hz, 1H), 5.83 (dd, J=4.5Hz, 9Hz, 1H), 6.23 (bs, 2H), 6.79—7.30 (m, 4H),8.56 (d, J=9Hz).

Elemental Analysis (for $C_{19}H_{16}Cl_2N_2O_7S$).

| | | | |
|---|---|---|---|
| Calculated: | C: 46.83; | H: 3.31; | N: 5.75. |
| Found: | C: 45.00; | H: 3.45; | N: 5.09. |

Synthesis Example 4

(1) Potassium (1.28 g, 33 mmol) was added to anhydrous tert-butyl alcohol (30 ml) under argon atmosphere, and the mixture was heated under reflux for 1 hour to prepare a potassium tert-butoxide solution. Then, potassium tert-butoxide was obtained according to the conventional method, and anhydrous heptane (120 ml) was added to form a slurry. To the ice-cooled slurry was added triphenylphosphine (8.64 g, 33 mmol) and, then, a solution of chloroform (3.94 g, 33 mmol) dissolved in anhydrous heptane (30 ml) was added dropwise over 1 hour with stirring. After completion of the dropwise addition, the reaction mixture was concentrated under reduced pressure to 50 ml. A solution of 4-tert-butoxycarbonylaminophenylglyoxylic acid methyl ester (4.62 g, 165 mmol) in benzene (20 ml) was added to the resulting concentrate under ice-cooling, and the mixture was stirred for 30 minutes and, then, stirred at room temperature for 1 hour. Then, ethyl acetate was added to this reaction mixture, and then resulting mixture was washed with water. The organic phase was dried over anhydrous sodium sulfate, and the solvent was distilled off to afford an oily residue. Purification of the oily residue by column chromatography yielded 2-dichloromethylene-2-(4-tert-butoxycarbonylaminophenyl)acetic acid methyl ester (3.92 g, 69%).

Melting point: 80—82°C.

NMR δ ppm ($CDCl_3$): 1.53 (s, 9H), 3.76 (s, 3H), 6.59 (bs, 1H), 7.23 (d, 2H), 7.36 (d, 2H).

(2) 2-Dichloromethylene-2-(4-tert-butoxycarbonylaminophenyl)acetic acid methyl ester (2.0 g, 5.78 mmol) was dissolved in a 30% aqueous ethyl alcohol (45 ml), 1 N sodium hydroxide (12 ml) was added thereto, and the mixture was stirred at room temperature for 15 hours. The reaction mixture was concentrated, and water (10 ml) was added to the obtained residue. This residue was extracted with ethyl acetate (10 ml × 2). The aqueous phase was separated and adjusted to pH 2 with a 10% hydrochloric acid. Then, the aqueous phase was extracted with ethyl acetate (20 ml × 3). The combined extracts were washed with aqueous sodium chloride and dried over anhydrous sodium sulfate. The solvent was distilled off to yield 2-dichloromethylene-2-(4-tert-butoxycarbonylaminophenyl)acetic acid (1.86 g, 97%) as white crystals.

Melting point: 149—151°C (dec.).

NMR δ ppm ($CDCl_3$): 1.53 (s, 9H), 6.96 (bs, 1H), 7.30 (d, 2H), 7.40 (d, 2H), 7.89 (bs, 1H).

Example 4

(1) By following the procedure of Example 2 but substituting 2-dichloromethylene-2-(4-tert-butoxycarbonylaminophenyl)acetic acid for 2-dichloromethylene-2-(4-hydroxyphenyl)acetic acid, 7-[2-dichloromethylene-2-(4-tert-butoxycarbonylaminophenyl)acetamido]-3-acetoxymethyl-3-cephem-4-carboxylic acid was prepared.

Melting point: 155—163°C (dec.).

NMR δ ppm ($d_6$-acetone): 1.50 (s, 9H), 2.00 (s, 3H, 3.50 ($q_{AB}$, J=18Hz, 2H), 4.83 ($q_{AB}$, K=12Hz, 2H), 5.13 (d, J=4.5Hz, 1H), 5.68 (dd, J=4.5Hz, J=9Hz, 1H), 7.33 (d, J=9Hz, 2H), 7.46 (d, J=9Hz, 2H), 9.40 (s, 1H), 9.71 (d, J=9Hz, 1H).

Elemental Analysis (for $C_{24}H_{25}Cl_2N_3O_8S$).

Calculated: C: 49.15; H: 4.30; N: 7.17.

Found: C: 48.28; H: 4.18; N: 7.05.

2. 7 - [2 - Dichloromethylene - 2 - (4 - tert - butoxycarbonylaminophenyl)acetamido] - 3 - acetoxymethyl - 3 - cephem - 4 - carboxylic acid (0.26 g, 0.44 mmol) was dissolved in anisole (6 ml), and the resulting solution was cooled to 0° to 5°C. Trifluoroacetic acid (4 ml) was added to this solution, and the resulting mixture was stirred for 3 hours. The reaction mixture was concentrated under reduced pressure, and the residue was poured into a solution of ether and n-hexane (1:1). The obtained fine precipitate was collected by filtration, washed with a solution of ether and n-hexane (1:1), and dried over phosphor pentoxide under reduced pressure to yield 7 - [2 - dichloromethylene - 2 - (4 - aminophenyl)-acetamido] - 3 - acetoxymethyl - 3 - cephem - 4 - carboxylic acid (0.11 g, 52%).

Melting point: 164—171°C (dec.).

NMR δ ppm ($d_6$-acetone): 2.00 (s, 3H), 3.53 ($q_{AB}$, J=18Hz, 2H), 4.91 ($q_{AB}$, J=12Hz, 2H), 5.16 (d, J=4.5Hz, 1H), 5.84 (dd, J=4.5 Hz, J=9Hz, 1H), 6.63 (d, J=9Hz, 2H), 6.96 (d, J=9Hz, 1H), 7.21 (d, J=9Hz, 2H).

Elemental Analysis (for $C_{19}H_{17}Cl_2N_3O_6S$).

Calculated: C: 46.93; H: 3.52; N: 8.64.

Found: C: 45.38; H: 3.52; N: 8.07.

Synthesis Example 5

(1) By following the procedure of Synthesis Example 4-(1) but substituting 2 - tert - butoxycarbonylaminothiazol - 4 - yl - glyoxylic acid ethyl ester for 4 - tert - butoxycarbonylaminophenylglyoxylic acid methyl ester, 2-dichloromethylene - 2 - (2 - tert - butoxycarbonylaminothiazol - 4 - yl)acetic acid ethyl ester was prepared.

Melting point: 93—97°C.

NMR δ ppm ($CDCl_3$): 1.23 (t, 3H), 1.50 (s, 9H), 4.23 (q, 2H), 7.36 (s, 1H).

(2) By following the procedure of Synthesis Example 4-(2) but using 2-dichloromethylene-2-(2-tert-butoxycarbonylaminothiazol-4-yl)acetic acid ethyl ester as a raw material, 2-dichloromethylene-2-(2-tert-butoxycarbonylaminothiazol-4-yl)acetic acid was prepared.

Melting point: 195—196°C.

NMR δ ppm ($d_6$-DMSO): 1.46 (s, 9H), 5.00 (bs, 1H), 7.50 (s, 1H), 11.56 (bs, 1H).

Example 5

(1) The 2-dichloromethylene-2-(2-tert-butoxycarbonylaminothiazol-4-yl)acetic acid (1.15 g, 3.39 mmol) obtained in Synthesis Example 5-(2) was suspended in methylene chloride (15 ml), and phosphorus pentachloride (0.71 g, 3.4 mmol) was added under ice-cooling. The resulting mixture was stirred at room temperature for 30 minutes, and the solvent was distilled off under reduced pressure. The obtained residue was washed with a mixture of anhydrous heptane and n-hexane (1:1) to afford 2-dichloromethylene-2-(2-tert-butoxycarbonylaminothiazol-4-yl)acetic acid chloride.

On the other hand, 7-aminocephalosporanic acid (0.92 g, 3.4 mmol) was suspended in chloroform (20 ml), hexamethyldisilazane (2.73 g, 17 mmol) was added, and the mixture was heated under reflux for 1.5 hours. After completion of the reaction, the chloroform and excess hexamethyldisilazane were distilled off under reduced pressure. To the obtained residue was added another portion of anhydrous chloroform (15 ml), and the mixture was cooled to 0° to 5°C. Thereafter, the previously prepared 2-dichloromethylene-2-(2-tert-butoxycarbonylaminothiazol-4-yl)acetic acid chloride was dissolved in anhydrous chloroform (10 ml), and this solution was added dropwise to the aforesaid cooled mixture. The resulting reaction mixture was stirred for 2 hours under ice-cooling with ice, and was poured into ice-water (40 ml). Then, the reaction mixture was adjusted to pH 7.5 to 8.0 by the addition of sodium hydrogencarbonate, and extracted with ethyl acetate. The aqueous phase was separated and filtered through Celite. The filtrate was adjusted to pH 2 with a 10% hydrochloric acid and extracted with ethyl acetate (30 ml × 3). The combined extracts were washed with aqueous sodium chloride and dried over anhydrous sodium sulfate. Then, the ethyl acetate phase was treated with activated carbon, and concentrated under reduced pressure to give an amorphous solid. This solid was dissolved in a small portion of ethyl acetate, and the obtained solution was poured into

a mixture of ether and n-hexane (1:1). The resulting precipitate was filtered off, washed with a mixture of ether and n-hexane (1:1), and dried over phosphorus pentoxide under reduced pressure to yield 7 - [2 - dichloromethylene - 2 - (2 - tert - butoxycarbonylaminothiazol - 4 - yl)acetamido] - 3 - acetoxymethyl - 3 - cephem - 4 - carboxylic acid (0.41 g, 20%).

Melting point:
140—145°C.

NMR δ ppm ($d_6$-acetone): 1.56 (s, 9H), 2.03 (s, 3H), 3.59 ($q_{AB}$, J=18Hz, 2H), 4.96 ($q_{AB}$, J=12Hz, 2H), 5.23 (d, J=4.5Hz, 1H), 5.91 (dd, J=4.5Hz, J=9Hz, 1H), 7.43 (s, 1H).

Elemental Analysis (for $C_{21}H_{22}Cl_2N_4O_8S_2$).

Calculated: C: 42.50; H: 3.74; N: 9.44.
Found: C: 41.43; H: 3.73; N: 9.11.

(2) By following the procedure of Example 4-(2) but using 7 - [2 - dichloromethylene - 2 - (2 - tert - butoxycarbonylaminothiazol - 4 - yl)acetamido] - 3 - acetoxymethyl - 3 - cephem - 4 - carboxylic acid as a raw material, 7 - [2 - dichloromethylene - 2 - (2 - aminothiazol - 4 - yl)acetamido] - 3 - acetoxymethyl - 3 - cephem - 4 - carboxylic acid was prepared.

Melting point: 137—142°C (dec.).

NMR δ ppm ($d_6$-acetone): 2.00 (s, 3H), 3.56 ($q_{AB}$, J=18Hz, 2H), 4.91 ($q_{AB}$, J=13.5Hz, 2H), 5.16 (d, J=4.5Hz, 1H), 5.86 (dd, J=4.5Hz, 9Hz, 1H), 6.91 (s, 1H).

Elemental Analysis (for $C_{16}H_{14}Cl_2N_4O_6S_2$).

Calculated: C: 38.95; H: 2.86; N: 11.36.
Found: C: 38.23; H: 3.01; N: 10.42.

Example 6

(1) 2-Dichloromethylene-2-(2-tert-butoxycarbonylaminothiazol-4-yl)acetic acid (0.577 g, 1.7 mmol), prepared as described in Synthesis Example 5-(2), was suspended in methylene chloride (10 ml), and phosphorus pentachloride (0.35 g, 1.7 mmol) was added thereto under ice-cooling. The resulting mixture was stirred for 1 hour and, then, stirred at room temperature for further 30 minutes. The reaction mixture was concentrated under reduced pressure to yield 2-dichloromethylene-2-(2-tert-butoxycarbonylaminothiazol-4-yl)acetic acid chloride in a form of crystals.

On the other hand, 7-amino-3-(1-methyl-1H-tetrazol-5-yl)thiomethyl-3-cephem-4-carboxylic acid (0.56 g, 1.7 mmol) was suspended in ethyl acetate (10 ml), and bis(trimethylsilyl) acetamide (0.61 g) was added thereto. The resulting reaction mixture was stirred at 40°C for 2 hours and then cooled with ice. To this reaction mixture was added dropwise a solution of the above obtained 2-dichloromethylene-2-(2-tert-butoxycarbonylaminothiazol-4-yl)acetic acid chloride in methylene chloride (10 ml). The resulting reaction mixture was stirred for 2 hours under ice-cooling, and ice-water (20 ml) was added thereto. The pH-value of the reaction mixture was adjusted to 7.5 by the addition of aqueous sodium hydrogencarbonate, followed by extraction with ethyl acetate (30 ml × 2). The aqueous phase was separated, followed by filtration through Celite. The filtrate was cooled with ice, the pH-value was adjusted to 2.0 by the addition of a 10% hydrochloric acid, and the precipitated crystals were collected by filtration. The crystals were washed with water, and dried over phosphorus pentoxide under reduced pressure to afford 7 - [2 - dichloromethylene - 2 - (2 - tert - butoxycarbonylaminothiazol - 4 - yl)acetamido] - 3 - (1 - methyl - 1H - tetrazol - 5 - yl)thiomethyl - 3 - cephem - 4 - carboxylic acid (0.855 g, 78%).

NMR δ ppm ($d_6$-DMSO): 1.50 (s, 9H), 3.69 ($q_{AB}$, J=18Hz, 2H), 3.92 (s, 3H), 4.33 ($q_{AB}$, J=18Hz, 2H), 5.13 (d, J=6Hz, 1H), 5.73 (dd, J=6Hz, J=9Hz, 1H), 7.43 (s, 1H), 9.73 (d, J=9Hz, 1H).

(2) 7 - [2 - Dichloromethylene - 2 - (2 - tert - butoxycarbonylaminothiazol - 4 - yl)acetamido] - 3 - (1 - methyl - 1H - tetrazol - 5 - yl)thiomethyl - 3 - cephem - 4 - carboxylic acid (0.85 g, 1.31 mmol) was dissolved in anisole (4 ml), and the resulting solution was cooled to 0° to 5°C. Trifluoroacetic acid (15 ml) was added thereto, and the mixture was stirred for 22 hours. The reaction mixture thus obtained was concentrated under reduced pressure, water (20 ml) was added to the residue, and the pH-value was adjusted to 7.5 by the addition of aqueous sodium hydrogencarbonate. This solution was extracted with ether (30 ml) and, then, with a mixed solvent containing ethyl acetate and ether (40 ml, 1:1). The aqueous phase was separated, cooled with ice, and adjusted to pH 2.0 by the addition of a 10% hydrochloric acid. The precipitated crystals were collected by filtration, washed with water, and dried over phosphorus pentoxide under reduced pressure to yield 7 - [2 - dichloromethylene - 2 - (2 - aminothiazol - 4 - yl)-acetamido] - 3 - (1 - methyl - 1H - tetrazol - 5 - yl)thiomethyl - 3 - cephem - 4 - carboxylic acid (0.263 g). The aqueous phase freed from the above crystals was extracted with ethyl acetate (40 ml × 2). The extract was washed with aqueous sodium chloride, and dried over anhydrous sodium sulfate. The solvent was removed under reduced pressure to yield 7 - [2 - dichloromethylene - 2 - (2 - aminothiazol - 4 - yl)-acetamido] - 3 - (1 - methyl - 1H - tetrazol - 5 - yl)thiomethyl - 3 - cephem - 4 - carboxylic acid (0.355 g).

Melting point: 160—165°C. (dec.).

NMR δ ppm ($d_6$-DMSO): 3.66 ($q_{AB}$, J=18Hz, 2H), 3.92 (s, 3H), 4.30 ($q_{AB}$, J=18Hz, 2H), 4.43 (bs, 3H), 5.10 (d, J=6Hz, 1H), 5.70 (dd, J=6Hz, J=7.5Hz, 1H), 6.89 (s, 1H), 9.66 (d, J=7.5Hz, 1H).

Elemental Analysis (for $C_{16}H_{14}Cl_2N_8O_4S_3$).

Calculated: C: 34.98; H: 2.57; N: 20.39.

Found: C: 33.89; H: 2.61; N: 19.85.

(3) 7 - [2 - Dichloromethylene - (2 - aminothiazol - 4 - yl)acetamido] - 3 - (1 - methyl - 1H - tetrazol - 5 - yl)thiomethyl - 3 - cephem - 4 - carboxylic acid (0.355 g) was dissolved in a 28% aqueous ethyl alcohol (14 ml.). The pH-value was adjusted to 7.5 by the addition of sodium hydrogencarbonate. The resulting solution was purified through a column packed with Amberlite XAD—2 to yield 7 - [2 - dichloromethylene - (2 - aminothiazol - 4 - yl)acetamido] - 3 - (1 - methyl - 1H - tetrazol - 5 - yl)thiomethyl - 3 - cephem - 4 - carboxylic acid sodium salt as white powder.

Elemental Analysis (for $C_{16}H_{13}Cl_2N_8O_4S_3Na{\cdot}2H_2O$).

Calculated: C: 31.64; H: 2.82; N: 18.45.

Found: C: 32.28; H: 2.62; N: 17.78.

## Example 7

(1) 2-Dichloromethylene-2-(2-tert-butoxycarbonylaminothiazol-4-yl)acetic acid (0.678 g, 2 mmol), prepared as described in Synthesis Example 5—(2), was suspended in methylene chloride (10 ml.), and phosphorus pentachloride (0.417 g, 2 mmol) was added thereto under ice-cooling. The resulting mixture was stirred for 30 minutes and, then, stirred at room temperature for further 30 minutes. The reaction mixture was concentrated under reduced pressure to yield 2-dichloromethylene-2-(2-tert-butoxycarbonylaminothiazol-4-yl)acetic acid chloride in a form of crystals.

On the other hand, 7 - amino - 3 - (1 - carboxymethyl - tetrazol - 5 - yl)thiomethyl - 3 - cephem - 4 - carboxylic acid (0.744 g, 2 mmol) was suspended in ethyl acetate (10 ml.), and bis(trimethylsilyl)acetamide (1.01 g, 5 mmol) was added. The resulting mixture was stirred at room temperature for 1 hour and then cooled with ice. To this reaction mixture was added dropwise a solution of the above obtained 2 - dichloromethylene - 2 - (2 - tert - butoxycarbonylaminothiazol - 4 - yl)acetic acid chloride in methylene chloride (10 ml.). The resulting reaction mixture was stirred for 2 hours under ice-cooling, and ice-water (20 ml.) was added thereto. The pH-value of the reaction mixture was adjusted to 2.0 by the addition of aqueous sodium hydrogencarbonate, followed by filtration through Celite. The filtrate was extracted with ethyl acetate (30 ml. × 3). The extracts were washed with aqueous sodium chloride (1 ml. × 3), and dried over anhydrous sodium sulfate. The solvent was removed by distillation to yield 7 - [2 - dichloromethylene - 2 - (2 - tert - butoxycarbonylaminothiazol - 4 - yl)acetamido] - 3 - (1 - carboxymethyltetrazol - 5 - yl)thiomethyl - 3 - cephem - 4 - carboxylic acid (1.08 g, 78%).

NMR δ ppm ($d_6$-DMSO): 1.50 (s, 9H), 3.63 ($q_{AB}$, J=21Hz, 2H), 4.33 ($q_{AB}$, J=12Hz, 2H), 5.03 (d, J=6Hz, 1H), 5.10 (bs, 2H), 5.69 (dd, J=6Hz, J=9Hz, 1H), 7.30 (s, 1H), 9.17 (bs, 2H), 9.53 (d, J=9Hz, 1H).

(2) By following the procedure of Example 6—(2) but using 7 - [2 - dichloromethylene - 2 - (2 - tert - butoxycarbonylaminothiazol - 4 - yl)acetamido] - 3 - (1 - carboxymethyltetrazol - 5 - yl)thiomethyl - 3 - cephem - 4 - carboxylic acid as a raw material, 7 - [2 - dichloromethylene - 2 - (2 - aminothiazol - 4 - yl)acetamido] - 3 - (1 - carboxymethyltetrazol - 5 - yl)thiomethyl - 3 - cephem - 4 - carboxylic acid was prepared.

NMR δ ppm ($d_6$-DMSO): 3.66 ($q_{AB}$, J=21Hz, 2H), 4.30 ($q_{AB}$, J=12Hz, 2H), 5.10 (d, J=6Hz, 1H), 5.23 (bs, 2H), 5.76 (dd, J=6Hz, J=9Hz, 1H), 6.15 (bs, 4H), 6.89 (s, 1H), 9.63 (d, J=9Hz, 1H).

(3) By following the procedure of Example 6—(3) but using 7 - [2 - dichloromethylene - 2 - (2 - aminothiazol - 4 - yl)acetamido] - 3 - (1 - carboxymethyltetrazol - 5 - yl)thiomethyl - 3 - cephem - 4 - carboxylic acid as a raw material, 7 - [2 - dichloromethylene - 2 - (2 - aminothiazol - 4 - yl)acetamido] - 3 - (1 - carboxymethyltetrazol - 5 - yl)thiomethyl - 3 - cephem - 4 - carboxylic acid disodium salt was prepared.

Melting point: 147—155°C. (dec.).

Elemental Analysis (for $C_{17}H_{12}Cl_2N_8O_6S_3Na_2{\cdot}5H_2O$).

Calculated: C: 28.07; H: 3.05; N: 15.40.

Found: C: 27.94; H: 2.53; N: 14.77.

## Example 8

(1) 2-Dichloromethylene-2-(2-tert-butoxycarbonylaminothiazol-4-yl)acetic acid (0.678 g, 2 mmol), prepared as described in Synthesis Example 5—(2), was suspended in methylene chloride (10 ml.), and phosphorus pentachloride (0.417 g, 2 mmol) was added thereto under ice-cooling. The resulting mixture was stirred for 30 minutes and, then, stirred at room temperature for further 30 minutes. The reaction mixture was concentrated under reduced pressure to yield 2-dichloromethylene-2-(2-tert-butoxycarbonylaminothiazol-4-yl)acetic acid chloride in a form of crystals.

On the other hand, 7-aminodesacetoxycephalosporanic acid (0.428 g, 2 mmol) was suspended in methylene chloride (10 ml.), and bis(trimethylsilyl)acetamide (1.3 g) was added. The resulting reaction mixture was stirred at room temperature for 2 hours and then cooled with ice. To this reaction mixture was added dropwise a solution of the above obtained 2-dichloromethylene-2-(2-tert-butoxycarbonylaminothiazol-4-yl)acetic acid chloride in methylene chloride (10 ml.). The resulting reaction mixture was stirred for 1.5 hours under ice-cooling, and ice-water (20 ml.) was added thereto. The pH-value of the reaction mixture was adjusted to 2.0 by the addition of aqueous sodium hydrogencarbonate, and extracted with methylene chloride (20 ml. × 3). The extracts were washed with aqueous sodium chloride, and dried over

anhydrous sodium sulfate. Then, the solvent was removed by distillation to yield 7-[2-dichloromethylene-2-(2-tert-butoxycarbonylaminothiazol-4-yl)acetamido]desacetoxycephalosporanic acid (0.92 g, 86%).

NMR δ ppm ($d_6$-DMSO): 1.50 (s, 9H), 2.03 (s, 3H), 3.43 ($q_{AB}$, J=18Hz, 2H), 5.07 (d, J=6Hz, 1H), 5.66 (dd, J=6Hz, 1H), 7.43 (s, 1H), 9.63 (d, J=9Hz, 1H).

(2) By following the procedure of Example 6—(2) but using 7 - [2 - dichloromethylene - 2 - (2 - tert-butoxycarbonylaminothiazol - 4 - yl)acetamido]desacetoxycephalosporanic acid as a raw material, 7 - [2 - dichloromethylene - 2 - (2 - aminothiazol - 4 - yl)acetamido]desacetoxycephalosporanic acid was prepared.

NMR δ ppm ($d_6$-DMSO): 2.00 (s, 3H), 3.26 ($q_{AB}$, J=18Hz, 2H), 4.03 (bs, 2H), 4.96 (d, J=4.5Hz, 1H), 5.53 (dd, J=4.5Hz, J=9Hz, 1H), 6.83 (s, 1H), 7.00 (bs, 1H), 9.45 (d, J=9Hz, 1H).

(3) 7-[2-Dichloromethylene-2-(2-aminothiazol-4-yl)acetamido]desacetoxycephalosporanic acid (0.35 g) was dissolved in ethyl acetate (5 ml.), and a 2N solution of 2-ethylhexanoic acid sodium salt in n-butyl alcohol was added thereto. Crystals precipitated from the reaction mixture. The crystals were filtered off, washed with small amounts of ethyl acetate and ether, and dried over phosphorus pentoxide under reduced pressure to yield 7-[2-dichloromethylene-2-(2-aminothiazol-4-yl)acetamido]desacetoxycephalosporanic acid sodium salt (0.21 g).

Melting point: 168—173°C. (dec.).

Elemental Analysis (for $C_{14}H_{11}Cl_2N_4S_2O_4Na$).

Calculated: C: 36.37; H: 2.40; N: 12.12.
Found: C: 35.89; H: 2.53; N: 11.54.

## Example 9

(1) 7 - [2 - Dichloromethylene - 2 - (2 - aminothiazol - 4 - yl)acetamido] - 3 - acetoxymethyl - 3 - cephem - 4 - carboxylic acid (0.986 g, 2 mmol) and 2 - methylthio - 1,3,4 - thiadiazole - 5 - thiol (0.36 g, 2.2 mmol) were suspended in a phosphate buffer solution (40 ml.) having a pH-value of 6.4, and sodium hydrogencarbonate (0.336 g, 4.0 mmol) was added thereto. The resulting mixture was stirred at 55°C. for 16 hours. After filtration of the reaction mixture, the filtrate was cooled with ice and adjusted to a pH-value of 2.0 by the addition of a 10% hydrochloric acid. The resulting precipitate was separated and dried over phosphorus pentoxide under reduced pressure to yield 7 - [2 - dichloromethylene - 2 - (aminothiazol - 4 - yl)acetamido] - 3 - (2 - methylthio - 1,3,4 - thiadiazol - 5 - yl)thiomethyl - 3 - cephem - 4 - carboxylic acid (0.44 g).

NMR δ ppm ($d_6$-DMSO): 2.73 (s, 3H), 3.66 (m, 2H), 4.03 (bs, 3H), 4.30 (m, 2H), 5.00 (d, J=4.5Hz, 1H), 5.59 (dd, J=4.5Hz, J=9Hz, 1H), 6.74 (s, 1H), 9.50 (d, J=9Hz, 1H).

(2) By following the procedure of Example 6—(3) but using 7 - [2 - dichloromethylene - 2 - (aminothiazol - 4 - yl)acetamido] - 3 - (2 - methylthio - 1,3,4 - thiadiazol - 5 - yl)thiomethyl - 3 - cephem - 4 - carboxylic acid as a raw material, 7 - [2 - dichloromethylene - 2 - (aminothiazol - 4 - yl)acetamido] - 3 - (2 - methylthio - 1,3,4 - thiadiazol - 5 - yl)thiomethyl - 3 - cephem - 4 - carboxylic acid sodium salt was prepared.

Melting point: 169—174°C. (dec.).

Elemental Analysis (for $C_{17}H_{13}Cl_2N_6O_4S_5Na \cdot 2H_2O$).

Calculated: C: 30.91; H: 2.59; N: 12.72.
Found: C: 30.51; H: 2.33; N: 12.61.

## Example 10

(1) 7 - [2 - Dichloromethylene - 2 - (2 - aminothiazol - 4 - yl)acetamido] - 3 - acetoxymethyl - 3 - cephem - 4 - carboxylic acid (0.986 g, 2 mmol) and 1 - (2 - dimethylaminoethyl) - 1H - tetrazol - 5 - thiol (0.38 g, 2.2 mmol) were suspended in water (8 ml.). Sodium hydrogencarbonate (0.168 g, 2 mmol) was added thereto, and the resulting mixture was stirred at 65°C. for 2 hours. The reaction mixture was cooled, and the resulting precipitate was filtered off. The so obtained precipitate was suspended in water (5 ml.), and the pH-value was adjusted to 6.25 by the addition of a 3N sodium hydroxide solution. Purification through a column packed with Amberlite XAD—2 gave 7 - [2 - dichloromethylene - 2 - (2 - aminothiazol - 4 - yl)acetamido] - 3 - [1 - (2 - dimethylaminoethyl) - 1H - tetrazol - 5 - yl]thiomethyl - 3 - cephem - 4 - carboxylic acid (0.106 g).

Melting point: 170—175°C. (dec.).

NMR δ ppm ($d_6$-DMSO): 2.35 (s, 6H), 3.78 (m, 2H), 3.66 (m, 2H), 4.26 (m, 2H), 4.46 (m, 2H), 5.07 (d, J=6Hz, 1H), 5.69 (dd, J=6Hz, J=9Hz), 6.89 (s, 1H), 9.60 (d, J=9Hz, 1H).

Elemental Analysis (for $C_{19}H_{21}Cl_2N_9O_4S_3$).

Calculated: C: 37.63; H: 3.49; N: 20.78.
Found: C: 36.59; H: 3.66; N: 19.98.

(2) By following the procedure of Example 6—(3) but using 7 - [2 - dichloromethylene - 2 - (2 - aminothiazol - 4 - yl)acetamido] - 3 - [1 - (2 - dimethylaminoethyl) - 1H - tetrazol - 5 - yl]thiomethyl - 3 - cephem - 4 - carboxylic acid as a raw material, 7 - [2 - dichloromethylene - 2 - (2 - aminothiazol - 4 - yl)acetamido] - 3 - [1 - (2 - dimethylaminoethyl) - 1H - tetrazol - 5 - yl]thiomethyl - 3 - cephem - 4 - carboxylic acid sodium salt was prepared.

Melting point: 163—165°C. (dec.).

Elemental Analysis (for $C_{19}H_{20}Cl_2N_9O_4S_3Na \cdot 2H_2O$).

Calculated: C: 34.08; H: 3.61; N: 18.83.
Found: C: 35.64; H: 3.85; N: 18.65.

Synthesis Example 6

(1) After washing a 50% sodium hydride (0.33 g, 6.99 mmol) with n-hexane, anhydrous THF (10 ml.) was added afresh thereto, and the mixture was cooled with ice. When this reaction was over, a solution of 2-(2-tert-butoxycarbonylaminothiazol-4-yl)acetic acid ethyl ester (1.0 g, 3.4 mmol) and ethyl formate (0.30 g, 4.1 mmol) in anhydrous THF (15 ml.) was added dropwise thereto with stirring. After completion of the dropwise addition, the reaction mixture was stirred at room temperature for 8.5 hours. Aqueous ammonium chloride was added thereto, and the mixture was extracted with ethyl acetate. The extract was washed with aqueous sodium chloride and dried over anhydrous sodium sulfate. The solvent was distilled off to yield 2-formyl-2-(2-tert-butoxycarbonylaminothiazol-4-yl)acetic acid ethyl ester (1.0 g, 93%).

NMR δ ppm ($CDCl_3$): 1.23 (t, 3H), 2.07 (s, 9H), 4.20 (q, 2H), 7.40, 7.50 (s, 1H), 7.96, 8.13 (s, 1H).

(2) After cooling dimethylformamide (1.58 g, 21.6 mmol) with ice, phosphorus oxychloride (2.64 g, 17.3 mmol) was added thereto, and the resulting mixture was stirred at room temperature for 2 hours. To this mixture was added dropwise a solution of 2-formyl-2-(2-tert-butoxycarbonylaminothiazol-4-yl)acetic acid ethyl ester (4.52 g, 14.4 mmol) in anhydrous ethyl acetate (20 ml.). The resulting reaction mixture was stirred at room temperature for 24 hours and, then, the pH-value was adjusted to 8.0 by the addition of sodium hydrogencarbonate. The reaction mixture was extracted with ethyl acetate, and the extract was washed with aqueous sodium chloride and dried over anhydrous sodium sulfate. The solvent was distilled off under reduced pressure to give an oily residue. This residue was purified by column chromatography to yield 2-chloromethylene-2-(2-tert-butoxycarbonylaminothiazol-4-yl)acetic acid ethyl ester (2.61 g, 55%).

NMR δ ppm ($CDCl_3$): 1.16 (t, 3H), 1.53 (s, 9H), 4.17 (q, 2H), 7.20 (s, 1H), 7.56 (s, 1H).

(3) To a solution of 2-chloromethylene-2-(2-tert-butoxycarbonylaminothiazol-4-yl)acetic acid ethyl ester (2.61 g, 7.84 mmol) in ethyl alcohol (40 ml.), a 1N sodium hydroxide (13 ml.) was added, and the resulting mixture was stirred at room temperature for 2 days. The reaction mixture was concentrated under reduced pressure. Water was added to the obtained residue, and this mixture was extracted with ethyl acetate. Then the aqueous phase was separated, adjusted to pH 1.0 by the addition of a 10% hydrochloric acid, and extracted with ethyl acetate. The combined extracts were washed with aqueous sodium chloride and dried over anhydrous sodium sulfate. The solvent was distilled off to yield 2-chloromethylene-2-(2-tert-butoxycarbonylaminothiazol-4-yl)acetic acid (1.36 g, 57%).

NMR δ ppm ($d_6$-DMSO): 1.50 (s, 9H), 5.76 (bs, 2H), 7.23 (s, 1H), 7.56 (s, 1H).

Example 11

(1) 2-Chloromethylene-2-(2-tert-butoxycarbonylaminothiazol-4-yl)acetic acid (0.70 g, 2.29 mmol) was suspended in methylene chloride (10 ml.), and phorphorus pentachloride (0.48 g, 2.29 mmol) was added thereto under ice-cooling. The resulting mixture was stirred for 30 minutes and, then, stirred at room temperature for further 30 minutes. The reaction mixture was concentrated under reduced pressure to yield 2-chloromethylene-2-(2-tert-butoxycarbonylamino-1,3-thiazol-4-yl)acetic acid chloride.

On the other hand, 7 - amino - 3 - (1 - methyl - 1H - tetrazol - 5 - yl)thiomethyl - 3 - cephem - 4 - carboxylic acid (0.70 g, 2.29 mmol) was suspended in ethyl acetate (10 ml.), and bis(trimethylsilyl)acetamide (0.88 g, 4.35 mmol) was added. The resulting reaction mixture was stirred at room temperature for 1 hour and then cooled with ice. To this reaction mixture was added dropwise a solution of the above obtained 2 - chloromethylene - 2 - (2 - tert - butoxycarbonylaminothiazol - 4 - yl)acetic acid chloride in methylene chloride (10 ml.). The resulting reaction mixture was stirred for 2 hours under ice-cooling, and ice-water (10 ml.) was added thereto. Thereafter, the reaction mixture was extracted with ethyl acetate (40 ml. × 2). The extracts were washed with aqueous sodium chloride, and dried over anhydrous sodium sulfate. Then, the solvent was removed by distillation to yield 7 - [2 - chloromethylene - 2 - (2 - tert - butoxycarbonylaminothiazol - 4 - yl)acetamido] - 3 - (1 - methyl - 1H - tetrazol - 5 - yl)thiomethyl - 3 - cephem - 4 - carboxylic acid (1.25 g, 89%).

NMR δ ppm ($d_6$-DMSO): 1.50 (s, 9H), 3.70 ($q_{AB}$, J=15Hz, 2H), 3.92 (s, 3H), 4.31 ($q_{AB}$, J=15Hz, 2H), 5.10 (d, J=3Hz, 1H), 5.73 (dd, J=3Hz, J=9Hz, 1H), 7.23 (s, 1H), 7.40 (s, 1H), 9.26 (d, J=9Hz, 1H).

(2) By following the procedure of Example 6—(2) but using 7 - [2 - chloromethylene - 2 - (2 - tert - butoxycarbonylaminothiazol - 4 - yl)acetamido] - 3 - (1 - methyl - 1H - tetrazol - 5 - yl)thiomethyl - 3 - cephem - 4 - carboxylic acid as a raw material, 7 - [2 - chloromethylene - 2 - (2 - aminothiazol - 4 - yl)acetamido] - 3 - (1 - methyl - 1H - tetrazol - 5 - yl)thiomethyl - 3 - cephem - 4 - carboxylic acid was prepared.

NMR δ ppm ($d_6$-DMSO): 3.66 ($q_{AB}$, J=15Hz, 2H), 3.89 (s, 3H), 4.26 ($q_{AB}$, J=15Hz, 2H), 4.73 (bs, 3H), 5.03 (d, J=4.5Hz, 1H), 5.73 (dd, J=4.5Hz, J=9Hz), 6.89 (s, 1H), 7.30 (s, 1H), 9.79 (d, J=9Hz, 1H).

(3) By following the procedure of Example 8—(3) but using 7 - [2 - chloromethylene - 2 - (2 - aminothiazol - 4 - yl)acetamido] - 3 - (1 - methyl - 1H - tetrazol - 5 - yl)thiomethyl - 3 - cephem - 4 - carboxylic acid as a raw material, 7 - [2 - chloromethylene - 2 - (2 - aminothiazol - 4 - yl)acetamido] - 3 - (1 - methyl - 1H - tetrazol - 5 - yl)thiomethyl - 3 - cephem - 4 - carboxylic acid sodium salt was prepared.

Melting point: 171—175°C. (dec.).

NMR δ ppm ($d_6$-DMSO): 3.59 ($q_{AB}$, J=15Hz, 2H), 3.92 (s, 3H), 4.43 ($q_{AB}$, J=15Hz, 2H), 5.00 (d, J=3Hz, 1H), 5.69 (dd, J=3Hz, 9Hz, 1H), 6.89 (s, 1H), 7.30 (s, 1H), 9.73 (d, J=9Hz, 1H).

Elemental Analysis (for $C_{16}H_{14}ClN_8O_4S_3Na \cdot H_2O$)

Calculated: C: 34.63; H: 2.91; N: 20.19.

Found: C: 35.23; H: 3.00; N: 19.24.

Example 12

By following the procedure of Example 11—(1) but substituting 7 - aminocephalosporanic acid for 7 - amino - 3 - (1 - methyl - 1H - tetrazol - 5 - yl)thiomethyl - 3 - cephem - 4 - carboxylic acid, 7 - [2 - chloromethylene - 2 - (2 - tert - butoxycarbonylaminothiazol - 4 - yl)acetamido] - 3 - acetoxymethyl - 3 - cephem - 4 - carboxylic acid was prepared.

NMR δ ppm ($d_6$-DMSO): 1.50 (s, 9H), 2.00 (s, 3H), 3.53 ($q_{AB}$, J=18Hz, 2H), 4.86 ($q_{AB}$, J=13.5Hz, 2H), 5.13 (d, J=6Hz, 1H), 5.76 (dd, J=6Hz, 9Hz, 1H), 7.26 (s, 1H), 7.45 (s, 1H), 9.33 (d, J=9Hz, 1H).

Example 13

By following the procedure of Example 11—(1) but substituting 7 - amino - 3 - (2 - methylthio - 1,3,4 - thiadiazol - 5 - yl)thiomethyl - 3 - cephem - 4 - carboxylic acid for 7 - amino - 3 - (1 - methyl - 1H - tetrazol - 5 - yl)thiomethyl - 3 - cephem - 4 - carboxylic acid, 7 - [2 - chloromethylene - 2 - (2 - tert - butoxycarbonylaminothiazol - 4 - yl)acetamido] - 3 - (2 - methylthio - 1,3,4 - thiadiazol - 5 - yl)thiomethyl - 3 - cephem - 4 - carboxylic acid was prepared.

NMR δ ppm ($d_6$-DMSO): 1.50 (s, 9H), 2.70 (s, 3H), 4.33 ($q_{AB}$, J=12Hz, 2H), 5.10 (d, J=4.5Hz, 1H), 5.73 (dd, J=4.5Hz, 7.5Hz, 1H), 7.20 (s, 1H), 7.36 (s, 1H), 9.23 (d, J=7.5Hz).

Example 14

By following the procedure of Example 11—(1) but substituting 7 - amino - 3 - [(2,5 - dihydro - 6 - hydroxy - 2 - methyl - 5 - oxo - as - triazine - 3 - yl)thiomethyl] - 3 - cephem - 4 - carboxylic acid for 7 - amino - 3 - (1 - methyl - 1H - tetrazol - 5 - yl)thiomethyl - 3 - cephem - 4 - carboxylic acid, 7 - [2 - chloromethylene - 2 - (2 - tert - butoxycarbonylaminothiazol - 4 - yl)acetamido] - 3 - [(2,5 - dihydro - 6 - hydroxy - 2 - methyl - 5 - oxo - as - triazine - 3 - yl)thiomethyl] - 3 - cephem - 4 - carboxylic acid was prepared.

NMR δ ppm ($d_6$-DMSO): 1.43 (s, 9H), 3.56 (s, 3H), 4.26 ($q_{AB}$, J=16.5Hz, 2H), 5.07 (d, J=4.5Hz, 1H), 5.69 (dd, J=4.5Hz, 7.5Hz), 7.16 (s, 1H), 7.33 (s, 1H), 9.20 (d, J=7.5Hz, 1H).

Example 15

By following the procedure of Example 8—(1) but substituting 2 - chloromethylene - 2 - (2 - tert - butoxycarbonylaminothiazol - 4 - yl)acetic acid for 2 - dichloromethylene - 2 - (2 - tert - butoxycarbonylaminothiazol - 4 - yl)acetic acid, 7 - [2 - chloromethylene - 2 - (2 - tert - butoxycarbonylaminothiazol - 4 - yl)acetamido]desacetoxycephalosporanic acid was prepared.

NMR δ ppm ($d_6$-DMSO): 1.50 (s, 9H), 2.00 (s, 3H), 3.41 ($q_{AB}$, J=18Hz, 2H), 5.07 (d, J=3Hz, 1H), 5.63 (dd, J=4.5Hz, J=7.5Hz, 1H), 7.23 (s, 1H), 7.41 (s, 1H), 9.20 (d, J=7.5Hz, 1H).

Example 16

By following the procedure of Example 6—(2) but using 7 - [2 - chloromethylene - 2 - (2 - tert - butoxycarbonylaminothiazol - 4 - yl)acetamido] - 3 - acetoxymethyl - 3 - cephem - 4 - carboxylic acid as a raw material, 7 - [2 - chloromethylene - 2 - (2 - aminothiazol - 4 - yl)acetamido] - 3 - acetoxymethyl - 3 - cephem - 4 - carboxylic acid was prepared.

NMR δ ppm ($d_6$-DMSO): 2.03 (s, 3H), 4.89 ($q_{AB}$, J=13.5Hz, 2H), 5.17 (d, J=6Hz, 1H), 5.83 (dd, J=4.5Hz, 9Hz, 1H), 7.03 (s, 1H), 7.43 (s, 1H), 7.83 (bs, 3H), 9.66 (d, J=9Hz, 1H).

Example 17

By following the procedure of Example 6—(2) but using 7 - [2 - chloromethylene - 2 - (2 - tert - butoxycarbonylaminothiazol - 4 - yl)acetamido] - 3 - (2 - methylthio - 1,3,4 - thiadiazol - 5 - yl)thiomethyl - 3 - cephem - 4 - carboxylic acid as a raw material, 7 - [2 - chloromethylene - 2 - (2 - aminothiazol - 4 - yl)acetamido] - 3 - (2 - methylthio - 1,3,4 - thiadiazol - 5 - yl)thiomethyl - 3 - cephem - 4 - carboxylic acid was prepared.

NMR δ ppm ($d_6$-DMSO): 2.69 (s, 3H), 4.33 ($q_{AB}$, J=13.5Hz, 2H), 5.10 (d, J=4.5Hz, 1H), 5.83 (dd, J=4.5Hz, J=9Hz, 1H), 6.92 (s, 1H), 7.33 (s, 1H), 9.79 (d, J=9Hz, 1H).

Example 18

By following the procedure of Example 6—(2) but using 7 - [2 - chloromethylene - 2 - (2 - tert - butoxycarbonylaminothiazol - 4 - yl)acetamido] - 3 - [(2,5 - dihydro - 6 - hydroxy - 2 - methyl - 5 - oxo - as - triazin - 3 - yl)thiomethyl] - 3 - cephem - 4 - carboxylic acid as a raw material, 7 - [2 - chloromethylene - 2 - (2 - aminothiazol - 4 - yl)acetamido] - 3 - [(2,5 - dihydro - 6 - hydroxy - 2 - methyl - 5 - oxo - as - triazin - 3 - yl)thiomethyl] - 3 - cephem - 4 - carboxylic acid was prepared.

NMR δ ppm ($d_6$-DMSO): 3.83 (s, 3H), 4.33 ($q_{AB}$, J=6Hz, 2H), 5.13 (d, J=4.5Hz, 1H), 5.84 (dd, J=4.5Hz, J=7.5Hz, 1H), 7.10 (s, 1H), 7.56 (s, 1H), 8.89 (bs, 3H), 9.36 (d, J=7.5Hz, 1H).

Example 19

By following the procedure of Example 6—(2) but using 7 - [2 - chloromethylene - 2 - (2 - tert - butoxycarbonylaminothiazol - 4 - yl)acetamido]desacetoxycephalosporanic acid as a raw material, 7 - [2 - chloromethylene - 2 - (2 - aminothiazol - 4 - yl)acetamido]desacetoxycephalosporanic acid was prepared.

NMR δ ppm ($d_6$-DMSO): 2.07 (s, 3H), 3.43 ($q_{AB}$, J=16.5Hz, 2H), 5.07 (d, J=4.5Hz, 1H), 5.66 (dd, J=4.5Hz, 9Hz, 1H), 7.03 (s, 1H), 7.50 (s, 1H), 9.16 (bs, 3H), 9.56 (d, J=9Hz, 1H).

Example 20

By following the procedure of Example 8—(3) but using 7 - [2 - chloromethylene - 2 - (2 - aminothiazol - 4 - yl)acetamido] - 3 - acetoxymethyl - 3 - cephem - 4 - carboxylic acid as a raw material, 7 - [2 - chloromethylene - 2 - (2 - aminothiazol - 4 - yl) - acetamido] - 3 - acetoxymethyl - 3 - cephem - 4 - carboxylic acid sodium salt was prepared.

Elemental Analysis (for $C_{16}H_{14}ClN_4O_6S_2Na$).

Calculated: C: 39.96; H: 2.93; N: 11.65.
Found: C: 40.96; H: 3.35; N: 10.71.

Example 21

By following the procedure of Example 8—(3) but using 7 - [2 - chloromethylene - 2 - (2 - aminothiazol - 4 - yl)acetamido] - 3 - (2 - methylthio - 1,3,4 - thiadiazol - 5 - yl)thiomethyl - 3 - cephem - 4 - carboxylic acid as a raw material, 7 - [2 - chloromethylene - 2 - (2 - aminothiazol - 4 - yl)acetamido] - 3 - (2 - methylthio - 1,3,4 - thiadiazol - 5 - yl)thiomethyl - 3 - cephem - 4 - carboxylic acid sodium salt was prepared.

Elemental Analysis (for $C_{17}H_{14}ClN_6O_4S_5Na{\cdot}1H_2O$).

Calculated: C: 33.86; H: 2.67; N: 23.93.
Found: C: 34.13; H: 2.81; N: 13.26.

Example 22

By following the procedure of Example 8—(3) but using 7 - [2 - chloromethylene - 2 - (2 - aminothiazol - 4 - yl)acetamido] - 3 - [(2,5 - dihydro - 6 - hydroxy - 2 - methyl - 5 - oxo - as - triazin - 3 - yl)thiomethyl] - 3 - cephem - 4 - carboxylic acid (syn-isomer) as a raw material, 7 - [2 - chloromethylene - 2 - (2 - aminothiazol - 4 - yl)acetamido] - 3 - [(2,5 - dihydro - 6 - hydroxy - 2 - methyl - 5 - oxo - as - triazin - 3 - yl)thiomethyl - 3 - cephem - 4 - carboxylic acid disodium salt was prepared.

Elemental Analysis (for $C_{18}H_{14}ClN_7O_6S_3Na_2{\cdot}2H_2O$).

Calculated: C: 33.89; H: 2.84; N: 15.52.
Found: C: 35.10; H: 2.95; N: 14.67.

Example 23

By following the procedure of Example 8—(3) but using 7 - [2 - chloromethylene - 2 - (2 - aminothiazol - 4 - yl)acetamido]desacetoxycephalosporanic acid (syn-isomer) as a raw material, 7 - [2 - chloromethylene - 2 - (2 - aminothiazol - 4 - yl)acetamido]desacetoxycephalosporanic acid sodium salt was prepared.

Elemental Analysis (for $C_{14}H_{12}ClN_4O_4S_2Na{\cdot}2H_2O$).

Calculated: C: 36.64; H: 3.51; N: 12.21.
Found: C: 37.41; H: 3.12; N: 11.42.

Example 24

7 - [2 - chloromethylene - 2 - (2 - aminothiazol - 4 - yl)acetamido] - 3 - acetoxymethyl - 3 - cephem - 4 - carboxylic acid (0.20 g, 0.43 mmol) was dissolved in a mixture of pyridine (2 ml.) and water (3 ml.), and the resulting solution was heated at 40°C. for 44 hours with stirring. The so obtained reaction mixture was concentrated under reduced pressure, a 50% aqueous ethyl alcohol (5 ml.) was added thereto, and the obtained reaction mixture was concentrated under reduced pressure. When ethyl alcohol was added to the residue, a yellow precipitate was formed. The precipitate was filtered off and dried over phosphorus pentoxide under reduced pressure to yield 7 - [2 - chloromethylene - 2 - (2 - aminothiazol - 4 - yl)acetamido] - 3 - pyridiniummethyl - 3 - cephem - 4 - carboxylic acid betaine.

Elemental Analysis (for $C_{19}H_{16}ClN_5O_4S_2$).

Calculated: C: 47.75; H: 3.37; N: 14.65.
Found: C: 46.13; H: 3.53; N: 13.25.

Synthesis Example 7

Dimethylformamide (10 ml.) was cooled with ice, phosphorus oxychloride (0.58 g, 3.82 mmol) was added, and the mixture was stirred at room temperature for 2 hours. To this reaction mixture was added 2 - formyl - 2 - (2 - tert - butoxycarbonylaminothiazol - 4 - yl)acetic acid ethyl ester (1 g, 3.19 mmol), and the mixture was heated at 80°C. for 1 hour with stirring. Water was added to the reaction mixture, and the pH-value was adjusted to 8 by the addition of aqueous sodium hydrogencarbonate. The reaction mixture was then extracted with a mixture of ethyl acetate and ether (1:1). The extract was washed with aqueous sodium chloride and dried over anhydrous sodium sulfate. The solvents were distilled off to yield an oily residue, which was purified by column chromatography to afford 2 - chloromethylene - 2 - (2 - aminothiazol - 4 - yl)acetic acid ethyl ester containing a small amount of dimethylformamide.

NMR δ ppm ($CDCl_3$): 1.30 (t, 3H), 4.26 (q, 2H), 7.00 (s, 1H), 7.50 (s, 1H), and 2.10 (s, 6H), 8.30 (s, 1H) DMF proton.

Synthesis Example 8

(1) A 50% sodium hydride in oil (1.51 g, 31.5 mmol) was washed with n-hexane and suspended in anhydrous THF (20 ml.), and the suspension was cooled with ice. To this suspension, a solution of 2-(2-aminothiazol-4-yl)acetic acid ethyl ester (2.93 g, 16 mmol) and ethyl formate (2.33 g, 31 mmol) in THF (30 ml.) was added dropwise with stirring. The reaction mixture was stirred at room temperature for 48 hours, and aqueous ammonium chloride was added thereto. After extraction with ethyl acetate, the extract was washed with aqueous sodium chloride and dried over anhydrous sodium sulfate. The solvent was distilled off to yield 2-formyl-2-(2-aminothiazol-4-yl)acetic acid ethyl ester (2.86 g, 85%).

NMR δ ppm ($CDCl_3$-$d_6$-DMSO): 1.33 (t, 3H), 4.16 (q, 2H), 7.50 (s, 1H), 8.00 (s, 1H), 8.53 (s, 1H).

(2) Phosphorus oxychloride (0.86 g, 5.6 mmol) was added at room temperature to dimethylformamide (8 ml.), and the mixture was stirred for 20 minutes. Then, 2-formyl-2-(2-aminothiazol-4-yl)acetic acid ethyl ester (1.0 g, 4.67 mmol) was added, and stirring was continued at room temperature for 1 hour. The pH-value was adjusted to 8 by the addition of aqueous sodium hydrogencarbonate, and the reaction mixture was extracted with ethyl acetate. The extract was washed with aqueous sodium chloride, and dried over anhydrous sodium sulfate. The solvent was distilled off to give 2-chloromethylene-2-(2-aminothiazol-4-yl)acetic acid ethyl ester (1.05 g) containing dimethylformamide and 2-formyl-2-(2-aminothiazol-4-yl)acetic acid ethyl ester. This compound showed the same NMR spectrum as those of the compound prepared in Synthesis Example 7.

(3) 2-Chloromethylene-2-(2-aminothiazol-4-yl)acetic acid ethyl ester (1.05 g, 4.67 mmol) was dissolved in 17% aqueous ethyl alcohol (60 ml.), 1N sodium hydroxide (10 ml.) was added thereto, and the resulting mixture was stirred at room temperature for 16 hours. After concentration the reaction mixture under reduced pressure, water was added to the residue, and the resulting mixture was extracted with ethyl acetate. The aqueous phase was separated. Adjustment of the pH value to 2 by the addition of a 10% hydrochloric acid caused a precipitate to be formed. The precipitate was filtered off, and dried over phosphorus pentoxide under reduced pressure to yield 2-chloromethylene-2-(2-aminothiazol-4-yl)acetic acid (0.45 g, yield 47%).

NMR δ ppm ($d_6$-DMSO): 4.00 (bs, 3H), 7.33 (s, 1H), 7.59 (s, 1H).

Synthesis Example 9

(1) A viscous liquid mixture of 2-chloromethylene-2-(2-aminothiazol-4-yl)acetic acid ethyl ester and DMF in a ratio of 1:1 (4.3 g, 14 mmol) was stirred under ice-cooling. On the other hand, a solution was prepared by stirring acetic anhydride and formic acid in an equimolar ratio at 50°C. for 1 hour with stirring. This solution was cooled to room temperature, and 5 ml. of this solution was gradually added dropwise to the aforesaid viscous liquid mixture. After the dropwise addition was completed, the resulting mixture was stirred at 60°C. for 1 hour, and water (30 ml.) was added under ice-cooling. The reaction mixture thus obtained was concentrated under reduced pressure with an aspirator to give a system of crystal and water. The liquid was separated using ethyl acetate (100 ml.). The organic phase was washed with water and, then, with aqueous sodium hydrogencarbonate, and again with water. The organic phase was then dried over anhydrous magnesium sulfate, and concentrated under reduced pressure to yield 2-chloromethylene-2-(2-formylaminothiazol-4-yl)acetic acid ethyl ester (3.7 g, 100%) as crystals.

Melting point: 103—104°C.

NMR δ ppm ($d_6$-DMSO): 1.25 (t, J=7Hz, 3H), 4.23 (q, J=7Hz, 2H), 7.45 (s, 1H), 7.68 (s, 1H), 8.53 (s, 1H), 12.51 (bs, 1H).

(2) 2-Chloromethylene-2-(2-formylaminothiazol-4-yl)acetic acid ethyl ester (7.73 g, 29.7 mmol) was dissolved in ethyl alcohol (100 ml.). A solution of potassium hydroxide (3.33 g, 85.5% pure, 59.4 mmol) in water (100 ml.) was gradually added thereto under ice-cooling. The resulting mixture was stirred at room temperature for 5 hours, and concentrated under reduced pressure with an aspirator. Ethyl alcohol was distilled off, followed by liquid separation by use of ethyl acetate (100 ml.). The aqueous phase was slightly acidified by the addition of 2N hydrochloric acid while cooling with ice-water and stirring. The formed crystals were filtered off to yield 2-chloromethylene-2-(2-formylaminothiazol-4-yl)acetic acid (4.84 g, 70%) in a form of crystals.

Melting point: not less than 300°C.

NMR δ ppm ($d_6$-DMSO): 7.50 (s, 1H), 7.70 (s, 1H), 8.65 (s, 1H).

Example 25

(1) A suspension of 2-chloromethylene-2-(2-formylaminothiazol-4-yl)acetic acid (14.69 g, 0.063 mol) in anhydrous THF (200 ml.) was cooled to 0° to 5°C., and phosphorus pentachloride (13.16 g, 0.063 mol) was added little by little with violent stirring. After the addition was over, stirring was continued at 0° to 5°C. for 1 hour and, then, at room temperature for 1 hour. Concentration of the reaction solution at room temperature and under reduced pressure gave 2-chloromethylene-2-(2-formylaminothiazol-4-yl)acetic acid chloride as light yellow crystals.

(2) Bis(trimethylsilyl)acetamide (31.87 g, 0.157 mol) was added to a suspension of 7 - amino - 3 - (1 - methyl - 1H - tetrazol - 5 - yl)thiomethyl - 3 - cephem - 4 - carboxylic acid (20.7 g, 0.063 mol) in anhydrous ethyl acetate (200 ml.). The mixture was stirred at room temperature for 2 hours to form a solution, which was then cooled to 0° to 5°C. To this solution, a suspension of the above-obtained acid chloride in

anhydrous ethyl acetate (180 ml.) was added. The reaction mixture thus obtained was stirred at 0° to 5°C. for 2 hours, water (260 ml.) was added, and the resulting mixture was ice-cooled. The pH-value was adjusted to 7.5 by the addition of aqueous sodium carbonate, followed by extraction. The aqueous phase was separated, ice-cooled, and adjusted to pH 2.0 by the addition of a 10% hydrochloric acid. (Crystals were formed and an oily material was formed partially.) The aqueous phase was then extracted twice (400 ml. and 300 ml.) with a mixture of ethyl acetate and THF. The organic phase obtained was washed with water (100 ml. × 2), and dried over anhydrous sodium sulfate. Then, the solvent was removed by distillation to yield 7 - [2 - chloromethylene - 2 - (2 - formylaminothiazol - 4 - yl)acetamido] - 3 - (1 - methyl - 1H - tetrazol - 5 - yl)thiomethyl - 3 - cephem - 4 - carboxylic acid (38.6 g) as yellow powder.

NMR δ ppm ($d_6$-DMSO): 3.73 (m, 2H), 4.00 (s, 3H)—4.3 (m, 2H), 5.16 (d, J=6Hz, 1H), 5.8 (dd, J=6Hz, 9Hz, 1H), 7.3 (s, 1H), 7.6 (s, 1H), 8.6 (s, 1H), 9.3 (d, J=9Hz, 1H).

(3) A solution of the formylated product (38.6 g) prepared as described above in methyl alcohol-THF (700 ml., 1:1) was cooled to 0° to 5°C., concentrated hydrochloric acid (12.6 g) was added thereto, and the resulting mixture was stirred at room temperature for 16 hours. After the reaction was over, the solvents were removed by distillation under reduced pressure. To the obtained residue was added water (400 ml.) and ethyl acetate-THF (400 ml., 1:1), and the pH-value was adjusted to 7.0 by the addition of aqueous sodium hydrogencarbonate. The aqueous phase was separated, washed with ethyl acetate (200 ml.), and then ice-cooled. The pH-value was adjusted to 3.0 by the addition of a 10% hydrochloric acid with stirring. The crystals formed were collected by filtration, washed with water, and dried over phosphorus pentoxide under reduced pressure to yield 7 - [2 - chloromethylene - 2 - (2 - aminothiazol - 4 - yl)acetamido] - 3 - (1 - methyl - 1H - tetrazol - 5 - yl)thiomethyl - 3 - cephem - 4 - carboxylic acid (23.97 g, 74%).

NMR δ ppm ($d_6$-DMSO): 3.66 ($q_{AB}$, J=15Hz, 2H), 3.89 (s, 3H), 4.26 ($q_{AB}$, J=15Hz, 2H), 4.73 (bs, 3H), 5.03 (d, J=4.5Hz, 1H), 5.73 (dd, J=4.5Hz, J=9Hz), 6.89 (s, 1H), 7.30 (s, 1H), 9.79 (d, J=9Hz, 1H).

## Example 26

7 - [2 - chloromethylene - 2 - (2 - aminothiazol - 4 - yl)acetamido] - 3 - (1 - methyl - 1H - tetrazol - 5 - yl)thiomethyl - 3 - cephem - 4 - carboxylic acid (0.267 g, 0.518 mmol) was dissolved in methyl alcohol-THF (10 ml., 1:1). An activated carbon (20 mg.) was added thereto, and the resulting mixture was stirred for 30 minutes and filtered. After removing the solvents by distillation under reduced pressure, anhydrous THF (3 ml.) was added thereto, followed by the addition of a solution (1 ml.) of 1N 2-ethylhexanoic acid sodium salt in methyl alcohol. The crystals formed were collected by filtration, washed with THF (1 ml. × 2), and dried over phosphorus pentoxide under reduced pressure to afford 7 - [2 - chloromethylene - 2 - (2 - aminothiazol - 4 - yl)acetamido] - 3 - (1 - methyl - 1H - tetrazol - 5 - yl)thiomethyl - 3 - cephem - 4 - carboxylic acid sodium salt (0.217 g, 78%).

Melting point: 171—175°C. (dec.).

NMR δ ppm (DMSO-$d_6$): 3.59 ($q_{AB}$, J=15Hz, 2H), 3.92 (s, 3H), 4.43 ($q_{AB}$, J=15Hz, 2H), 5.00 (d, J=3Hz, 1H), 5.69 (dd, J=3Hz, 9Hz, 1H), 6.89 (s, 1H), 7.30 (s, 1H), 9.73 (d, J=9Hz, 1H).

## Synthesis Example 10

By following the procedure of Synthesis Example 1 but substituting 4-methoxyphenylglyoxylic acid tert-butyl ester for 2-furylglyoxylic acid tert-butyl ester, 2-dichloromethylene-2-(4-methoxyphenyl)acetic acid was prepared.

Melting point: 117—122°C.

IR (KBr): 1690 $cm^{-1}$.

NMR δ ppm ($CDCl_3$): 3.86 (s, 3H), 7.00 (m, 2H), 7.40 (m, 2H), 9.70 (bs, 1H).

## Example 27

2-Dichloromethylene-2-(4-methoxyphenyl)acetic acid (0.94 g, 3.92 mmol) was dissolved in chloroform (5 ml.), thionyl chloride (0.69 g, 5.85 mmol) was added thereto, and the resulting mixture was heated under reflux for 3 hours. After completion of the reaction, the reaction mixture was concentrated to give an oily residue (0.65 g).

On the other hand, 7-aminocephalosporanic acid (1.28 g, 3.78 mmol) was suspended in anhydrous chloroform (10 ml.), hexamethyldisilazane (3,78 g, 23.5 mmol) was added thereto, and the resulting mixture was heated under reflux for 1.5 hours. After completion of the reaction, chloroform and excess hexamethyldisilazane were removed by distillation. Then, anhydrous chloroform (10 ml.) was added to the obtained residue, and the resulting mixture was stirred while cooling to 0° to 5°C. To this mixture was added dropwise a solution of the above-obtained oily residue in chloroform (10 ml.). After stirring for 3 hours, the resulting reaction mixture was poured onto ice-water (60 ml.), and the pH-value was adjusted to 8 by the addition of sodium hydrogencarbonate, followed by extraction with ethyl acetate. The aqueous phase was separated, adjusted to pH 2 by the addition of a 10% hydrochloric acid, and extracted thrice with ethyl acetate (30 ml. each).

The extracts were washed with aqueous sodium chloride, and dried over anhydrous sodium sulfate. Then, the solvent was removed by distillation to yield 7 - [2 - dichloromethylene - 2 - (4 - methoxyphenyl)acetamido] - 3 - acetoxymethyl - 3 - cephem - 4 - carboxylic acid (1.47 g, 75%).

Melting point: 140—142°C. (dec.).

NMR δ ppm ($d_6$-acetone): 2.00 (s, 3H), 3.56 ($q_{AB}$, J=18Hz, 2H), 3.79 (s, 3H), 4.92 ($q_{AB}$, J=13.5Hz, 2H), 5.16 (d, J=4.5Hz, 1H), 5.86 (dd, J=4.5Hz, 9Hz, 1H), 6.92 (d, J=9Hz, 2H), 7.43 (d, J=9Hz, 2H), 8.56 (d, J=9Hz, 1H), 9.56 (bs, 1H).

Elemental Analysis (for $C_{20}H_{18}Cl_2N_2O_7S$).

Calculated: C: 47.92; H: 3.62; N: 5.59.

Found: C: 47.75; H: 3.81; N: 5.42.

Synthesis Example 11

By following the procedure of Synthesis Example 1 but substituting 4-methylphenylglyoxylic acid tert-butyl ester for 2-furylglyoxylic acid tert-butyl ester, 2-dichloromethylene-2-(4-methylphenyl)acetic acid was prepared.

Melting point: 98—106°C.

IR (KBr): 1700 $cm^{-1}$.

NMR δ ppm ($CDCl_3$): 2.33 (s, 3H), 7.13 (s, 4H), 10.16 (bs, 1H).

Example 28

By following the procedure of Example 27 but substituting 2-dichloromethylene-2-(4-methylphenyl)acetic acid for 2-dichloromethylene-2-(4-methoxyphenyl)acetic acid, 7-[2-dichloromethylene-2-(4-methylphenyl)acetamido]-3-acetoxymethyl-3-cephem-4-carboxylic acid was prepared.

Melting point: 149—151°C.

NMR δ ppm ($d_6$-acetone): 2.03 (s, 3H), 2.33 (s, 3H), 3.56 ($q_{AB}$, J=18Hz, 2H), 4.86 ($q_{AB}$, J=13.5Hz, 2H), 5.07 (d, J=6Hz, 1H), 5.76 (dd, J=4.5Hz, J=9Hz, 1H), 7.13 (d, J=9Hz, 2H), 7.33 (d, J=9Hz, 2H), 8.53 (d, J=9Hz, 1H), 9.26 (bs, 1H).

Elemental Analysis (for $C_{20}H_{18}Cl_2N_2O_6S$).

Calculated: C: 49.49; H: 3.74; N: 5.77.

Found: C: 49.25; H: 3.84; N: 5.67.

Synthesis Example 12

By following the procedure of Synthesis Example 1 but substituting 4-chlorophenylglyoxylic acid tert-butyl ester for 2-furylglyoxylic acid tert-butyl ester, 2-dichloromethylene-2-(4-chlorophenyl)acetic acid was prepared.

Melting point: 44—98°C.

IR (KBr): 1700 $cm^{-1}$.

NMR δ ppm ($CDCl_3$): 7.26 (s, 4H), 10.70 (s, 1H).

Example 29

By following the procedure of Example 27 but substituting 2 - dichloromethylene - 2 - (4 - chlorophenyl)acetic acid for 2 - dichloromethylene - 2 - (4 - methoxyphenyl)acetic acid, 7 - [2 - dichloromethylene - 2 - (4 - chlorophenyl)acetamido] - 3 - acetoxymethyl - 3 - cephem - 4 - carboxylic acid was prepared.

Melting point: 155—156°C. (dec.).

NMR δ ppm ($d_6$-acetone): 2.00 (s, 3H), 3.56 ($q_{AB}$, J=18Hz, 2H), 4.92 ($q_{AB}$, J=13.5Hz, 2H), 5.16 (d, J=6Hz, 1H), 5.84 (dd, J=6Hz, 9Hz, 1H), 7.30—7.63 (m, 4H), 8.66 (d, J=9Hz, 1H), 9.10 (bs, 1H).

Elemental Analysis (for $C_{19}H_{15}Cl_3N_2O_6S$).

Calculated: C: 45.12; H: 2.99; N: 5.54.

Found: C: 45.14; H: 3.22; N: 5.42.

Synthesis Example 13

By following the procedure of Synthesis Example 1 but substituting 3,4-dichlorophenylglyoxylic acid tert-butyl ester for 2-furylglyoxylic acid tert-butyl ester, 2-dichloromethylene-2-(3,4-dichlorophenyl)acetic acid was prepared.

Melting point: 78—80°C.

IR (KBr): 1690 $cm^{-1}$.

NMR δ ppm ($CDCl_3$): 7.00—7.70 (m, 3H), 10.50 (bs, 1H).

Example 30

By following the procedure of Example 27 but substituting 2-dichloromethylene-2-(3,4-dichlorophenyl)acetic acid for 2-dichloromethylene-2-(4-methoxyphenyl)acetic acid, 7-[2-dichloromethylene-2-(3,4-dichlorophenyl)acetamido]-3-acetoxymethyl-3-cephem-4-carboxylic acid was prepared.

Melting point: 143—146°C. (dec.).

NMR δ ppm ($d_6$-acetone): 2.03 (s, 3H), 3.59 ($q_{AB}$, J=18Hz, 2H), 4.96 ($q_{AB}$, J=13.5Hz, 2H), 5.20 (d, J=4.5Hz, 1H), 5.86 (dd, J=4.5Hz, 9Hz, 1H), 7.40—7.73 (m, 3H), 8.73 (d, J=9Hz, 1H).

Elemental Analysis (for $C_{19}H_{14}Cl_4N_2O_6S$).

Calculated: C: 42.25; H: 2.61; N: 5.19.

Found: C: 42.30; H: 2.80; N: 5.12.

Synthesis Example 14

By following the procedure of Synthesis Example 1 but substituting 5-chloro-2-thienylglyoxylic acid tert-butyl ester for 2-furylglyoxylic acid tert-butyl ester, 2-dichloromethylene-2-(5-chloro-2-thienyl)acetic acid was prepared.

Melting point: 112—115°C.

IR (KBr): 1710 $cm^{-1}$.

NMR δ ppm ($CDCl_3$): 6.86—7.13 (m, 2H), 9.16 (bs, 1H).

Example 31

By following the procedure of Example 27 but substituting 2 - dichloromethylene - 2 - (5 - chloro - 2 - thienyl)acetic acid for 2 - dichloromethylene - 2 - (4 - methoxyphenyl)acetic acid, 7 - [2 - dichloromethylene - 2 - (5 - chloro - 2 - thienyl)acetamido] - 3 - acetoxymethyl - 3 - cephem - 4 - carboxylic acid was prepared.

Melting point: 141—144°C. (dec.).

NMR δ ppm ($d_6$-acetone): 2.00 (s, 3H), 3.61 ($q_{AB}$, J=18Hz, 2H), 4.97 ($q_{AB}$, J=13.5Hz, 2H), 5.20 (d, J=9Hz, 1H), 5.91 (dd, J=4.5Hz, 9Hz, 1H), 6.13 (bs, 1H), 7.07 (m, 2H), 8.76 (d, J=9Hz, 1H).

Elemental Analysis (for $C_{17}H_{13}Cl_3N_2O_6S_2$).

Calculated: C: 39.74; H: 2.94; N: 5.45.

Found: C: 39.85; H: 2.59; N: 5.76.

Synthesis Example 15

By following the procedure of Synthesis Example 1 but substituting 5-bromo-2-thienylglyoxylic acid tert-butyl ester for 2-furylglyoxylic acid tert-butyl ester, 2-dichloromethylene-2-(5-bromo-2-thienyl)acetic acid was prepared.

Melting point: 100—105°C.

IR (KBr): 1700 $cm^{-1}$.

NMR δ ppm ($CDCl_3$): 6.60 (m, 1H), 6.96 (m, 1H), 7.06 (m, 1H), 7.60 (m, 1H), 10.06 (bs, 1H).

Example 32

By following the procedure of Example 27 but substituting 2 - dichloromethylene - 2 - (5 - bromo - 2 - thienyl)acetic acid for 2 - dichloromethylene - 2 - (4 - methoxyphenyl)acetic acid, 7 - [2 - dichloromethylene - 2 - (5 - bromo - 2 - thienyl)acetamido] - 3 - acetoxymethyl - 3 - cephem - 4 - carboxylic acid was prepared.

Melting point: 165—168°C. (dec.).

NMR δ ppm ($d_6$-acetone): 2.00 (s, 3H), 3.59 ($q_{AB}$, J=18Hz, 1H), 4.92 ($q_{AB}$, J=13.5Hz, 2H), 5.23 (d, J=4.5Hz, 1H), 5.92 (dd, J=4.5Hz, 9Hz, 1H), 7.07 (d, J=3Hz, 1H), 7.16 (d, J=3Hz, 1H), 7.75 (bs, 1H), 8.79 (d, J=9Hz, 1H).

Elemental Analysis (for $C_{17}H_{13}BrCl_2N_2O_6S_2$).

Calculated: C: 36.70; H: 2.35; N: 5.03.

Found: C: 36.51; H: 2.37; N: 5.00.

Example 33

By following the procedure of Example 27 but substituting 2 - dichloromethylene - 2 - (2 - furyl)acetic acid for 2 - dichloromethylene - 2 - (4 - methoxyphenyl)acetic acid, 7 - [2 - dichloromethylene - 2 - (2 - furyl)acetamido] - 3 - acetoxymethyl - 3 - cephem - 4 - carboxylic acid was prepared.

Melting point: 138—142°C. (dec.).

NMR δ ppm ($d_6$-acetone): 2.00 (s, 3H), 3.56 ($q_{AB}$, J=18Hz, 2H), 4.90 ($q_{AB}$, J=13.5Hz, 2H), 5.20 (d, J=6Hz, 1H), 5.91 (dd, J=4.5Hz, 9Hz, 1H), 6.54 (dd, J=1.5Hz, 3Hz, 1H), 6.84 (d, J=3Hz, 1H), 7.63 (d, J=1.5Hz, 1H), 8.66 (d, J=9Hz, 1H), 8.83 (bs, 1H).

Elemental Analysis (for $C_{17}H_{14}Cl_2N_2O_7S$).

Calculated: C: 44.26; H: 3.06; N: 6.07.

Found: C: 43.67; H: 3.08; N: 5.99.

Synthesis Example 16

(1) Potassium (0.47 g, 12 mg-atom) was added to anhydrous tert-butyl alcohol (5 ml.) under argon atmosphere, and the mixture was heated under reflux for 1 hour to prepare a potassium tert-butoxide solution. The resulting mixture was cooled with water bath, and chloromethyltriphenylphosphonium iodide (4.38 g, 10 mmol) was added thereto little by little. After stirring for 1.5 hours a solution of 2-thienylglyoxylic acid tert-butyl ester (1.0 g, 4.7 mmol) in anhydrous tert-butyl alcohol (5 ml.) was added, and the resulting mixture was stirred at room temperature for 2 hours. After completion of the reaction, the reaction mixture was concentrated under reduced pressure, and the obtained residue was extracted with n-hexane.

The n-hexane was removed by distillation to give a red oily residue. Purification of this residue by column chromatography yielded 2-chloromethylene-2-(2-thienyl)acetic acid tert-butyl ester (1.0 g, 87%).

NMR δ ppm ($CDCl_3$): 1.59 (s, 9H), 6.46—7.73 (m, 4H).

(2) 2-Chloromethylene-2-(2-thienyl)acetic acid tert-butyl ester (1.0 g) was dissolved in acetic acid (15 ml.), 6N hydrochloric acid (5 ml.) was added, and the resulting mixture was heated under reflux for 1

hour. After the reaction was over, the reaction mixture was concentrated under reduced pressure. The residue was purified by column chromatography to yield 2-chloromethylene-2-(2-thienyl)acetic acid (0.67 g, 76%).

NMR δ ppm ($CDCl_3$): 7.10 (m, 1H), 7.23 (m, 1H), 7.23, 7.46 (s, 1H), 10.10 (bs, 1H).

Example 34

Anhydrous dimethylformamide (0.28 g, 3.85 mmol) and phosphorus oxychloride (0.59 g, 3.85 mmol) were added to anhydrous ethyl acetate (2 ml.), and the Vilsmeier's reagent was prepared according to a conventional method. The obtained reaction mixture was cooled to 0° to 5°C., and a solution of 2-chloromethylene-2-(2-thienyl)acetic acid (0.93 g, 4.17 mmol) in anhydrous ethyl acetate (10 ml.) was added dropwise to the reaction mixture with stirring. The mixture was stirred at that temperature for further one hour.

On the other hand, 7 - amino - 3 - (1 - methyl - 1H - tetrazol - 5 - yl)thiomethyl - 3 - cephem - 4 - carboxylic acid (1.55 g, 4.74 mmol) was suspended in anhydrous ethyl acetate (20 ml.), and bis(trimethylsilyl)acetamide (1.13 g, 5.61 mmol) was added thereto. This mixture was stirred for 1 hour at room temperature, and then cooled to −10°C. To this mixture was added dropwise the previously prepared reaction mixture described above, and stirring was continued for 1.5 hours at that temperature. Water (30 ml.) was added to the reaction mixture. The pH-value of this mixture was adjusted to 7.5 to 8.0 by the addition of aqueous sodium hydrogencarbonate, and the ethyl acetate phase was separated. The aqueous phase was adjusted to pH 2 with a 10% hydrochloric acid and extracted thrice with ethyl acetate (30 ml.). The combined ethyl acetate phase and extracts were washed with aqueous sodium chloride and dried over anhydrous sodium sulfate. The solvent was distilled off to give 7 - [2 - chloromethylene - 2 - (2 - thienyl)acetamido] - 3 - (1 - methyl - 1H - tetrazol - 5 - yl)thiomethyl - 3 - cephem - 4 - carboxylic acid (0.89 g, 47%).

Melting point: 125—132°C. (dec.).

NMR δ ppm ($d_6$-acetone): 3.76 ($q_{AB}$, J=16.5Hz, 2H), 3.96 (s, 3H), 4.36 ($q_{AB}$, J=16.5Hz, 2H), 5.16 (m, 1H), 5.83, 5.92 (m, 1H), 6.76, 7.03 (s, 1H), 7.07 (m, 1H), 7.40 (m, 1H), 7.56 (m, 1H).

Elemental Analysis (for $C_{17}H_{15}ClN_6O_4S_3$).

Calculated: C: 40.92; H: 3.03; N: 16.84.

Found: C: 39.13; H: 3.18; N: 15.73.

Synthesis Example 17

By following the procedure of Synthesis Example 1 but substituting 3-thienylacetic acid tert-butyl ester for 2-furylglyoxylic acid tert-butyl ester, 2-dichloromethylene-2-(3-thienyl)acetic acid was prepared.

NMR δ ppm ($CDCl_3$): 7.26 (m, 2H), 7.56 (m, 1H), 9.66 (m, 1H).

Example 35

By following the procedure of Example 34 but substituting 2 - dichloromethylene - 2 - (3 - thienyl)acetic acid for 2 - monochloromethylene - 2 - (2 - thienyl)acetic acid, 7 - [2 - dichloromethylene - 2 - (3 - thienyl)acetamido] - 3 - (1 - methyl - 1H - tetrazol - 5 - yl)thiomethyl - 3 - cephem - 4 - carboxylic acid was prepared.

Melting point: 134—137°C.

NMR δ ppm ($d_6$-acetone): 3.80 ($q_{AB}$, J=18Hz, 2H), 3.96 (s, 3H), 4.40 ($q_{AB}$, J=18Hz, 2H), 5.21 (d, J=4.5Hz, 1H), 5.89 (dd, J=4.5Hz, 9Hz, 1H), 7.33 (m, 1H), 7.52 (m, 1H), 7.73 (m, 1H), 8.66 (d, J=9Hz, 1H).

Elemental Analysis (for $C_{17}H_{14}Cl_2N_6O_4S_3$).

Calculated: C: 38.28; H: 2.65; N: 15.75.

Found: C: 37.55; H: 2.67; N: 14.71.

Synthesis Example 18

By following the procedure of Synthesis Example 1—(1) but substituting (1-methyl-2-pyrrolyl)glyoxylic acid ethyl ester for 2-furylglyoxylic acid tert-butyl ester, 2-dichloromethylene-2-(1-methyl-2-pyrrolyl)acetic acid ethyl ester was prepared as an oil.

NMR δ ppm ($CDCl_3$): 1.30 (t, 3H), 4.20 (q, 2H), 6.03—6.23 (m, 2H), 6.66 (m, 1H).

Example 36

To a solution of 2 - dichloromethylene - 2 - (1 - methyl - 2 - pyrrolyl)acetic acid ethyl ester (1.0 g, 4.0 mmol) in ethyl alcohol (10 ml.), a solution of potassium hydroxide (0.27 g, 4.8 mmol) in ethyl alcohol (2 ml.) was added, and the resulting mixture was stirred at room temperature for 16 hours. The reaction mixture was concentrated under reduced pressure. The obtained residue was washed with ether, and dried over phosphorus pentoxide under reduced pressure for 3 hours to give white crystals (0.6 g). The crystals obtained were suspended in anhydrous benzene (10 ml.), dimethylformamide (10 mg.) was added thereto, and the mixture was ice-cooled. Then, a solution of oxalyl chloride (0.38 g, 3 mmol) in benzene (3 ml.) was added dropwise. The resulting mixture was stirred at that temperature for 1 hour, and concentrated under reduced pressure to give 2 - dichloromethylene - 2 - (1 - methyl - 2 - pyrrolyl)acetic acid chloride in a form of light brown oil. By using the thus obtained chloride and carrying out a reaction according to the method

described in Example 27, 7 - [2 - dichloromethylene - 2 - (1 - methyl - 2 - pyrrolyl)acetamido] - 3 - acetoxymethyl - 3 - cephem - 4 - carboxylic acid was obtained.

Melting point: 130—135°C. (dec.).

NMR δ ppm ($d_6$-acetone): 2.00 (s, 3H), 3.56 ($q_{AB}$, J=18Hz, 2H), 3.89 (s, 3H), 4.92 ($q_{AB}$, J=13.5Hz, 2H), 5.16 (d, J=6Hz, 1H), 5.83 (dd, J=4.5Hz, 9Hz, 1H), 6.39 (m, 2H), 6.66 (m, 1H), 7.26 (bs, 1H), 9.63 (d, J=9Hz, 1H).

Elemental Analysis (for $C_{18}H_{17}Cl_2N_3O_6S$).

Calculated: C: 45.58; H: 3.61; N: 8.86.
Found: C: 46.10; H: 4.20; N: 7.84.

## Example 37

6-Aminopenicillanic acid (0.48 g, 2.24 mmol) was suspended in methylene chloride (10 ml.), triethylamine (0.45 g, 4.48 mmol) was added thereto, and the resulting mixture was stirred at room temperature for 30 minutes. After the reaction mixture was ice-cooled, a solution of 2-dichloromethylene-2-(2-thienyl)acetic acid chloride (0.54 g, 2.24 mmol) in methylene chloride (8 ml.) was added thereto. The obtained reaction mixture was stirred for 2 hours under ice-cooling and, then, stirred at room temperature for 30 minutes. The reaction mixture was concentrated under reduced pressure. The residue thus obtained was dissolved in water (30 ml.) and washed with ether. The aqueous phase was adjusted to pH 2.0 by the addition of hydrochloric acid, and extracted with ether (20 ml. × 2). The extracts were washed with water (5 ml. × 2) and dried over anhydrous sodium sulfate. After removing ether by distillation, the residue thus obtained was dissolved in another portion of ether (10 ml.), and a 2 M solution (0.8 ml.) of 2-ethylhexanoic acid potassium salt in n-butyl alcohol was added thereto. Ether was then added to cause white crystals to precipitate, which were then collected to yield the potassium salt of 6-[2-dichloromethylene-2-(2-thienyl)acetamido]penicillanic acid (0.57 g, 56%).

Melting point: 165—169°C. (dec.).

NMR δ ppm ($d_6$-DMSO): 1.46 (s, 3H), 1.53 (s, 3H), 4.03 (s, 1H), 5.46 (m, 2H), 7.10 (m, 1H), 7.20 (m, 1H), 7.66 (m, 1H), 9.59 (bs, 1H).

Elemental Analysis (for $C_{15}H_{13}Cl_2N_2O_4S_2K$).

Calculated: C: 39.21; H: 2.85; N: 6.09.
Found: C: 40.16; H: 3.60; N: 5.91.

## Synthesis Example 19

Phenylglyoxylic acid methyl ester (3.00 g, 18 mmol) and triphenylphosphine (4.79 g, 18 mmol) were dissolved in carbon tetrachloride (15 ml.), and the resulting solution was heated under reflux for 7 hours. n-Hexane was added to this reaction mixture, and then filtered. The filtrate was concentrated and distillated under reduced pressure to yield a fraction (1.15 g) having a boiling point of 95—105°C./1 mmHg. The reaction product thus obtained was dissolved in acetic acid (11 ml.), 6N hydrochloric acid (7 ml.) was added thereto, and the resulting mixture was heated under reflux for 24 hours. The reaction mixture was distilled off under reduced pressure. The crystals thus obtained were recrystallized from n-hexane to yield 2-dichloromethylene-2-phenylacetic acid (0.62 g, Yield: 16% based on phenylglyoxylic acid methyl ester.)

Melting point: 129—132.5°C.

NMR δ ppm ($CDCl_3$): 7.43 (s, 5H), 9.92 (s, 1H).

## Example 38

2-Dichloromethylene-2-phenylacetic acid (0.60 g, 2.76 mmol) was dissolved in chloroform (3 ml.), thionyl chloride (0.49 g, 4.14 mmol) was added thereto, and the resulting mixture was heated under reflux for 3 hours. After completion of the reaction, the reaction mixture was concentrated to give an oily residue (0.65 g).

On the other hand, 7 - aminocephalosporanic acid (0.89 g, 3.3 mmol) was suspended in anhydrous chloroform (8 ml.), hexamethyldisilazane (2.66 g, 16.5 mmol) was added thereto, and the resulting mixture was heated under reflux for 1.5 hours. After completion of the reaction, the chloroform and excess hexamethyldisilazane were removed by distillation under reduced pressure. Then, anhydrous chloroform (8 ml.) was added to the obtained residue, and the resulting mixture was stirred while cooling it to 0 to 5°C. To this mixture was added dropwise a solution of the above-obtained oily residue in chloroform (10 ml.). After stirring for 3 hours, the resulting reaction mixture was poured onto ice-water (60 ml.), and the pH-value was adjusted to 8 by the addition of sodium hydrogencarbonate, followed by extraction with ethyl acetate. The aqueous phase was separated, adjusted to pH 2 by the addition of a 10% hydrochloric acid, and extracted thrice with ethyl acetate (30 ml. each). The extracts were washed with aqueous sodium chloride, and dried over anhydrous sodium sulfate. The solvent was removed by distillation to yield 7 - (2 - dichloromethylene - 2 - phenylacetamido) - 3 - acetoxymethyl - 3 - cephem - 4 - carboxylic acid (0.93 g, 72%).

Melting point: 147—151°C. (dec.).

NMR δ ppm ($d_6$-acetone): 2.03 (s, 3H), 3.53 ($q_{AB}$, J=16.5Hz, 2H), 4.92 ($q_{AB}$, J=13.5Hz, 2H), 5.17 (d, J=6Hz, 1H), 5.83 (dd, J=6Hz, 9Hz, 1H), 7.00 (bs, 1H), 7.33—7.59 (m, 5H), 8.59 (d, J=9Hz, 1H).

Elemental Analysis (for $C_{19}H_{16}N_2O_6SCl_2$).

Calculated: C: 48.42; H: 3.42; N: 5.94.
Found: C: 48.07; H: 3.52; N: 5.79.

Synthesis Example 20

(1) 2-Thienylglyoxylic acid tert-butyl ester (2.0 g, 9.43 mmol) and triphenylphosphine (4.94 g, 18.86 mmol) were dissolved in carbon tetrachloride (15 ml.), and the resulting solution was heated under reflux for 6 hours. After the reaction was completed, the reaction mixture was concentrated under reduced pressure to afford a brownish-black oily residue. This residue was extracted with hexane. The hexane was distilled off from the obtained extract to yield 2-dichloromethylene-2-(2-thienyl)acetic acid tert-butyl ester (2.00 g, 76%).

NMR δ ppm ($CDCl_3$): 1.56 (s, 9H), 7.07 (m, 1H), 7.20 (m, 1H), 7.40 (m, 1H).

(2) 2-Dichloromethylene-2-(2-thienyl)acetic acid tert-butyl ester (2.00 g, 2.36 mmol) was dissolved in acetic acid (12 ml.), 6N hydrochloric acid (3 ml.) was added thereto, and the resulting mixture was heated under reflux for 2 hours. After the reaction was completed, the reaction mixture was concentrated under reduced pressure. The residue was dissolved in ether and washed with aqueous sodium chloride. The ether extract was dried over anhydrous sodium sulfate, the solvent was removed by distillation to yield 2-dichloromethylene-2-(2-thienyl)acetic acid (1.24 g, 78%).

Melting point: 108—113°C.

NMR δ ppm ($CDCl_3$): 7.00 (m, 1H), 7.18 (m, 1H), 7.40 (m, 1H), 10.43 (bs, 1H).

Example 39

Anhydrous dimethylformamide (0.33 g, 4.57 mmol) and phosphorus oxychloride (0.70 g, 4.57 mmol) were added to anhydrous ethyl acetate (2 ml.), and the Vilsmeier's reagent was prepared according to a conventional method. The obtained reaction mixture was cooled to 0 to 5°C., and a solution of 2-dichloromethylene-2-(2-thienyl)acetic acid (0.93 g, 4.17 mmol) in anhydrous ethyl acetate (10 ml.) was added dropwise to the reaction mixture with stirring. The mixture was stirred at that temperature for further one hour.

On the other hand, 7 - amino - 3 - (1 - methyl - 1H - tetrazol - 5 - yl)thiomethyl - 3 - cephem - 4 - carboxylic acid (1.85 g, 5.65 mmol) was suspended in anhydrous ethyl acetate (20 ml.), and bis(trimethylsilyl)acetamide (1.37 g, 6.75 mmol) was added thereto. This mixture was stirred for one hour at room temperature, and then cooled to −10°C. To the resulting mixture was added dropwise the previously prepared reaction mixture described above, and stirring was continued for 1.5 hours at that temperature. Water (30 ml.) was added to the reaction mixture. The pH-value of this mixture was adjusted to 7.5 to 8.0 by the addition of aqueous sodium hydrogencarbonate, and the ethyl acetate phase was separated. The aqueous phase was adjusted to pH 2 with a 10% hydrochloric acid and extracted thrice with ethyl acetate (30 ml. each). The combined ethyl acetate phase and extracts were washed with aqueous sodium chloride and dried over anhydrous sodium sulfate. The solvent was distilled off to yield 7 - [2 - dichloromethylene - 2 - (2 - thienyl)acetamido] - 3 - (1 - methyl - 1H - tetrazol - 5 - yl)thiomethyl - 3 - cephem - 4 - carboxylic acid (1.57 g, 71%).

Melting point: 120—124°C. (dec.).

NMR δ ppm ($d_6$-acetone): 3.76 ($q_{AB}$, J=16.5Hz, 2H), 3.94 (s, 3H), 4.36 ($q_{AB}$, J=16.5Hz, 2H), 5.21 (d, J=4.5Hz, 1H), 5.92 (dd, J=4.5Hz, 9Hz, 1H), 6.59 (bs, 1H), 7.07 (m, 1H), 7.28 (m, 1H), 7.59 (d, J=4.5Hz, 1H), 8.37 (bd, J=9Hz, 1H).

Elemental Analysis (for $C_{17}H_{14}N_6O_4S_3Cl_2$)

Calculated: C: 38.28; H: 2.65; N: 15.75.
Found: C: 38.27; H: 3.09; N: 14.56.

Example 40

By following the procedure of Example 38 but substituting 2 - dichloromethylene - 2 - (3 - thienyl)acetic acid for 2 - dichloromethylene - 2 - phenylacetic acid, 7 - [2 - dichloromethylene - 2 - (3 - thienyl)acetamido] - 3 - acetoxymethyl - 3 - cephem - 4 - carboxylic acid was prepared.

Melting point: 142—145°C. (dec.).

NMR δ ppm ($d_6$-acetone): 2.00 (s, 3H), 3.56 ($q_{AB}$, J=19.5Hz, 2H), 4.94 ($q_{AB}$, J=15Hz, 2H), 5.20 (d, J=4.5Hz, 1H), 5.89 (dd, J=4.5Hz, 9Hz, 1H), 7.28 (m, 1H), 7.50 (m, 1H), 7.71 (m, 1H), 8.63 (d, J=9Hz, 1H).

Elemental Analysis (for $C_{17}H_{14}Cl_2N_2O_6S_2$).

Calculated: C: 42.77; H: 2.96; N: 5.87.
Found: C: 41.90; H: 3.12; N: 5.72.

Example 41

By following the procedure of Example 38, but substituting 2 - dichloromethylene - 2 - (2 - thienyl)acetic acid for 2 - dichloromethylene - 2 - phenylacetic acid, 7 - [2 - dichloromethylene - 2 - (2 - thienyl)acetamido] - 3 - acetoxymethyl - 3 - cephem - 4 - carboxylic acid was obtained.

Melting point: 112—116°C. (dec.).

NMR δ ppm ($d_6$-acetone): 2.00 (s, 3H), 3.59 ($q_{AB}$, 2H), 4.92 ($q_{AB}$, J=13.5Hz, 2H), 5.24 (d, J=4.5Hz, 1H), 5.96 (dd, J=4.5Hz, 9Hz, 1H), 7.11 (m, 1H), 7.33 (m, 1H), 7.66 (d, J=4.5Hz, 1H), 8.74 (bs, 1H).

Elemental Analysis (for $C_{17}H_{14}Cl_2N_2O_6S_2$).

Calculated: C: 42.77; H: 2.96; N: 5.87.
Found: C: 41.81; H: 2.96; N: 5.68.

Test Example

Antibacterial activities of the compounds according to this invention, which had been prepared in the preceding Examples, were measured according to the method prescribed by The Committee of Amendment of "The method for measurement of the minimum inhibition concentration", The Chemotherapy Association, Japan (See Chemotherapy, Vol. 22, No. 6, pp. 1126—1128 (1974) and Vol. 29, No. 1, pp. 76—79 (1981)).

The microorganisms used in this Test Example as well as their corresponding abbreviations A to P are shown in Table 1. The abbreviations A to P will be used in the following Tables 2 to 8.

Test results are shown in terms of the minimum inhibition concentration (μg/ml.) in the following Tables 2 to 8.

TABLE 1

| Microorganism (strain) | Abbreviation |
|---|---|
| *Staphylococcus aureus* 6538–P | A |
| *Bacillus subtilis* ATCC 6633 | B |
| *Klebsiella pneumoniae* | C |
| *Salmonella typhimurium* 3173 | D |
| *Serratia marcescens* 1023 | E |
| *Serratia marcescens* IFO–3736 | F |
| *Proteus mirabillis* B–30–2 IFO–3849 | G |
| *Proteus vulgaris* OX–19 | H |
| *Proteus rettgeri* NIH–96 | I |
| *Proteus morgani Kono* | J |
| *Escherichia coli* NIHJ | K |
| *Escherichia coli* NIHJC–2 | L |
| *Pseudomonas aeruginosa* NCTC–10490 | M |
| *Enterobactor cloacae* NCTC–9394 | N |
| *Citrobactor freundii* NIH–10018–68 | O |
| *Proteus inconstans* NIH–118 | P |

TABLE 2

| Strain \ Compound | 1 | 5–(2) | 6–(2) | 6–(3) | Cefotaxime |
|---|---|---|---|---|---|
| A | 0.39 | 1.56 | 0.78 | 0.78 | 0.39 |
| B | 1.56 | 12.5 | 6.25 | 25 | 25 |
| C | 25 | 6.25 | 12.5 | 6.25 | 0.1 |
| D | 12.5 | 6.25 | 6.25 | 3.125 | 0.1 |
| E | 1.56 | 12.5 | 12.5 | 12.5 | 50 |
| F | 50 | 50 | 25 | 25 | 0.2 |
| G | 50 | 12.5 | 12.5 | 12.5 | 0.1 |
| H | 0.2 | 0.2 | 0.1 | 0.1 | <0.025 |
| I | <0.025 | <0.025 | <0.025 | <0.025 | <0.025 |
| J | 50 | 25 | 6.25 | 6.25 | 1.56 |
| K | <0.025 | 0.1 | <0.025 | 0.05 | <0.025 |
| L | 25 | 12.5 | 12.5 | 12.5 | 0.2 |
| M | 3.125 | 6.25 | 3.125 | 3.125 | 0.78 |
| N | 6.25 | 1.56 | 1.56 | 1.56 | 0.39 |
| O | 12.5 | 6.25 | 6.25 | 6.25 | 0.2 |

TABLE 3

| Strain \ Compound | 7–(3) | 8–(3) | 9–(2) | 10–(1) | Cefotaxime |
|---|---|---|---|---|---|
| A | 6.25 | 25 | 0.78 | 3.125 | 0.39 |
| B | 12.5 | 100 | 3.125 | 12.5 | 0.78 |
| C | 12.5 | >100 | 12.5 | 12.5 | 0.1 |
| D | 6.25 | >100 | 12.5 | 12.5 | 0.1 |
| E | – | – | – | – | – |
| F | 25 | >100 | 50 | 50 | 0.39 |
| G | 1.56 | 100 | 25 | 25 | 0.1 |
| H | 0.1 | 12.5 | 0.2 | 0.78 | <0.025 |
| I | <0.025 | 0.2 | <0.025 | 0.05 | <0.025 |
| J | 3.125 | >100 | 12.5 | 12.5 | 0.78 |
| K | <0.025 | 6.25 | <0.025 | 0.1 | <0.025 |
| L | 25 | 100 | 25 | 12.5 | 0.2 |
| M | 6.25 | >100 | 12.5 | 25 | 0.39 |
| N | 3.125 | 100 | 3.125 | 3.125 | 0.2 |
| O | 12.5 | >100 | 12.5 | 12.5 | 0.2 |

TABLE 4

| Strain \ Compound | 11–(3) | 20 | 21 | 22 | 23 | 24 | Cefotaxime |
|---|---|---|---|---|---|---|---|
| A | 0.39 | 1.56 | 0.2 | 0.78 | 3.125 | 6.25 | 0.39 |
| B | 3.125 | 6.25 | 3.125 | 6.25 | 50 | 25 | 6.25 |
| C | 0.78 | 1.56 | 3.125 | 0.78 | 50 | 25 | 0.05 |
| D | 0.78 | 3.125 | 3.125 | 1.56 | 50 | 25 | 0.1 |
| E | 6.25 | 12.5 | 3.125 | 6.25 | 100 | 25 | 100 |
| F | 0.78 | 6.25 | 3.125 | 1.56 | >100 | 50 | 0.2 |
| G | 0.39 | 1.56 | 1.56 | 0.39 | 25 | 12.5 | 0.025 |
| H | 0.025 | 0.2 | 0.025 | 0.1 | 12.5 | 3.125 | 0.0125 |
| I | <0.00625 | 0.025 | <0.00625 | 0.0125 | 0.39 | 0.78 | <0.00625 |
| J | 0.2 | 1.56 | 0.78 | 0.39 | >100 | 25 | 0.39 |
| K | <0.00625 | 0.05 | <0.00625 | <0.00625 | 6.25 | 1.56 | <0.00625 |
| L | 0.78 | 3.125 | 1.56 | 3.125 | 100 | 25 | 0.1 |
| M | 1.56 | 6.25 | 6.25 | 6.25 | >100 | >100 | 0.39 |
| N | 0.2 | 0.78 | 0.39 | 0.39 | 12.5 | 12.5 | 0.2 |
| O | 0.78 | 1.56 | 3.125 | 1.56 | 100 | 25 | 0.2 |

TABLE 5

| Strain \ Compound | 2 | 3 | 4–(1) | 4–(2) | 5–(1) | 37 | Cephalothin |
|---|---|---|---|---|---|---|---|
| A | 0.39 | 0.39 | 0.39 | 0.39 | 1.56 | 0.10 | 0.10 |
| B | 3.125 | 3.125 | 6.25 | 1.56 | 25 | 1.56 | 0.20 |
| C | >100 | 50 | >100 | >100 | 12.50 | >100 | 6.25 |
| D | 100 | 12.50 | >100 | 100 | 12.50 | >100 | 3.175 |
| E | 6.25 | 0.25 | 6.25 | 3.125 | 25 | 0.05 | 0.10 |
| F | >100 | 50 | >100 | >100 | 12.50 | >100 | >100 |
| G | >100 | 50 | >100 | >100 | 12.50 | >100 | 12.50 |
| H | 6.25 | 0.76 | >100 | 6.25 | 0.39 | 50 | 25 |
| I | 0.20 | 0.10 | 6.25 | 0.20 | <0.025 | 0.25 | 0.78 |
| J | >100 | 100 | >100 | >100 | 12.50 | >100 | >100 |
| K | 0.39 | 0.20 | 6.25 | 0.39 | 0.10 | 0.78 | 0.78 |
| L | >100 | 50 | >100 | >100 | 25 | >100 | 12.50 |
| M | 6.25 | 6.25 | 50 | 12.50 | 12.50 | 6.25 | >100 |
| N | >100 | 12.50 | >100 | >100 | 12.50 | 100 | 50 |
| O | >100 | 25 | >100 | >100 | 25 | >100 | 25 |

TABLE 6

| Strain \ Compound | 27 | 28 | 29 | 30 | 34 | Cephalothin |
|---|---|---|---|---|---|---|
| A | >100 | >100 | >100 | 100 | 25 | 6.25 |
| B | 3.125 | 3.125 | 1.56 | 0.78 | 0.78 | 0.05 |
| C | >100 | >100 | >100 | >100 | 100 | 25 |
| D | >100 | >100 | >100 | >100 | 12.5 | 6.25 |
| E | 6.25 | 6.25 | 12.5 | 6.25 | 1.56 | 0.39 |
| F | >100 | >100 | >100 | >100 | 100 | >100 |
| G | >100 | >100 | >100 | >100 | 100 | 25 |
| H | >100 | 100 | >100 | >100 | 12.5 | >100 |
| I | 3.125 | 3.125 | 3.125 | 6.25 | 0.05 | >100 |
| J | >100 | >100 | >100 | >100 | 100 | >100 |
| K | 100 | >100 | 100 | 100 | 1.56 | 25 |
| L | >100 | >100 | >100 | >100 | 25 | 25 |
| M | 100 | 50 | >100 | 100 | 100 | >100 |
| N | >100 | >100 | >100 | >100 | 12.5 | >100 |
| O | >100 | >100 | >100 | >100 | 100 | >100 |

TABLE 7

| Strain \ Compound | 31 | 32 | 33 | 35 | 36 | Cephalothin |
|---|---|---|---|---|---|---|
| A | 0.2 | 0.1 | 0.39 | 0.2 | 0.78 | 0.1 |
| B | 6.25 | 6.25 | 6.25 | 3.125 | 12.5 | 0.05 |
| C | >100 | >100 | 100 | 50 | >100 | 25 |
| D | >100 | >100 | 50 | 25 | >100 | 6.25 |
| E | 100 | 12.5 | 25 | 3.125 | 100 | 0.78 |
| F | >100 | >100 | 100 | 100 | >100 | >100 |
| G | >100 | >100 | 100 | 100 | >100 | 12.5 |
| H | 100 | 100 | 6.25 | 25 | 50 | 100 |
| I | 1.56 | 0.78 | 0.1 | 0.1 | 1.56 | 12.5 |
| J | >100 | >100 | >100 | >100 | >100 | >100 |
| K | 25 | 25 | 1.56 | 3.125 | 50 | 12.5 |
| L | >100 | >100 | 100 | 50 | >100 | 50 |
| M | 100 | 50 | 12.5 | 12.5 | >100 | >100 |
| N | >100 | >100 | 25 | 12.5 | >100 | >100 |
| O | >100 | >100 | 50 | 100 | >100 | >100 |

TABLE 8

| Compound / Strain | 38 | 39 | 40 | 41 | Cephalothin Na salt |
|---|---|---|---|---|---|
| A | 1.56 | 0.2 | 0.39 | 0.2 | 0.1 |
| I | – | 0.05 | 0.39 | 50 | >100 |
| H | – | 50 | 25 | 0.39 | >100 |
| K | 3.10 | 0.39 | 1.56 | 0.39 | 25 |
| M | >100 | 6.25 | 25 | 25 | >100 |
| N | – | 12.50 | 100 | 50 | >100 |
| P | – | 3.125 | 25 | 1.56 | >100 |

**Claims**

1. A compound having the following formula:

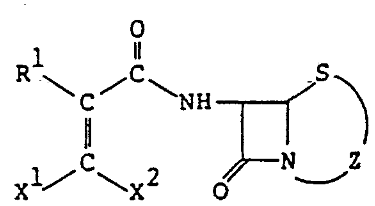

in which $R^1$ denotes an aromatic group selected from the group consisting of a phenyl radical, a thiazolyl radical, a thienyl radical, a furyl radical and a pyrrolyl radical, which may be substituted by a halogen atom, a nitro radical, an amino radical, a cyano radical, a hydroxy radical, a lower alkyl radical having 1 to 4 carbon atoms, a lower alkoxy radical having 1 to 4 carbon atoms, a lower alkylthio radical having 1 to 4 carbon atoms; Z represents a group

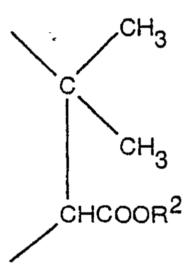

or

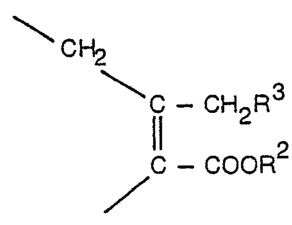

(wherein $R^2$ denotes hydrogen, a salt forming cation or a protective group for carboxylic acid, and $R^3$ denotes hydrogen or a residue selected from the group consisting of a hydroxyl radical, a mercapto radical, a lower aliphatic carboxylic acid acyloxy radical having 2 to 4 carbon atoms, an aromatic carboxylic acid acyloxy radical selected from the group consisting of mandelyloxy, 2-carboxy-benzoyloxy, 2-(carboethoxycarbamoyl)benzoyloxy and 2-(carboethoxysulfamoyl)benzoyloxy, a carbymoyloxy radical, a phenylglycyloxy radical, a quaternary ammonium salt and a heterocyclic ring which is bound via S selected from the group comprising pyridyl, N-oxidepyridyl, pyridazinyl, N-oxidepyridazinyl, pyrazolyl, diazolyl,

thiazolyl, thiadiazolyl, oxydiazolyl, triazolyl, and tetrazolyl, which may be substituted by a lower alkyl having from 1 to 3 carbon atoms, a lower alkoxy radical having from 1 to 3 carbon atoms, a halogen atom, a trihalogeno-substituted lower alkyl radical having from 1 to 3 carbon atoms, a hydroxy radical, a mercapto radical, an amino radical, a carboxyl radical, a carbamoyl radical, a di-lower alkylamino lower alkyl having from 1 to 3 carbon atoms or a carboxymethyl radical; and $X^1$ and $X^2$ each represent hydrogen or a halogen atom, provided that both $X^1$ and $X^2$ cannot represent hydrogen at the same time.

2. A compound as claimed in Claim 1 wherein $X^1$ and $X^2$ each are a chlorine atom or a hydrogen.

3. A compound as claimed in Claim 1 which is selected from the group consisting of

7-[2-dichloromethylene-2-(2-furyl)acetamido]-3-(1-methyl-1H-tetrazol-5-yl)thiomethyl-3-cephem-4-carboxylic acid,

7-[2-dichloromethylene-2-(4-hydroxyphenyl)acetamido]-3-acetoxymethyl-3-cephem-4-carboxylic acid,

7-[2-dichloromethylene-2-(3-hydroxyphenyl)acetamido]-3-acetoxymethyl-3-cephem-4-carboxylic acid,

7-[2-dichloromethylene-2-(4-tert-butoxycarbonylaminophenyl)acetamido]-3-acetoxymethyl-3-cephem-4-carboxylic acid,

7-[2-dichloromethylene-2-(4-aminophenyl)acetamido]-3-acetoxymethyl-3-cephem-4-carboxylic acid,

7-[2-dichloromethylene-2-(2-tert-butoxycarbonylaminothiazol-4-yl)acetamido]-3-acetoxymethyl-3-cephem-4-carboxylic acid,

7-[2-dichloromethylene-2-(2-aminothiazol-4-yl)acetamido]-3-acetoxymethyl-3-cephem-4-carboxylic acid,

7-[2-dichloromethylene-2-(2-tert-butoxycarbonylaminothiazol-4-yl)acetamido]-3-(1-methyl-1H-tetrazol-5-yl)thiomethyl-3-cephem-4-carboxylic acid,

7-[2-dichloromethylene-2-(2-aminothiazol-4-yl)acetamido]-3-(1-methyl-1H-tetrazol-5-yl)thiomethyl-3-cephem-4-carboxylic acid,

7-[2-dichloromethylene-2-(2-tert-butoxycarbonylaminothiazol-4-yl)acetamido]-3-(1-carboxymethyltetrazol-5-yl)thiomethyl-3-cephem-4-carboxylic acid,

7-[2-dichloromethylene-2-(2-aminothiazol-4-yl)acetamido]-3-(1-carboxymethyltetrazol-5-yl)thiomethyl-3-cephem-4-carboxylic acid,

7-[2-dichloromethylene-2-(2-aminothiazol-4-yl)acetamido]-3-(1-carboxymethyltetrazol-5-yl)thiomethyl-3-cephem-4-carboxylic acid,

7-[2-dichloromethylene-2-(2-tert-butoxycarbonylaminothiazol-4-yl)acetamido]desacetoxy-cephalosporanic acid,

7-[2-dichloromethylene-2-(2-aminothiazol-4-yl)acetamido]desacetoxycephalosporanic acid,

7-[2-dichloromethylene-2-(2-aminothiazol-4-yl)acetamido]-3-(2-methylthio-1,3,4-thiadiazol-5-yl)thiomethyl-3-cephem-4-carboxylic acid,

7-[2-dichloromethylene-2-(2-aminothiazol-4-yl)acetamido]-3-[1-(2-dimethylaminoethyl)-1H-tetrazol-5-yl]thiomethyl-3-cephem-4-carboxylic acid,

7-[2-chloromethylene-2-(2-tert-butoxycarbonylaminothiazol-4-yl)acetamido]-3-(1-methyl-1H-tetrazol-5-yl)thiomethyl-3-cephem-4-carboxylic acid,

7-[2-chloromethylene-2-(2-aminothiazol-4-yl)acetamido]-3-(1-methyl-1H-tetrazol-5-yl)thiomethyl-3-cephem-4-carboxylic acid,

7-[2-chloromethylene-2-(2-tert-butoxycarbonylaminothiazol-4-yl)acetamido]-3-acetoxymethoxy-3-cephem-4-carboxylic acid,

7-[2-chloromethylene-2-(2-tert-butoxycarbonylaminothiazol-4-yl)acetamido]-3-(2-methylthio-1,3,4-thiadiazol-5-yl)thiomethyl-3-cephem-4-carboxylic acid,

7-[2-chloromethylene-2-(2-tert-butoxycarbonylaminothiazol-4-yl)acetamido]-3-[(2,5-dihydro-6-hydroxy-2-methyl-5-oxo-as-triazin-3-yl)thiomethyl]-3-cephem-4-carboxylic acid,

7-[2-chloromethylene-2-(2-tert-butoxycarbonylaminothiazol-4-yl)acetamido]desacetoxycephalosporanic acid,

7-[2-chloromethylene-2-(2-aminothiazol-4-yl)acetamido]-3-acetoxymethyl-3-cephem-4-carboxylic acid,

7-[2-chloromethylene-2-(2-aminothiazol-4-yl)acetamido]-3-(2-methylthio-1,3,4-thiadiazol-5-yl)thiomethyl-3-cephem-4-carboxylic acid,

7-[2-chloromethylene-2-(2-aminothiazol-4-yl)acetamido]-3-[(2,5-dihydro-6-hydroxy-2-methyl-5-oxo-as-triazin-3-yl)thiomethyl]-3-cephem-4-carboxylic acid,

7-[2-chloromethylene-2-(2-aminothiazol-4-yl)acetamido]desacetoxycephalosporanic acid,

7-[2-chloromethylene-2-(2-aminothiazol-4-yl)acetamido]-3-pyridiniummethyl-3-cephem-4-carboxylic acid betaine.

7-[2-chloromethylene-2-(2-formylaminothiazol-4-yl)acetamido]-3-(1-methyl-1H-tetrazol-5-yl)thiomethyl-3-cephem-4-carboxylic acid,

7-[2-dichloromethylene-2-(4-methoxyphenyl)acetamido]-3-cephem-4-carboxylic acid,

7-[2-dichloromethylene-2-(4-methylphenyl)acetamido]-3-acetoxymethyl-3-cephem-4-carboxylic acid,

7-[2-dichloromethylene-2-(4-chlorophenyl)acetamido]-3-acetoxymethyl-3-cephem-4-carboxylic acid,

7-[2-dichloromethylene-2-(3,4-dichlorophenyl)acetamido]-3-acetoxymethyl-3-cephem-4-carboxylic acid,

7-[2-dichloromethylene-2-(5-chloro-2-thienyl)acetamido]-3-acetoxymethyl-3-cephem-4-carboxylic acid,

7-[2-dichloromethylene-2-(5-bromo-2-thienyl)acetamido]-3-acetoxymethyl-3-cephem-4-carboxylic acid,

7-[2-dichloromethylene-2-(2-furyl)acetamido]-3-acetoxymethyl-3-cephem-4-carboxylic acid;

7-[2-chloromethylene-2-(2-thienyl)acetamido]-3-(1-methyl-1H-tetrazol-5-yl)thiomethyl-3-cephem-4-carboxylic acid,

7-[2-dichloromethylene-2-(3-thienyl)acetamido]-3-(1-methyl-1H-tetrazol-5-yl)thiomethyl-3-cephem-4-carboxylic acid,

7-[2-dichloromethylene-2-(1-methyl-2-pyrrolyl)acetamido]-3-acetoxymethyl-3-cephem-4-carboxylic acid,

7-(2-dichloromethylene-2-phenylacetamido)-3-acetoxymethyl-3-cephem-4-carboxylic acid,

7-[2-dichloromethylene-2-(2-thienyl)acetamido]-3-(1-methyl-1H-tetrazol-5-yl)thiomethyl-3-cephem-4-carboxylic acid,

7-[2-dichloromethylene-2-(3-thienyl)acetamido]-3-acetoxymethyl-3-cephem-4-carboxylic acid,

7-[2-dichloromethylene-2-(2-thienyl)acetamido]-3-acetoxymethyl-3-cephem-4-carboxylic acid,

7-[2-dichloromethylene-2-(2-aminothiazol-4-yl)acetamido]-3-carbamoyloxymethyl-3-cephem-4-carboxylic acid, and

7-[2-chloromethylene-2-(2-aminothiazol-4-yl)acetamido]-3-carbamoyloxymethyl-3-cephem-4-carboxylic acid.

4. A compound as claimed in Claim 1 which is 6-[2-dichloromethylene-2-(2-thienyl)acetamido]-penicillanic acid.

5. A compound as claimed in Claim 1 which is 6-[2-chloromethylene-2-(2-thienyl)acetamido]penicillanic acid.

6. A compound as claimed in Claim 3 which is 7-[2-chloromethylene-2-(2-aminothiazol-4-yl)acetamido]-3-(1-methyl-1H-tetrazol-5-yl)thiomethyl-3-cephem-4-carboxylic acid.

7. A compound as claimed in Claim 3 which is 7-[2-chloromethylene-2-(2-aminothiazol-4-yl)acetamido]-3-acetoxymethyl-3-cephem-4-carboxylic acid.

8. A compound as claimed in Claim 3 which is 7 - [2 - chloromethylene - 2 - (2 - tert - butoxycarbonylaminothiazol - 4 - yl)acetamido] - 3 - (2 - methylthio - 1,3,4 - thiadiazol - 5 - yl)thiomethyl - 3 - cephem - 4 - carboxylic acid.

9. A compound as claimed in Claim 3 which is 7-[2-dichloromethylene-2-(2-aminothiazol-4-yl)acetamido]-3-acetoxymethyl-3-cephem-4-carboxylic acid.

10. A compound as claimed in Claim 3 which is 7 - [2 - dichloromethylene - 2 - (2 - aminothiazol - 4 - yl)acetamido] - 3 - (1 - methyl - 1H - tetrazol - 5 - yl)thiomethyl - 3 - cephem - 4 - carboxylic acid.

11. A compound as claimed in Claim 3 which is 7 - [2 - dichloromethylene - 2 - (2 - aminothiazol - 4 - yl)acetamido] - 3 - (2 - methylthio - 1,3,4 - thiadiazol - 5 - yl)thiomethyl - 3 - cephem - 4 - carboxylic acid.

12. A process for the preparation of a compound having the formula:

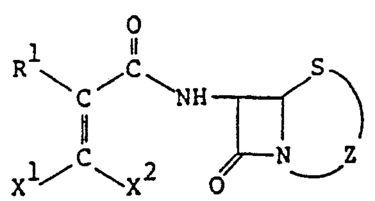

in which $R^1$ has the same meaning as given in Claim 1; Z represents a group

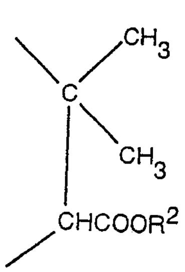

or

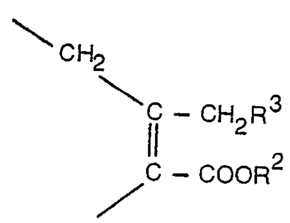

(wherein $R^2$ denotes hydrogen, a salt forming cation or a protective group for carboxylic acid, and $R^3$ has the same meaning as given in Claim 1); and $X^1$ and $X^2$ each represent hydrogen or a halogen atom, provided that both $X^1$ and $X^2$ cannot represent hydrogen at the same time, which comprises reacting a compound having the formula:

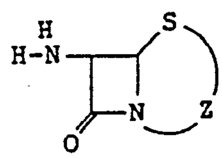

wherein Z has the same meaning as defined above,
a reactive derivative thereof with respect to the amino radical or a salt thereof, with an acid having the formula:

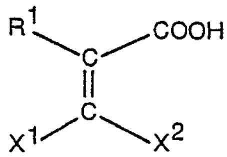

wherein $R^1$, $X^1$ and $X^2$ have the same meanings as defined above,
a reactive derivative thereof with respect to the carboxyl radical or a salt thereof.

13. A process as claimed in Claim 12 wherein the reaction is caried out in the presence of a dehydration agent or a condensing agent when the acid is reacted in the form of a free acid.

14. A process as claimed in Claim 12 wherein said reactive derivative of the acid is an acid chloride, an acid anhydride, a mixed acid anhydride, an active acid amide, an acid cyanide, an active ester or an acid azide.

15. A process as claimed in Claim 12 wherein the reaction is carried out in the presence of a solvent with cooling or at room temperature.

16. A process for the preparation of a compound having the formula:

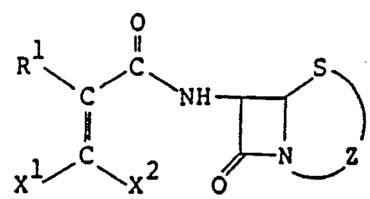

in which $R^1$ has the same meaning as given in Claim 1; Z represents a group

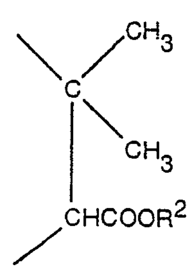

or

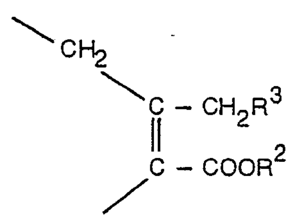

(wherein $R^2$ denotes hydrogen, a salt forming cation or a protective group for a carboxylic acid, and $R^3$ has the same meaning as given in Claim 1); and $X^1$ and $X^2$ each represent hydrogen or a halogen atom, provided that both $X^1$ and $X^2$ cannot represent hydrogen at the same time,
which comprises the first step of reacting a compound having the formula:

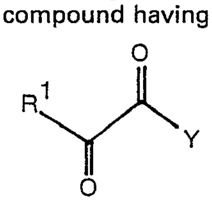

wherein $R^1$ has the same meaning as defined above; and Y represents a hydroxyl group or a protective group for a carboxylic acid,
with a Wittig reagent having the formula:

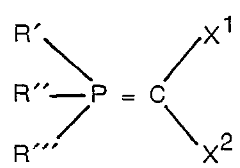

wherein R′, R″ and R‴ may be the same or different and each represent an aryl radical or an alkyl radical; and $X^1$ and $X^2$ have the same meanings as defined above,
to form a compound having the formula:

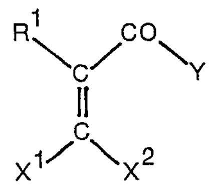

wherein $R^1$, $X^1$, $X^2$ and Y have the same meanings as defined above,
and the second step of reacting the thus formed compound, a reactive derivative thereof or a salt thereof, with a compound having the formula:

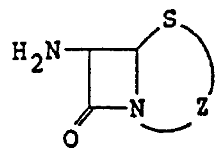

wherein Z has the same meaning as defined above,
a reactive derivative thereof with respect to the amino radical or a salt thereof.

17. A process as claimed in Claim 16 wherein R′, R″ and R‴ represent an aryl group.

18. A process as claimed in Claim 17 wherein R′, R″ and R‴ represent a phenyl group.

19. A process for the preparation of a compound having the formula:

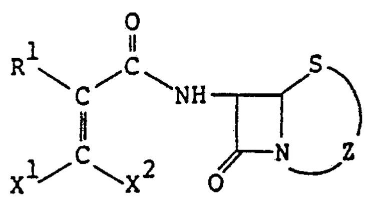

in which $R^1$ has the same meaning as given in Claim 1; Z represents a group

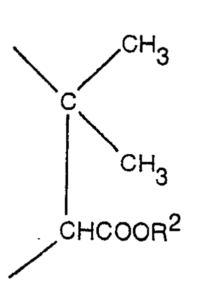

or

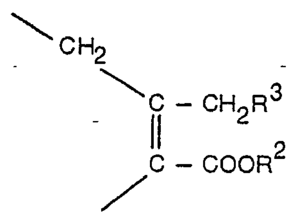

(wherein $R^2$ denotes hydrogen, a salt forming cation or a protective group for a carboxylic acid, and $R^3$ has the same meaning as given in Claim 1); and $X^1$ and $X^2$ each represent hydrogen or a halogen atom, provided that both $X^1$ and $X^2$ cannot represent hydrogen or a halogen atom at the same time,
which comprises the first step of reacting a compound having the formula:

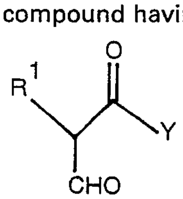

wherein $R^1$ has the same meaning as defined above; and Y represents a hydroxyl group or a protective group for a carboxylic acid,
with a halogenating agent to form a compound having the formula:

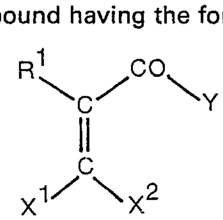

wherein $R^1$, $X^1$, $X^2$ and Y have the same meanings as defined above, and the second step of reacting the thus formed compound, a reactive derivative thereof or a salt thereof, with a compound having the formula:

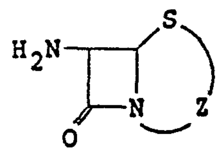

wherein Z has the same meanings as defined above, a reactive derivative thereof with respect to the amino radical or a salt thereof.

20. A process as claimed in Claim 19 wherein said halogenating agent is selected from the group consisting of phosphorus pentachloride, phosphorus oxychloride, thionyl chloride, phosphorus trichloride, phosgene and the Vilsmeier's reagent prepared from the above agent and dimethylformamide.

21. A compound having the formula:

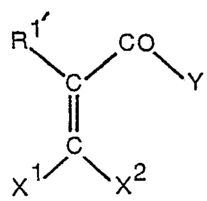

wherein $R^{1'}$ denotes an aromatic group selected from the group consisting of a thiazolyl radical, a thienyl radical, a furyl radical and a pyrrolyl radical, which may be substituted by a halogen atom, a nitro radical, an amino radical, a cyano radical, a hydroxy radical, a lower alkyl radical having 1 to 4 carbon atoms, a lower alkoxy radical having 1 to 4 carbon atoms; a lower alkylthio radical having 1 to 4 carbon atoms; $X^1$ and $X^2$ each represent hydrogen or a halogen atom, provided that $X^1$ and $X^2$ cannot represent hydrogen or a halogen atom at the same time; and Y denotes a hydroxyl group, a halogen atom, or a radical —OR (in which R stands for protective radical for a carboxylic acid).

22. A compound as claimed in Claim 21 which is selected from the group consisting of
2-chloromethylene-2-(2-tert-butoxycarbonylaminothiazol-4-yl)acetic acid ethyl ester,
2-chloromethylene-2-(2-tert-butoxycarbonylaminothiazol-4-yl)acetic acid,
2-chloromethylene-2-(2-aminothiazol-4-yl)acetic acid ethyl ester,
2-chloromethylene-2-(2-aminothiazol-4-yl)acetic acid ethyl ester,
2-chloromethylene-2-(2-aminothiazol-4-yl)acetic acid,
2-chloromethylene-2-(2-formylaminothiazol-4-yl)acetic acid ethyl ester,
2-chloromethylene-2-(2-formylaminothiazol-4-yl)acetic acid,
2-monochloromethylene-2-(2-thienyl)acetic acid tert-butyl ester,
2-chloromethylene-2-(2-thienyl)acetic acid.

**Patentansprüche**

1. Verbindung der folgenden Formel

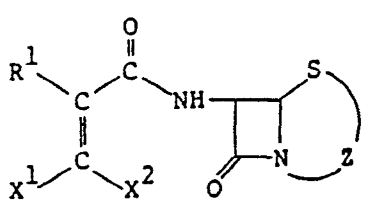

worin $R^1$ eine aromatische Gruppe darstellt, die ausgewählt ist aus der Gruppe, bestehend aus einem Phenylrest, einem Thiazolylrest, einem Thienylrest einem Furylrest und einem Pyrrolylrest, welche substituiert sein können mit einem Halogenatom, einer Nitrogruppe, einer Aminogruppe, einer Cyangruppe, einer Hydroxygruppe, einem Niedrigalkylrest mit 1 bis 4 Kohlenstoffatomen, einem Niedrigalkoxyrest mit 1 bis 4 Kohlenstoffatomen, einem Niedrigalkylthiorest mit 1 bis 4 Kohlenstoffatomen; Z eine Gruppe

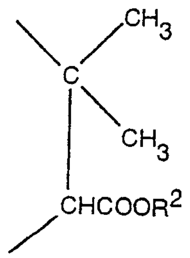

oder

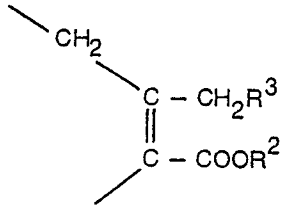

bedeutet (wobei $R^2$ Wasserstoff, ein salzbildendes Kation oder eine Schutzgruppe für Carbonsäuren darstellt und $R^3$ Wasserstoff oder einen Rest bedeutet, der ausgewählt ist aus der Gruppe bestehend aus einem Hydroxylrest, einem Mercaptorest, einem Acyloxyrest einer niedrigen aliphatischen Carbonsäure mit 2 bis 4 Kohlenstoffatomen, einem Acyloxyrest einer aromatischen Carbonsäure, ausgewählt aus der Gruppe, bestehend aus Mandelyloxy, 2-Carboxy-benzoyloxy, 2-(Carboethoxycarbamoyl)benzoyloxy und 2-(Carboethoxysulfamoyl)benzoyloxy, einem Carbymoyloxyrest, einem Phenylglycyloxyrest, einem quaternären Ammoniumsalz und einem heterocyclischen Ring, welcher über S gebunden ist, ausgewählt aus der Gruppe, bestehend aus Pyridyl, N-Oxidpyridyl, Pyridazinyl, N-Oxid-pyridazinyl, Pyrazolyl, Diazolyl, Thiazolyl, Thiadiazolyl, Oxydiazolyl, Triazolyl und Tetrazolyl, welcher substituiert sein kann mit einem Niedrigalkyl mit 1 bis 3 Kohlenstoffatatomen, einem Niedrigalkoxyrest mit 1 bis 3 Kohlenstoffatomen, einem Halogenatom, einem Trihalogen-substituierten Niedrigalkylrest mit 1 bis 3 Kohlenstoffatomen, einem Hydroxyrest, einem Mercaptorest, einem Aminorest, einem Carboxylrest, einem Carbamoylrest, einem Di-niedrig-alkylamino-niedrig-alkyl mit 1 bis 3 Kohlenstoffatomen oder einem Carboxymethylrest; und $X^1$ und $X^2$ jeweils Wasserstoff oder ein Halogenatom bedeuten, vorausgesetzt, dass sowohl $X^1$ und $X^2$ nicht gelichzeitig Wasserstoff darstellen können.

2. Verbindung nach Anspruch 1, wobei $X^1$ und $C^2$ jeweils ein Chloratom oder ein Wasserstoffatom darstellen.

3. Verbindung nach Anspruch 1, ausgewählt aus der Gruppe, bestehend aus:

7-[2-Dichloromethylen-2-(2-furyl)-acetamido]-3-(1-methyl-1H-tetrazol-5-yl)thiomethyl-3-cephem-4-carbonsäure,

7-[2-Dichloromethylen-2-(4-hydroxyphenyl)acetamido]-3-acetoxymethyl-3-cephem-4-carbonsäure,

7-[2-Dichloromethylen-2-(3-hydroxyphenyl)acetamido]-3-acetoxymethyl-3-cephem-4-carbonsäure,

7-[2-Dichloromethylen-2-(4-tert-butoxycarbonylaminophenyl)acetamido]-3-acetoxymethyl-3-cephem-4-carbonsäure,

7-[2-Dichloromethylen-2-(4-aminophenyl)acetamido]-3-acetoxymethyl-3-cephem-4-carbonsäure,

7-[2-Dichloromethylen-2-(2-tert-butoxycarbonylaminothiazol-4-yl)acetamido]-3-acetoxymethyl-3-cephem-4-carbonsäure,

7-[2-Dichloromethylen-2-(2-aminothiazol-4-yl)acetamido]-3-acetoxymethyl-3-cephem-4-carbonsäure,

7-[2-Dichloromethylen-2-(2-tert-butoxycarbonylaminothiazol-4-yl)acetamido]-3-(1-methyl-1H-tetrazol-5-yl)thiomethyl-3-cephem-4-carbonsäure,

7-[2-Dichloromethylen-2-(2-aminothiazol-4-yl)acetamido]-3-(1-methyl-1H-tetrazol-5-yl)thiomethyl-3-cephem-4-carbonsäure,

7-[2-Dichloromethylen-2-(2-tert-butoxycarbonylaminothiazol-4-yl)acetamido]-3-(1-carboxymethyltetrazol-5-yl)thiomethyl-3-cephem-4-carbonsäure,

7-[2-Dichloromethylen-2-(2-aminothiazol-4-yl)acetamido]-3-(1-carboxymethyltetrazol-5-yl)thiomethyl-3-cephem-4-carbonsäure,

7-[2-Dichloromethylen-2-(2-aminothiazol-4-yl)acetamido]-3-(1-carboxymethyltetrazol-5-yl)thiomethyl-3-cephem-4-carbonsäure,

7-[2-Dichloromethylen-2-(2-tert-butoxycarbonylaminothiazol-4-yl)acetamido]desacetoxycephalosporansäure,

7-[2-Dichloromethylen-2-(2-aminothiazol-4-yl)acetamido]desacetoxycephalosporansäure,

7-[2-Dichloromethylen-2-(aminothiazol-4-yl)acetamido]-3-(2-methylthio-1,3,4-thiadiazol-5-yl)-thiomethyl-3-cephem-4-carbonsäure,

7-[2-Dichloromethylen-2-(2-aminothiazol-4-yl)acetamido]-3-[1-(2-dimethylaminoethyl)-1H-tetrazol-5-yl]thiomethyl-3-cephem-4-carbonsäure,

7-[2-Chloromethylen-2-(2-tert-butoxycarbonylaminothiazol-4-yl)acetamido]-3-(1-methyl-1H-tetrazol-5-yl)thiomethyl-3-cephem-4-carbonsäure,

7-[2-Chloromethylen-2-(2-aminothiazol-4-yl)acetamido]-3-(1-methyl-1H-tetrazol-5-yl)thiomethyl-3-cephem-4-carbonsäure,

7-[2-Chloromethylen-2-(2-tert-butoxycarbonylaminothiazol-4-yl)acetamido]-3-acetoxymethyl-3-cephem-4-carbonsäure,

7-[2-Chloromethylen-2-(2-tert-butoxycarbonylaminothiazol-4-yl)acetamido]-3-(2-methylthio-1,3,4-thiadiazol-5-yl)thiomethyl-3-cephem-4-carbonsäure,

7-[2-Chloromethylen-2-(2-tert-butoxycarbonylaminothiazol-4-yl)acetamido]-3-[(2,5-dihydro-6-hydroxy-2-methyl-5-oxo-as-triazin-3-yl)thiomethyl]-3-cephem-4-carbonsäure,

7-[2-Chloromethylen-2-(2-tert-butoxycarbonylaminothiazol-4-yl)acetamido]-desacetoxycephalosporansäure,

7-[2-Chloromethylen-2-(2-aminothiazol-4-yl)acetamido]-3-acetoxymethyl-3-cephem-4-carbonsäure,

7-[2-Chloromethylen-2-(2-aminothiozol-4-yl)acetamido]-3-(2-methylthio-1,3,4-thiadiazol-5-yl)-thiomethyl-3-cephem-4-carbonsäure,

7-[2-Chloromethylen-2-(2-aminothiazol-4-yl)acetamido]-3-[(2,5-dihydro-6-hydroxy-2-methyl-5-oxo-as-triazin-3-yl)thiomethyl]-3-cephem-4-carbonsäure,

7-[2-Chloromethylen-2-(2-aminothiazol-4-yl)acetamido]desacetoxycephalosporansäure,

7-[2-Chloromethylen-2-(2-aminothiazol-4-yl)acetamido]-3-pyridiniummethyl-3-cephem-4-carbonsäure-betain,

7-[2-Chloromethylen-2-(2-formylaminothiazol-4-yl)acetamido]-3-(1-methyl-1H-tetrazol-5-yl)-thiomethyl-3-cephem-4-carbonsäure,

7-[2-Dichloromethylen-2-(4-methoxyphenyl)acetamido]-3-cephem-4-carbonsäure,

7-[2-Dichloromethylen-2-(4-methylphenyl)acetamido]-3-acetoxymethyl-3-cephem-4-carbonsäure,

7-[2-Dichloromethylen-2-(4-chlorophenyl)acetamido]-3-acetoxymethyl-3-cephem-4-carbonsäure,

7-[2-Dichloromethylen-2-(3,4-dichlorophenyl)acetamido]-3-acetoxymethyl-3-cephem-4-carbonsäure,

7-[2-Dichloromethylen-2-(5-chloro-2-thienyl)acetamido]-3-acetoxymethyl-3-cephem-4-carbonsäure,

7-[2-Dichloromethylen-2-(5-bromo-2-thienyl)acetamido]-3-acetoxymethyl-3-cephem-4-carbonsäure,

7-[2-Dichloromethylen-2-(2-furyl)acetamido]-3-acetoxymethyl-3-cephem-4-carbonsäure,

7-[2-Chloromethylen-2-(2-thienyl)acetamido]-3-(1-methyl-1H-tetrazol-5-yl)thiomethyl-3-cephem-4-carbonsäure,

7-[2-Dichloromethylen-2-(3-thienyl)acetamido]-3-(1-methyl-1H-tetrazol-5-yl)thiomethyl-3-cephem-4-carbonsäure,

7-[2-Dichloromethylen-2-(1-methyl-2-pyrrolyl)acetamido]-3-acetoxymethyl-3-cephem-4-carbonsäure,

7-(2-Dichloromethylen-2-phenylacetamido)-3-acetoxymethyl-3-cephem-4-carbonsäure,

7-[2-Dichloromethylen-2-(2-thienyl)acetamido]-3-(1-methyl-1H-tetrazol-5-yl)thiomethyl-3-cephem-4-carbonsäure,

7-[2-Dichloromethylen-2-(3-thienyl)acetamido]-3-acetoxymethyl-3-cephem-4-carbonsäure,

7-[2-Dichloromethylen-2-(2-thienyl)acetamido]-3-acetoxymethyl-3-cephem-4-carbonsäure,

7-[2-Dichloromethylen-2-(2-aminothiazol-4-yl)acetamido]-3-carbamoyloxymethyl-3-cephem-4-carbonsäure und

7-[2-Chloromethylen-2-(2-aminothiazol-4-yl)acetamido]-3-carbamoyloxymethyl-3-cephem-4-carbonsäure.

4. 6-[2-Dichloromethylen-2-(2-thienyl)acetamido]-penicillansäure.

5. 6-[2-Chloromethylen-2-(2-thienyl)acetamido]-penicillansäure.

6. 7-[2-Chloromethylen-2-(2-aminothiazol-4-yl)acetamido]-3-(1-methyl-1H-tetrazol-5-yl)thiomethyl-3-cephem-4-carbonsäure.

7. 7-[2-Chloromethylen-2-(2-aminothiazol-4-yl)acetamido]-acetoxymethyl-3-cephem-4-carbonsäure.

8. 7-[2-Chloromethylen-2-(2-tert-butoxycarbonylaminothiazol-4-yl)acetamido]-3-(2-methylthio-1,3,4-thiadiazol-5-yl)thiomethyl-3-cephem-4-carbonsäure.

9. 7-[2-Dichloromethylen-2-(2-aminothiazol-4-yl)acetamido]-3-acetoxymethyl-3-cephem-4-carbonsäure.

10. 7-[2-Dichloromethylen-2-(2-aminothiazol-4-yl)acetamido]-3-(1-methyl-1H-tetrazol-5-yl)thiomethyl-3-cephem-4-carbonsäure.

11. 7-[2-Dichloromethylen-2-(aminothiazol-4-yl)acetamido]-3-(2-methylthio-1,3,4-thiadiazol-5-yl)-thiomethyl-3-cephem-4-carbonsäure.

12. Verfahren zur Herstellung einer Verbindung der Formel:

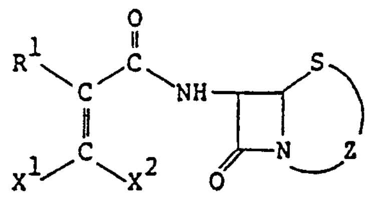

worin $R^1$ die in Anspruch 1 angegebene Bedeutung hat; Z eine Gruppe

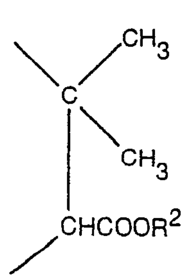

oder

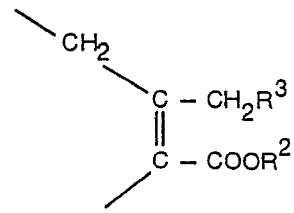

bedeutet (worin $R^2$ Wasserstoff, ein salzbildendes Kation oder eine Schutzgruppe für Carbonsäuren darstellt, und $R^3$ die in Anspruch 1 angegebene Bedeutung hat); und $X^1$ und $X^2$ jeweils Wasserstoff oder ein Halogenatom darstellen, vorausgesetzt, dass beide Substituenten $X^1$ und $X^2$ nicht gleichzeitig Wasserstoff darstellen können,
gekennzeichnet durch Umsetzung einer Verbindung der Formel:

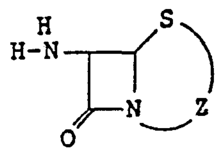

worin Z die vorstehend angegebene Bedeutung hat, einem rekationsfähigen Derivat derselben im Hinblick auf den Aminorest oder ein Salz derselben, mit einer Säure der Formel:

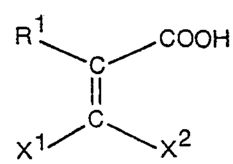

worin $R^1$, $X^1$ und $X^2$ die vorstehend angegebenen Bedeutungen haben, einem reaktionsfähigen Derivat derselben im Hinblick auf den Carbonsäurerest oder einem Salz derselben.

13. Verfahren nach Anspruch 12, wobei die Reaktion in Gegenwart eines Dehydratisierungsmittels oder eines Kondensierungsmittels durchgeführt wird, wenn die Säure in Form einer freien Säure umgesetzt wird.

14. Verfahren nach Anspruch 12, wobei das genannte reaktionsfähige Derivat der Säure ein Säurechlorid, ein Säureanhydrid, ein gemischtes Säureanhydrid, ein aktives Säureamid, ein Säurecyanid, einen aktiven Ester oder ein Säureazid darstellt.

15. Verfahren nach Anspruch 12, wobei die Reaktion in Gegenwart eines Lösungsmittels unter Kühlen oder bei Raumtemperatur durchgeführt wird.

16. Verfahren zur Herstellung einer Verbindung der Formel:

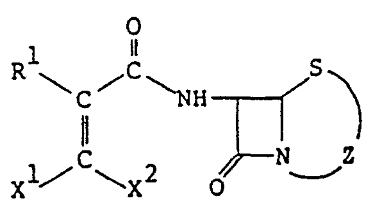

worin $R^1$ die in Anspruch 1 angegebene Bedeutung hat; Z eine Gruppe

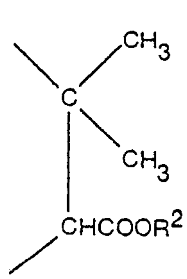

oder

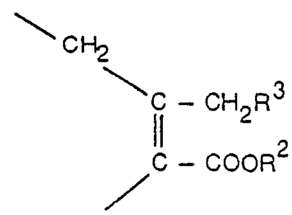

bedeutet (worin $R^2$ Wasserstoff, ein salzbildendes kation oder eine Schutzgruppe für eine Carbonsäure bedeutet, und $R^3$ die in Anspruch 1 angegebene Bedeutung hat); und $X^1$ und $X^2$ jeweils Wasserstoff oder

ein Halogenatom darstellen, vorausgesetzt, dass beide Substituenten $X^1$ und $X^2$ nicht gleichzeitig Wasserstoff bedeuten können,
gekennzeichnet durch einen ersten Schritt einer Umsetzung einer Verbindung der Formel:

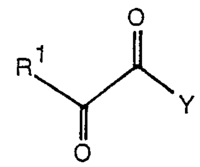

worin $R^1$ die vorstehend angegebene Bedeutung hat; und Y eine Hydroxylgruppe oder eine Schutzgruppe für eine Carbonsäure darstellt;
mit einem Wittig-Reagens der Formel:

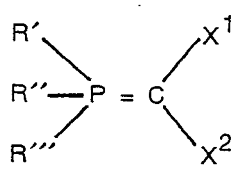

worin R′, R″ und R‴ gleich oder verschieden sein können und jeweils einen Arylrest oder einen Alkylrest bedeuten; und $X^1$ und $X^2$ die vorstehend angegebenen Bedeutungen haben;
unter Bildung einer Verbindung der Formel:

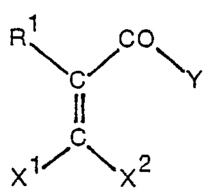

worin $R^1$, $X^1$, $X^2$ und Y die vorstehend angegebenen Bedeutungen haben,
und einem zweiten Schritt einer Umsetzung der so gebildeten Verbindung, einem reaktionsfähigen Derivat derselben oder einem Salz derselben mit einer Verbindung der Formel:

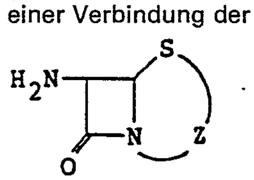

worin Z die vorstehend angegebene Bedeutung hat, einem reaktionsfähigen Derivat derselben im Hinblick auf den Aminorest oder einem Salz derselben.

17. Verfahren nach Anspruch 16, wobei R′, R″ und R‴ eine Arylgruppe darstellen.

18. Verfahren nach Anspruch 17, wobie R′, R″ und R‴ eine Phenylgruppe darstellen.

19. Verfahren zur Herstellung einer Verbindung der Formel:

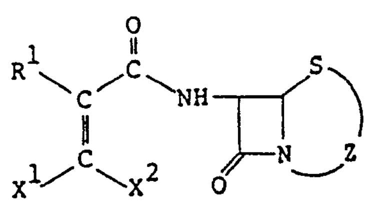

worin $R^1$ die in Anspruch 1 angegebene Bedeutung hat; Z eine Gruppe

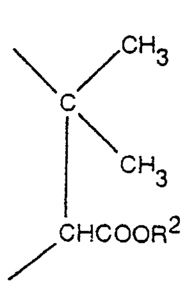

oder

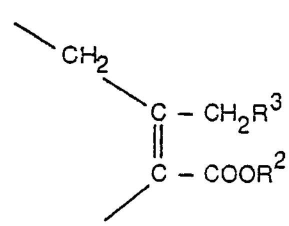

bedeutet (worin $R^2$ Wasserstoff oder ein salzbildendes kation oder eine Schutzgruppe für eine Carbonsäure darstellt und $R^3$ die in Anspruch 1 angegebene Bedeutung hat); und $X^1$ und $X^2$ jeweils Wasserstoff oder ein Halogenatom darstellen, vorausgesetzt, dass beide Substituenten $X^1$ und $X^2$ nicht gleichzeitig Wasserstoff oder ein Halogenatom darstellen können,

gekennzeichnet durch einen ersten Schritt einer Umsetzung einer Verbindung der Formel:

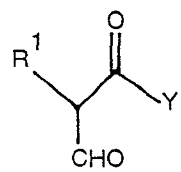

worin $R^1$ die vorstehend angegebene Bedeutung hat; und Y eine Hydroxylgruppe oder eine Schutzgruppe für eine Carbonsäure bedeutet,

mit einem Halogenierungsmittel unter Bildung einer Verbindung der Formel:

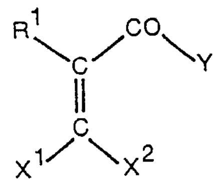

worin $R^1$, $X^1$, $X^2$ und Y die vorstehend angegebenen Bedeutungen haben,

und einem zweiten Schritt einer Umsetzung der so gebildeten Verbindung, einem reaktionsfähigen Derivat derselben oder einem Salz derselben, mit einer Verbindung der Formel:

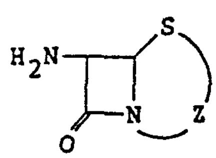

worin Z die vorstehend angegebene Bedeutung hat, einem reaktionsfähigen Derivat derselben im Hinblick auf den Aminorest oder einem Salz derselben.

20. Verfahren nach Anspruch 19, wobei das genannte Halogenierungsmittel ausgewählt wird aus der Gruppe, bestehend aus Phosphorpentachlorid, Phosphoroxychlorid, Thionylchlorid, Phosphortrichlorid, Phosgen und dem Vilsmeier-Reagens, hergestellt aus dem vorstehenden Agens und Dimethylformamid.

21. Verbindung der Formel:

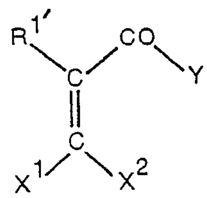

worin $R^{1'}$ eine aromatische Gruppe bedeutet, welche ausgewählt ist aus der Gruppe, bestehend aus einem Thiazolylrest, einem Thienylrest, einem Furylrest und einem Pyrrolylrest, welche substituiert sein können mit einem Halogenatom, einem Nitrorest, einem Aminorest, einem Cyanorest, einem Hydroxyrest, einem Niedrigalkylrest mit 1 bis 4 Kohlenstoffatomen, einem Niedrigalkoxyrest mit 1 bis 4 Kohlenstoffatomen, einem Niedrigalkylthiorest mit 1 bis 4 Kohlenstoffatomen; $X^1$ und $X^2$ jeweils Wasserstoff oder ein Halogenatom darstellen, vorausgesetzt, dass $X^1$ und $X^2$ nicht gleichzeitig Wasserstoff oder ein Halogenatom darstellen können; und Y eine Hydoxylgruppe, ein Halogenatom oder einen Rest —OR darstellt (worin R eine Schutzgruppe für eine Carbonsäure bedeutet).

22. Verbindung nach Anspruch 21, ausgewählt aus der Gruppe bestehend aus:

2-Chloromethylen-2-(2-tert-butoxycarbonylaminothiazol-4-yl)essigsäureethylester,
2-Chloromethylen-2-(2-tert-butoxycarbonylaminothiazol-4-yl)essigsäure,
2-Chloromethylen-2-(2-aminothiazol-4-yl)essigsäureethylester,
2-Chloromethylen-2-(2-aminothiazol-4-yl)essigsäureethylester,
2-Chloromethylen-2-(2-aminothiazol-4-yl)essigsäure,
2-Chloromethylen-2-(2-formylaminothiazol-4-yl)essigsäureethylester,
2-Chloromethylen-2-(2-formylaminothiazol-4-yl)essigsäure,

2-Monochloromethylen-2-(2-thienyl)essigsäure-tert-butylester, 2-Chloromethylen-2-(2-thienyl)essigsäure.

**Revendications**

1. Composé de formule:

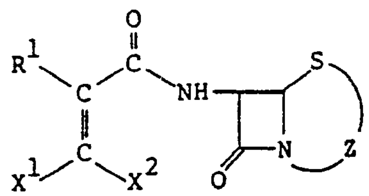

dans laquelle $R^1$ désigne un groupe aromatique choisi parmi un radical phényle, un radical thiazolyle, un radical thiényle, un radical furyle et un radical pyrrolyle, qui peut être substitué par un atome d'halogène, un radical nitro, un radical amino, un radical cyano, un radical hydroxy, un radical alkyle inférieur en $C_1$—$C_4$, un radical alcoxy inférieur en $C_1$—$C_4$, un radical (alkyl inférieur)thio en $C_1$—$C_4$; Z représente un groupe

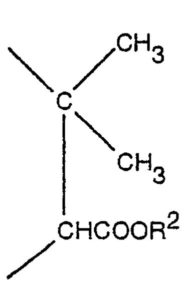

ou

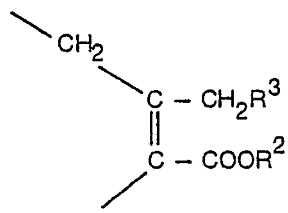

(où $R^2$ désigne l'hydrogène, un cation formant un sel ou un groupe protecteur pour l'acide carboxylique, et $R^3$ désigne l'hydrogène ou un radical choisi parmi un radical hydroxyle, un radical mercapto, un radical acyloxy d'acide carboxylique aliphatique inférieur ayant 2 à 4 atomes de carbone, un radical acyloxy d'acide carboxylique aromatique choisi parmi les radicaux mandélyloxy, 2-carboxy-benzoyloxy, 2-(carboéthoxycarbamoyl)benzoyloxy et 2-(carboéthoxysulfamoyl)benzoyloxy, un radical carbamoyloxy, un radical phénylglycyloxy, un sel d'ammonium quaternaire et un cycle hétérocyclique qui est lié par S, choisi dans le groupe comprenant les radicaux pyridyle, N-oxyde de pyridyle, pyridazinyle, N-oxyde de pyridazinyle, pyrazolyle, diazolyle, thiazolyle, thiadiazolyle, oxadiazolyle, triazolyle et tétrazolyle, qui peut être substitué par un radical alkyle inférieur en $C_1$—$C_3$, un radical alcoxy inférieur en $C_1$—$C_3$, un atome d'halogène, un radical alkyle inférieur trihalogéno-substitué en $C_1$—$C_3$, un radical hydroxy, un radical mercapto, un radical amino, un radical carboxyle, un radical carbamoyle, un radical di(alkyl inférieur)amino (alkyl inférieur) en $C_1$—$C_3$ ou un radical carboxyméthyle; et $X^1$ et $X^2$ représentent chacun l'hydrogène ou un atome d'halogène, sous réserve que $X^1$ et $X^2$ ne peuvent pas représenter simultanément l'hydrogène.

2. Composé selon la revendication 1, dans lequel $X^1$ et $X^2$ sont chacun un atome de chlore ou un hydrogène.

3. Composé selon la revendication 1, choisi dans le groupe constitué de

l'acide 7-[2-dichlorométhylène-2-(2-furyl)acétamido]-3-(1-méthyl-1H-tétrazol-5-yl)thiométhyl-3-céphème-4-carboxylique,

l'acide 7-[2-dichlorométhylène-2-(4-hydroxyphényl)acétamido]-3-acétoxyméthyl-3-céphème-4-carboxylique,

l'acide 7-[2-dichlorométhylène-2-(3-hydroxyphényl)acétamido]-3-acétoxyméthyl-3-céphème-4-carboxylique,

l'acide 7-[2-dichlorométhylène-2-(4-tert-butoxycarbonylaminophényl)acétamido]-3-acétoxyméthyl-3-céphème-4-carboxylique,

l'acide 7-[2-dichlorométhylène-2-(4-aminophényl)acétamido]-3-acétoxyméthyl-3-céphème-4-carboxylique,

l'acide 7-[2-dichlorométhylène-2-(2-tert-butoxycarbonylaminothiazol-4-yl)acétamido]-3-acétoxy-méthyl-3-céphème-4-carboxylique,

l'acide 7-[2-dichlorométhylène-2-(2-aminothiazol-4-yl)acétamido]-3-acétoxyméthyl-3-céphème-4-carboxylique,

l'acide 7-[2-dichlorométhylène-2-(2-tert-butoxycarbonylaminothiazol-4-yl)acétamido]-3-(1-méthyl-1H-tétrazol-5-yl)thiométhyl-3-céphème-4-carboxylique,

l'acide 7-[2-dichlorométhylène-2-(2-aminothiazol-4-yl)acétamido]-3-(1-méthyl-1H-tétrazol-5-yl)-thiométhyl-3-céphème-4-carboxylique,

l'acide 7-[2-dichlorométhylène-2-(2-tert-butoxycarbonylaminothiazol)-4-yl)acétamido]-3-(1-carboxyméthyltétrazol-5-yl)thiométhyl-3-céphème-4-carboxylique,

l'acide 7-[2-dichlorométhylène-2-(2-aminothiazol-4-yl)acétamido]-3-(1-carboxyméthyltétrazol-5-yl)thiométhyl-3-céphème-4-carboxylique,

l'acide 7-[2-dichlorométhylène-2-(2-aminothiazol-4-yl)acétamido]-3-(1-carboxyméthyltétrazol-5-yl)-thiométhyl-3-céphème-4-carboxylique,

l'acide 7-[2-dichlorométhylène-2-(2-tert-butoxycarbonylaminothiazol-4-yl)acétamido]désacétoxy-céphalosporanique,

l'acide 7-[2-dichlorométhylène-2-(2-aminothiazol-4-yl)acétamido]désacétoxycéphalosporanique,

l'acide 7-[2-dichlorométhylène-2-(aminothiazol-4-yl)acétamido]-3-(2-méthylthio-1,3,4-thiadiazol-5-yl)-thiométhyl-3-céphème-4-carboxylique,

l'acide 7-[2-dichlorométhylène-2-(2-aminothiazol-4-yl)acétamido]-3-[1-(2-diméthylaminoéthyl)(1H-tétrazol-5-yl]thiométhyl-3-céphème-4-carboxylique,

l'acide 7-[2-chlorométhylène-2-(2-tert-butoxycarbonylaminothiazol-4-yl)acétamido]-3-(1-méthyl-1H-tétrazol-5-yl)thiométhyl-3-céphème-4-carboxylique,

l'acide 7-[2-chlorométhylène-2-(2-aminothiazol-4-yl)acétamido]-3-(1-méthyl-1H-tétrazol-5-yl)-thiométhyl-3-céphème-4-carboxylique,

l'acide 7-[2-chlorométhylène-2-(2-tert-butoxycarbonylaminothiazol-4-yl)acétamido]-3-acétoxyméthyl-3-céphème-4-carboxylique,

l'acide 7-[2-chlorométhylène-2-(2-tert-butoxycarbonylaminothiazol-4-yl)acétamido]-3-(2-méthylthio-1,3,4-thiadiazol-5-yl)thiométhyl-3-céphème-4-carboxylique,

l'acide 7-[2-chlorométhylène-2-(2-tert-butoxycarbonylaminothiazol-4-yl)acétamido]-3-[(2,5-dihydro-6-hydroxy-2-méthyl-5-oxo-as-triazin-3-yl)thiométhyl]-3-céphème-4-carboxylique,

l'acide 7-[2-chlorométhylène-2-(2-tert-butoxycarbonylaminothiazol-4-yl)acétamido]désacétoxy-céphalosporanique,

l'acide 7-[2-chlorométhylène-2-(2-aminothiazol-4-yl)acétamido]-3-acétoxyméthyl-3-céphème-4-carboxylique,

l'acide 7-[2-chlorométhylène-2-(2-aminothiazol-4-yl)acétamido]-3-(2-méthylthio-1,3,4-thiadiazol-5-yl)-thiométhyl-3-céphème-4-carboxylique,

l'acide 7-[2-chlorométhylène-2-(2-aminothiazol-4-yl)acétamido]-3-[(2,5-dihydro-6-hydroxy-2-méthyl-5-oxo-as-triazin-3-yl)thiométhyl]-3-céphème-4-carboxylique,

l'acide 7-[2-chlorométhylène-2-(2-aminothiazol-4-yl)acétamido]désacétoxycéphalosporanique,

la bétaïne de l'acide 7-[2-chlorométhylène-2-(2-aminothiazol-4-yl)acétamido]-3-pyridiniumméthyl-3-céphème-4-carboxylique,

l'acide 7-[2-chlorométhylène-2-(2-formylaminothiazol-4-yl)acétamido]-3-(1-méthyl-1H-tétrazol-5-yl)-thiométhyl-3-cépheme-4-carboxylique,

l'acide 7-[2-dichlorométhylène-2-(4-méthoxyphényl)acétamido]-3-céphème-4-carboxylique,

l'acide 7-[2-dichlorométhylène-2-(4-méthylphényl)acétamido]-3-acétoxyméthyl-3-céphème-4-carboxylique,

l'acide 7-[2-dichlorométhylène-2-(4-chlorophényl)acétamido]-3-acétoxyméthyl-3-céphème-4-carboxylique,

l'acide 7-[2-dichlorométhylène-2-(3,4-dichlorophényl)acétamido]-3-acétoxyméthyl-3-céphème-4-carboxylique,

l'acide 7-[2-dichlorométhylène-2-(5-chloro-2-thiényl)acétamido]-3-acétoxyméthyl-3-céphème-4-carboxylique,

l'acide 7-[2-dichlorométhylène-2-(5-bromo-2-thiényl)acétamido]-3-acétoxyméthyl-3-céphème-4-carboxylique,

l'acide 7-[2-dichlorométhylène-2-(2-furyl)acétamido]-3-acétoxyméthyl-3-céphème-4-carboxylique,

l'acide 7-[2-chlorométhylène-2-(2-thiényl)acétamido]-3-(1-méthyl-1H-tétrazol-5-yl)thiométhyl-3-céphème-4-carboxylique,

l'acide 7-[2-dichlorométhylène-2-(3-thiényl)acétamido]-3-(1-méthyl-1H-tétrazol-5-yl)thiométhyl-3-céphème-4-carboxylique,

l'acide 7-[2-dichlorométhylène-2-(1-méthyl-2-pyrrolyl)acétamido]-3-acétoxyméthyl-3-céphème-4-carboxylique,

l'acide 7-(2-dichlorométhylène-2-phénylacétamido)-3-acétoxyméthyl-3-céphème-4-carhoxylique,

l'acide 7-[2-dichlorométhylène-2-(2-thiényl)acétamido]-3-(1-méthyl-1H-tétrazol-5-yl)thiométhyl-3-céphème-4-carboxylique,

l'acide 7-[2-dichlorométhylène-2-(3-thiényl)acétamido]-3-acétoxyméthyl-3-céphème-4-carboxylique,

l'acide 7-[2-dichlorométhylène-2-(2-thiényl)acétamido]-3-acétoxyméthyl-3-céphème-4-carboxylique,

l'acide 7-[2-dichlorométhylène-2-(2-aminothiazol-4-yl)acétamido]-3-carbamoyloxyméthyl-3-céphème-4-carboxylique et

l'acide 7-[2-chlorométhylène-2-(2-aminothiazol-4-yl)acétamido]-3-carbamoyloxyméthyl-3-céphème-4-carboxylique.

4. Composé selon la revendication 1, qui est l'acide 6-[2-dichlorométhylène-2-(2-thiényl)acétamido]-pénicillanique.

5. Composé selon la revendication 1, qui est l'acide 6-[2-chlorométhylène-2-(2-thiényl)acétamido]-pénicillanique.

6. Composé selon la revendication 3, qui est l'acide 7-[2-chlorométhylène-2-(2-aminothiazol-4-yl)-acétamido]-3-(1-méthyl-1H-tétrazol-5-yl)thiométhyl-3-céphème-4-carboxylique.

7. Composé selon la revendication 3, qui est l'acide 7-[2-chlorométhylène-2-(2-aminothiazol-4-yl)-acétamido]-3-acétoxyméthyl-3-céphème-4-carboxylique.

8. Composé selon la revendication 3, qui est l'acide 7-[2-chlorométhylène-2-(2-tert-butoxycarbonylaminothiazol-4-yl)acétamido]-3-(2-méthylthio-1,3,4-thiadiazol-5-yl)thiométhyl-3-céphème-4-carboxylique.

9. Composé selon la revendication 3, qui est l'acide 7-[2-dichlorométhylène-2-(2-aminothiazol-4-yl)-acétamido]-3-acétoxyméthyl-3-céphème-4-carboxylique.

10. Composé selon la revendication 3, qui est l'acide 7-[2-dichloiro-méthylène-2-(2-aminothiazol-4-yl)-acétamido]-3-(1-méthyl-1H-tétrazol-5-yl)thiométhyl-3-céphème-4-carboxylique.

11. Composé selon la revendication 3, qui est l'acide 7-[2-dichlorométhylène-2-(aminothiazol-4-yl)-acétamido]-3-(2-méthylthio-1,3,4-thiadiazol-5-yl)thiométhyl-3-céphème-4-carboxylique.

12. Procédé de préparation d'un composé de formule:

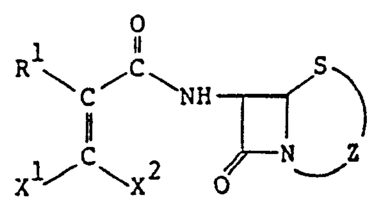

dans laquelle $R^1$ a la même signification que dans la revendication 1; Z représente un groupe

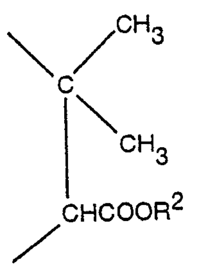

ou

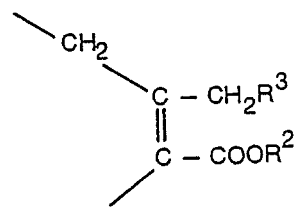

(où $R^2$ désigne l'hydrogène, un cation formant un sel ou un groupe protecteur pour l'acide carboxylique, et $R^3$ a la même signification que dans la revendication 1); et $X^1$ et $X^2$ représentent chacun l'hydrogène ou un atome d'halogène, sous réserve que $X^1$ et $X^2$ ne peuvent pas représenter simultanément l'hydrogène, qui consiste à faire réagir un composé de formule:

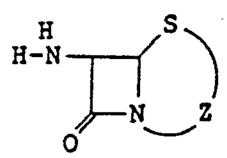

dans laquelle Z a la même signification que ci-dessus, un de ses dérivé réactifs en ce qui concerne le radical amino ou un de ses sels, avec un acide de formule:

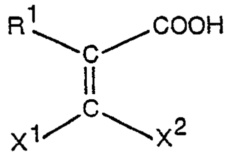

dans laquelle $R^1$, $X^1$ et $X^2$ ont les mêmes signfications que ci-dessus,
un de ses dérivés réactifs en ce qui concerne le radical carboxyle ou un de ses sels.

13. Procédé selon la revendication 12, dans lequel la réaction est effectuée en présence d'un agent de déshydratation ou d'un agent de condensation lorsque l'acide est mis à réagir sous la forme d'un acide libre.

14. Procédé selon la revendication 12, dans lequel ledit dérivé réactif de l'acide est un chlorure d'acide, un anhydride d'acide, un anhydride d'acide mixte, un amide actif, un cyanure d'acide, un ester actif ou un azide d'acide.

15. Procédé selon la revendication 12, dans lequel la réaction est effectuée en présence d'un solvant, en refroidissant ou à la température ambiante.

16. Procédé de préparation d'un composé de formule:

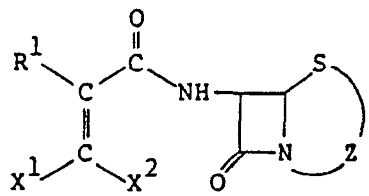

dans laquelle $R^1$ a la même signification que dans la revendication 1; Z représente un groupe

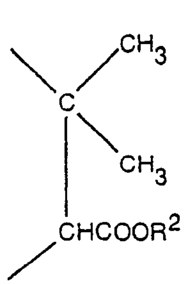

ou

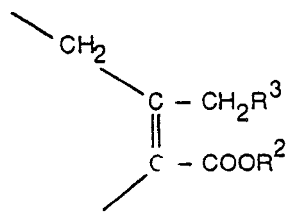

(où $R^2$ désigne l'hydrogène, un cation formant un sel ou un groupe protecteur pour un acide carboxylique, et $R^3$ a la même signification que dans la revendication 1);
et $X^1$ et $X^2$ représentent chacun l'hydrogène ou un atome d'halogène, sous réserve que $X^1$ et $X^2$ ne peuvent pas représenter simultanément l'hydrogène,

qui comprend le premier stade consistant à faire réagir un composé de formule:

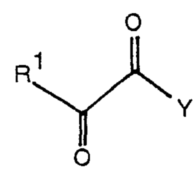

dans laquelle $R^1$ a la même signification que ci-dessus; et Y représente un groupe hydroxyle ou un groupe protecteur pour un acide carboxylique,

avec un réactif de Wittig de formule:

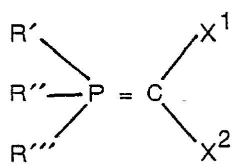

dans laquelle R′, R″ et R‴ peuvent être identiques ou différents et représentent chacun un radical aryle ou un radical alkyle; et $X^1$ et $X^2$ ont les mêmes significations que ci-dessus,

pour former un composé de formule:

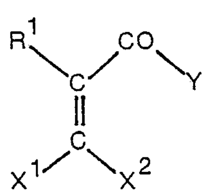

dans laquelle $R^1$, $X^1$, $X^2$ et Y ont les mêmes significations que ci-dessus,

et le second stade consistant à faire réagir le composé ainsi formé, un de ses dérivés réactifs ou un de des sels, avec un composé de formule:

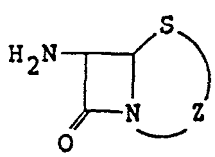

dans laquelle Z a la même signification que ci-dedssus, un de ses dérivés réactifs en ce que concerne le radical amino ou un de des sels.

17. Procédé selon la revendication 16, dans lequel R′, R″ et R‴ représentent un groupe alkyle.

18. Procédé selon la revendication 17, dans lequel R′, R″ et R‴ représentent un groupe phényle.

19. Procédé de préparation d'un composé de formule:

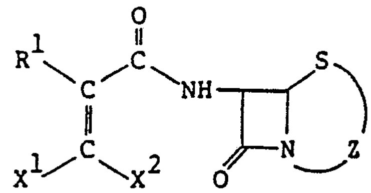

dans laquelle $R^1$ a la même signification que dans la revendication 1; Z représente un groupe

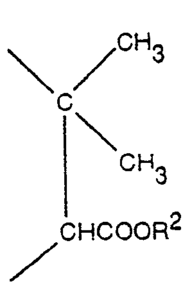

ou

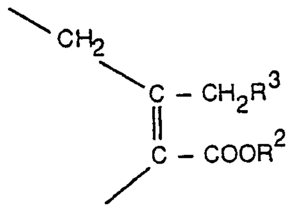

(où $R^2$ désigne l'hydrogène, un cation formant un sel ou un groupe protecteur pour un acide carboxylique, et $R^3$ a la même signification que dans la revendication 1); et $X^1$ et $X^2$ représentent chacun l'hydrogène ou un atome d'halogène, sous réserve que $X^1$ et $X^2$ ne peuvent pas représenter tous deux l'hydrogène ou un atome d'halogène simultanément,

qui comprend le premier stade consistant à faire réagir un composé de formule:

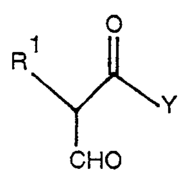

dans laquelle $R^1$ a la même signification que ci-dessus; et Y représente un groupe hydroxyle ou un groupe protecteur pour un acide carboxylique,

avec un agent halogénant pour former un composé de formule:

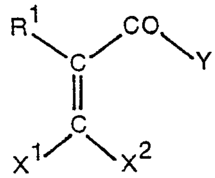

dans laquelle $R^1$, $X^1$, $X^2$ et Y ont les mêmes significations que ci-dessus,

et le second stade consistant à faire réagir le composé ainsi formé, un de ses dérivés réactifs ou un de des sels avec un composé répondant à la formule;

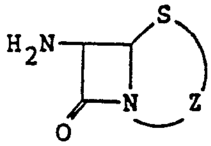

dans laquelle Z a la même signification que ci-dessus, un de ses dérivés réactifs en ce qui concerne le radical amino ou un des sels.

20. Procédé selon la revendication 19, dans lequel ledit agent halogénant est choisi dans le groupe constitué du pentachlorure de phosphore, de l'oxychlorure de phosphore, du chlorure de thionyle, du trichlorure de phosphore, du phosgène et du réactif de Vilsmeier préparé à partir de l'agent ci-dessus et de diméthylformamide.

21. Composé de formule:

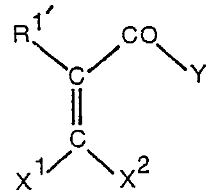

dans laquelle $R^{1'}$ désigne un groupe aromatique choisi parmi le groupe constitué par un radical thiazolyle, un radical thiényle, un radical furyle et un radical pyrrolyle, qui peut être substitué par un atome d'halogène, un radical nitro, un radical amino, un radical cyano, un radical hydroxy, un radical alkyle inférieur en $C_1$—$C_4$, un radical alcoxy inférieur en $C_1$—$C_4$, un radical (alkyl inférieur)thio en $C_1$—$C_4$; $X^1$ et $X^2$ representent chacun l'hydrogène ou un atome d'halogène, sous réserve que $X^1$ et $X^2$ ne peuvent pas représenter simultanément l'hydrogène ou un atome d'halogène; et Y désigne un groupe hydroxyle, un atome d'halogène ou un radical —OR (où R désigne un radical protecteur pour un acide carboxylique).

22. Composé selon la revendication 21, qui est choisi parmi
le 2-chlorométhylène-2-(2-tert-butoxycarbonylaminothiazol-4-yl)cétate d'éthyle,
l'acide 2-chlorométhylène-2-(2-tert-butoxycarbonylaminothiazol-4-yl)acétique,
le 2-chlorométhylène-2-(2-aminothiazol-4-yl)acétate d'éthyle,
le 2-chlorométhylène-2-(2-aminothiazol-4-yl)acétate d'éthyle,
l'acide 2-chlorométhylène-2-(2-aminothiazol-4-yl)acétique,
le 2-chlorométhylène-2-(2-formylaminothiazol-4-yl)acétate d'éthyle,
l'acide 2-chlorométhylène-2-(2-formylaminothiazol-4-yl)acétique,
le 2-monochlorométhylène-2-(2-thiényl)acétate de butyle,
l'acide 2-chlorométhylène-2-(2-thiényl)acétique.